# EUROPEAN PATENT APPLICATION

(11) **EP 4 092 113 A1**
(43) Date of publication of application: **23.11.2022**
(21) Application number: 20913226.5
(22) Date of filing: 13.01.2020
(51) Int. Cl.: C12N 5/079, A61K 35/30, A61P 25/28, A61P 3/10, G01N 33/50

(54) **DIFFERENTIATION METHOD FOR PROCURING LARGE AMOUNT OF CELLS BY CHOPPING 3D ORGANOIDS PREPARED FROM HUMAN PLURIPOTENT STEM CELLS**

(71) Applicant: Corestem Co., Ltd., Seongnam-si, Gyeonggi-do 13486 (KR)
(72) Inventor: LEE, Sang Hun, Seoul 04763 (KR); CHANG, Mi Yoon, Seoul 04763 (KR); KIM, Seung Won, Seoul 03009 (KR); WOO, Hye Ji, Seoul 07206 (KR); SULISTIO, Yanuar Alan, Seoul 04792 (KR)
(74) Representative: HGF
(86) International application number: PCT/KR2020/000581
(87) International publication number: WO 2021/145467

(57) **Abstract**

The present invention relates to a method of patterning and chopping 3D organoids prepared from human pluripotent stem cells, culturing the stem cells or progenitor cells, and inducing the differentiation thereof to obtain a large amount of finally differentiated cells. Compared to cells differentiated by a conventional differentiation method, the cells obtained in a large amount exhibit remarkably superior effects in terms of reproducibility, stability, and functionality, and thus are expected to be very useful for cell therapeutic agents or for the screening of therapeutic drugs.

## Description

### Technical Field

The present invention relates to a differentiation method, which separates and chops 3D organoids prepared from human pluripotent stem cells to secure a large amount of finally differentiated cells.

### Background Art

Parkinson's disease is a neurodegenerative disease caused by loss of dopamine neurons in the substantia nigra in the midbrain. It is known to be the most suitable disease for stem cell transplantation because the lesion site and lesion cells are clear.

For the cell therapy of Parkinson's disease, a technology to differentiate from stem cells into human midbrain-type dopamine neurons (mDA neurons) is required, and differentiation methods have been developed for a long time to differentiate from human pluripotent stem cells (hPSCs, human embryonic stem cells (hESCs) or human induced pluripotent stem cells (hiPSCs)) into mDA neurons [3D brain Organoids derived from pluripotent stem cells: promising experimental models for brain development and neurodegenerative disorders, Journal of Biomedical Science (2017) 24:59]. However, in using these differentiation methods, for clinical application, several problems have been presented as follows.

First, there is a problem of reproducibility. Because the existing two-dimensional method is very complicated, it is not well reproduced in other laboratories. In addition, the existing two-dimensional method was applied only to some pluripotent stem cell lines such as H9, which was used for development in all the developed methods, and differentiation into midbrain dopamine neurons did not occur well in several other pluripotent stem cell lines.

Second, there is a problem of stable expression of midbrain-specific factors. Midbrain-specific factors such as Nurr1, Foxa2, and Lmx1a are known to be critically important for the survival, function, and maintenance of midbrain dopamine neurons, and thus they are closely related to the ability to treat Parkinson's disease after transplantation. Because of this fact, all of the recently developed protocols for differentiating hPSC-mDA neurons were developed focusing on the expression of midbrain-specific factors in midbrain dopamine neurons. However, the expression of these midbrain-specific factors is very unstable and is easily reduced after long-term culture or transplantation of midbrain dopamine neurons. Therefore, in transplanting cells differentiated by the methods developed up to now, only the expression of some midbrain-specific factors (mainly Foxa2) is shown in the transplanted midbrain dopamine neurons.

Third, there is a problem in that astrocytes do not exist in the culture of differentiated mDA neurons. Astrocytes are not included in the culture of mDA neurons differentiated by the methods developed up to now. Astrocytes are cells that are responsible for the enhancement of survival and function of neurons such as mDA neurons, and are essential for the maintenance of normal survival and function of neurons in actual brain tissue. In addition, astrocytes are required to maintain the expression of midbrain-specific factors in mDA neurons.

Finally, there is a problem in the amount of neurons that can be obtained through differentiation of neurons. Since the dopaminergic neuron differentiation methods developed up to now induce differentiation into neurons directly without a neural stem cell stage capable of proliferating in the middle, there is a limit to the amount of finally differentiated neurons that can be obtained through one differentiation.

### Detailed Description of Invention

### Technical Problem

Accordingly, the present inventors made research efforts focusing on the fact that three-dimensional culture using organoids more closely implements the actual brain environment than two-dimensional cell culture, and enables healthy cell culture. Each organoid capable of sufficiently containing target cells, i.e., progenitor cells or stem cells, was patterned to prepare a cell population of a desired fate into a plurality of cell populations (target cell enriched). This organoid tissue was chopped, and progenitor cells or stem cells were cultured. Other technologies are applied to the development of a differentiation method by either patterning and quantitatively securing cells by a two-dimensional culture method, or borrowing only a three-dimensional sphere form in the intermediate stage in order to obtain cells, whereas in the present invention, cells in a desired state are sufficiently enriched (target cells) by appropriate culture through the organoid in a perfect form. Therefore, it was confirmed that each cell isolated in the present invention was more functionally superior by going through a complete three-dimensional artificial brain region called an organoid compared to other existing technologies.

### Solution to Problem

Therefore, an object of the present invention is to provide a method of patterning and chopping 3D organoids prepared from human pluripotent stem cells, culturing the stem cells or progenitor cells, and inducing the differentiation thereof to obtain a large amount of finally differentiated cells.

In addition, another object of the present invention is to provide a cell therapeutic agent comprising the differentiated cells obtained by the above method as an active ingredient.

In addition, another object of the present invention is to provide a drug screening method using the differentiated cells obtained by the above method.

### Effects of Invention

In the present invention, the finally differentiated cells may be differentiated in a large amount by chopping the prepared 3D organoids, separating stem cells or progenitor cells from the organoids and culturing the same to proliferate them, and thus a large amount of cells may be obtained at once. For example, when the method of the present invention is applied to mDA neuron differentiation, differentiation may be easily induced without cell line-dependent or batch-to-batch variation, and thus the method of the present invention may be easily reproduced in other laboratories. In addition, it was confirmed that, when the midbrain-type neural stem cells differentiated according to the present invention are transplanted into Parkinson's disease, a series of Nurr1, Foxa2, Lmx1a, and the like, which are known to be critically important for the survival, function, and maintenance of mDA neurons, are faithfully expressed in the transplanted mDA neurons, and thus the therapeutic effect of cell transplantation is remarkably improved. In addition, astrocytes, which are cells responsible for enhancing the survival and function of neurons, also exist along with mDA neurons, and thus astrocytes protect mDA neurons and enhance the function of mDA neurons, which seems to have contributed to the enhancement of cell transplantation treatment during transplantation.

Therefore, the differentiated cells obtained in such a large amount (including cerebral astrocyte progenitor cells, midbrain astrocyte progenitor cells, midbrain dopaminergic neural stem cells, hypothalamus neural stem cells, and the like, in addition to mDA neurons) may be used as a cell therapeutic agent or for the screening of drugs capable of being treated with cells.

### Brief Description of Drawings

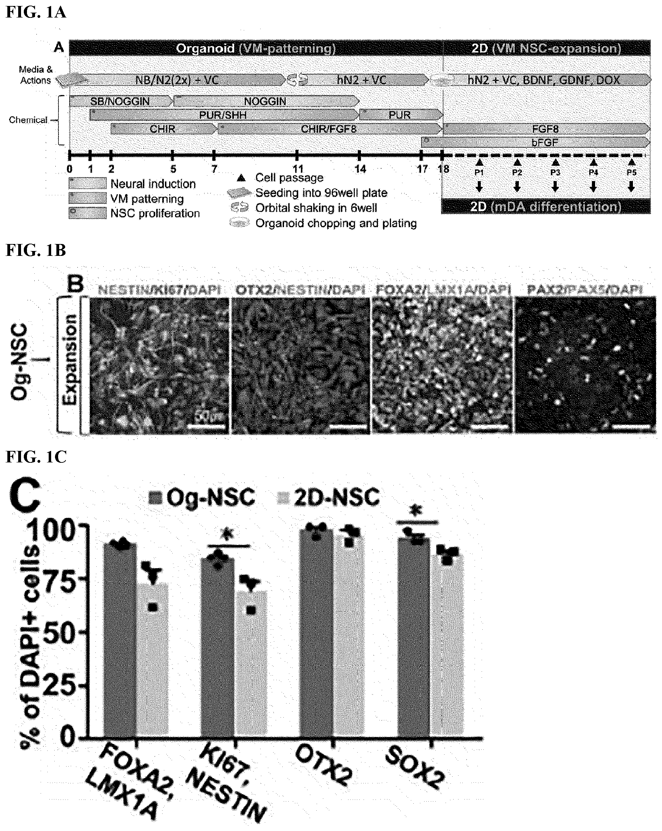

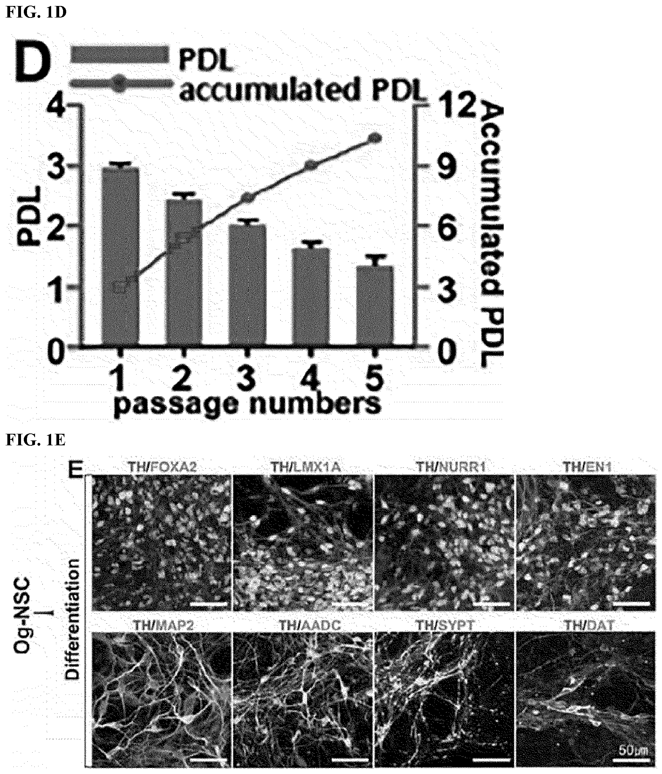

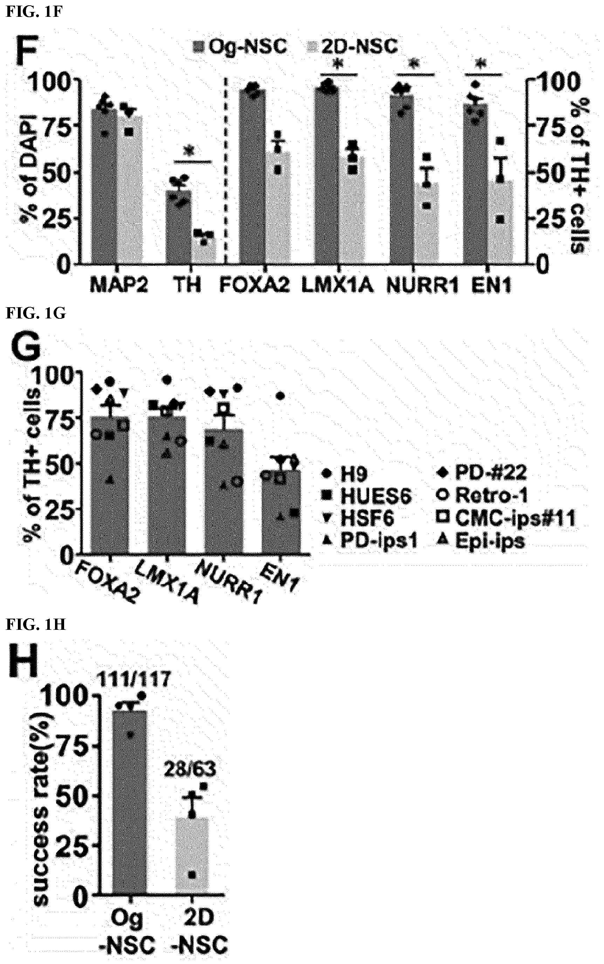

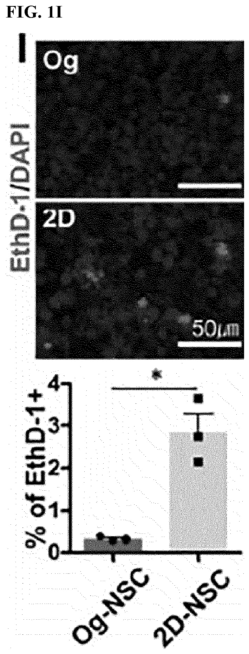

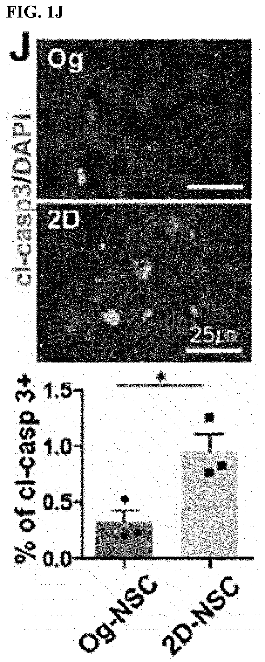

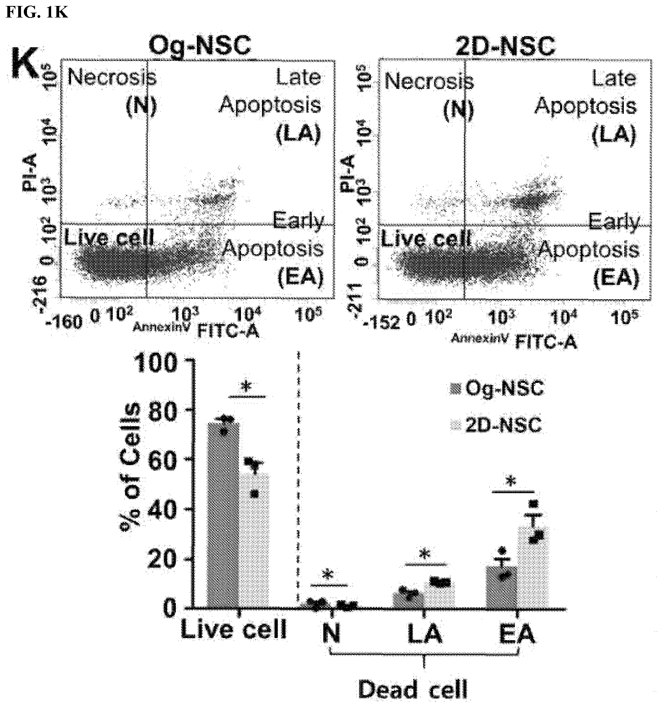

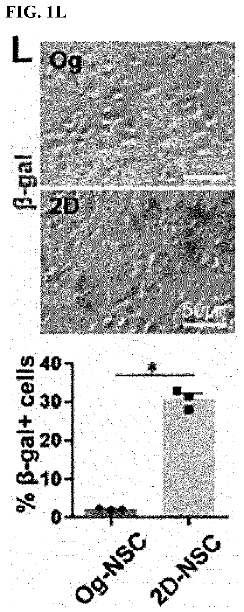

FIG. 1 shows a method for preparing midbrain-like organoids and extracting midbrain-type neural stem cells therefrom [A: A diagram showing a method for proliferating neural stem cells from organoids with bFGF in a 2D environment to obtain them. B and C: Confirmation of expression of midbrain-type markers (OTX2, FOXA2, LMX1A, PAX2, and PAX5) and neural stem cell markers (SOX2, NESTIN, and KI67) by fluorescence staining of midbrain-type neural stem cells proliferated from organoids for 2 weeks. D; Calculation of proliferation rate of midbrain-type neural stem cells proliferated from organoids. PDL (population doubling level, bars) and cumulative PDL (line). E-G: Confirmation of accurate differentiation of midbrain-type dopamine neurons after induction of differentiation of midbrain-type neural stem cells proliferated from organoids for 12 to 15 days. Confirmation of midbrain-type markers (FOXA2, LMX1A, NURR1, and EN1) and fully mature neuron differentiation markers (AADC, SYPT, and DAT) along with dopamine neuron markers (TH). F: Confirmation of % expression relative to neurons differentiated from 2D neural stem cells. G: It was confirmed that the protocol was stably implemented when using various human embryonic stem cells and induced pluripotent stem cells. H: Confirmation of stable differentiation success rate of an organoid-derived neural stem cell differentiation technology relative to a 2D differentiation method. (when performing each method, as a criterion of success, >70% (>30%) TH+ of MAP2+ neurons and >70% (>20%) NURR1+ of TH+ mDA neurons). I-L: An organoid-derived neural stem cell differentiation protocol can more stably inhibit cell death and differentiate cells. EthD-1+ dead cells (I), cleaved caspase 3+ dead cells (J), FACS-using Annexin+/PI+ cell calculation (K), and programmed cell death as β-galactosidase-stained cells (cell senescence) (L). ^{∗} *P=* 0.0045 (I), 0.02 (J), 0.008-0.044 (L), 0.003 (K), n= 3 independent experiments, one-tailed Student's t-test. Scale bars, 25 µm (J), 50 µm (B, E, I, and K)].

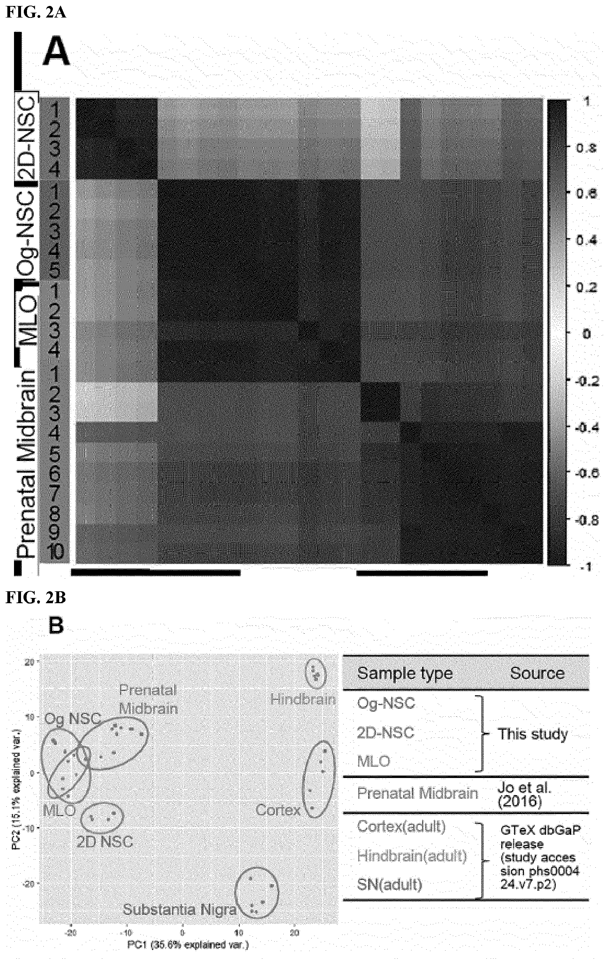

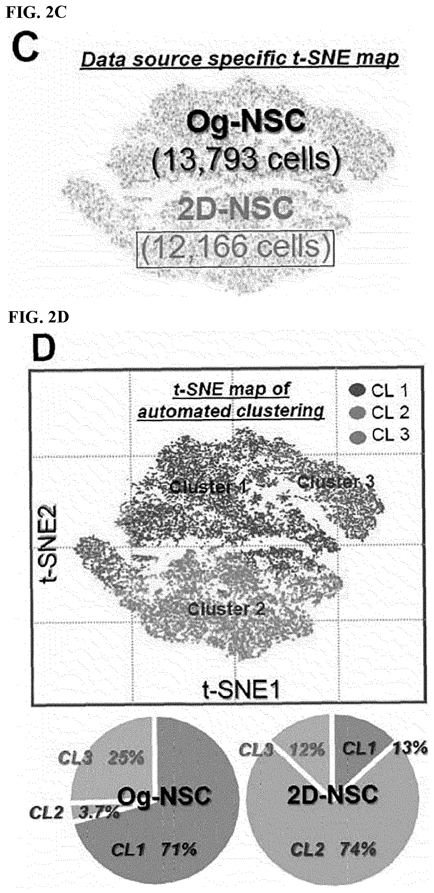

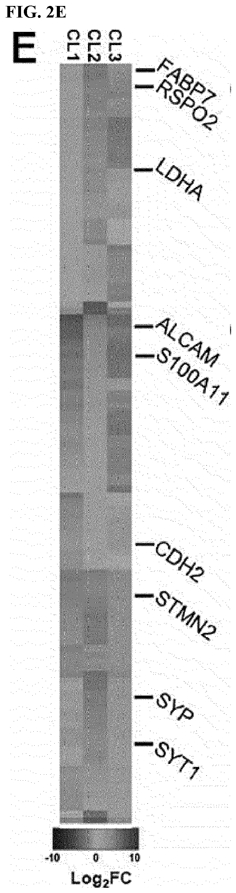

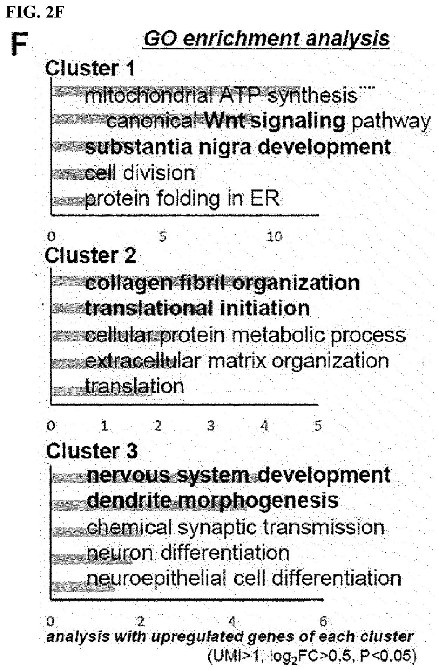

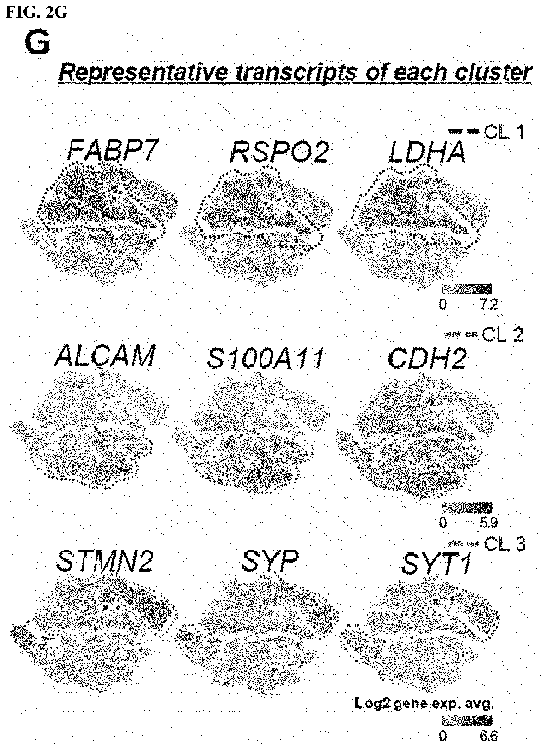

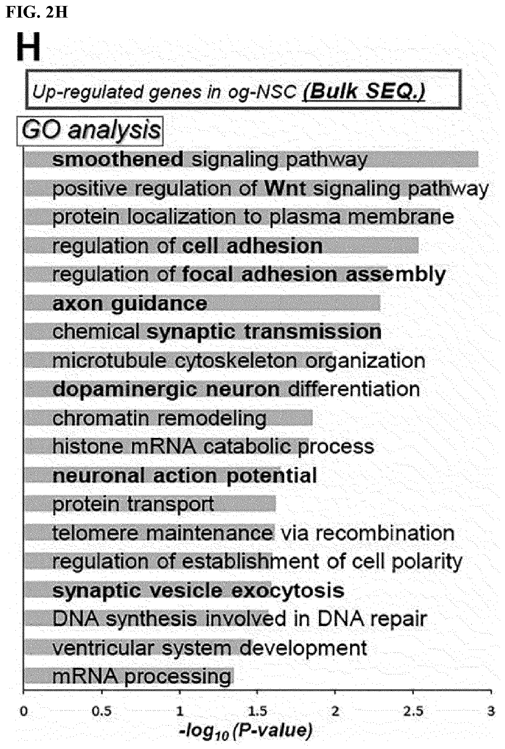

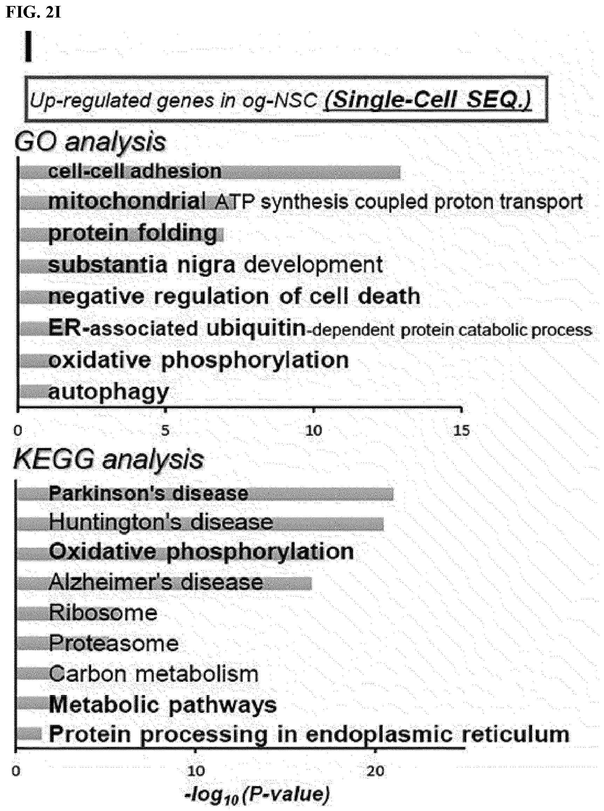

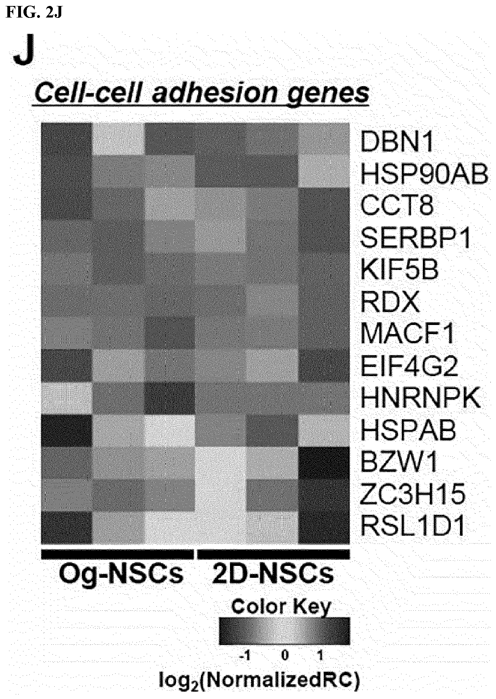

FIG. 2 shows the analysis of the transcriptional regulation (transcriptome characteristics) of organoid-derived neural stem cells [A and B: Analysis of differences in regulation of transcription factors by bulk RNA sequencing from RNA of midbrain-like organoids (MLO), organoid-derived neural stem cells (Og-NSCs), and 2D derived neural stem cells (2D-NSCs) that were prepared, and previously reported transcription factors of midbrain tissue during development (published transcriptome datasets of prenatal midbrain), adult striatum with dopaminergic neurons (adult substantia nigra), adult cerebral cortex (cortex), and hindbrain. A: Spearman's correlation plot. Analysis of the DEGs (differentially expressed genes) of 2D neural stem cells and organoid-derived neural stem cells. B: PCA (principle component analysis) of bulk RNA sequencing data. The PCA score is centered on the 1,000 genes with the greatest difference in expression. C-G: Analysis of transcriptional regulation of organoid-derived neural stem cells (Og-NSCs) and 2D derived neural stem cells (2D-NSCs) by single cell RNA sequencing. (13,793 and 12,166 cells were analyzed, respectively) C: A t-SNE (t-distributed stochastic neighbor embedding) map is expressed in data-based color. D: Three separate k-means are clustered by t-SNE projection. (Loupe Cell Browser ver. 3.0.1, 10X Genomics, Inc.). The analysis of Og- and 2D-neural stem cells is analyzed as a Venn diagram, respectively (below). E: Hierarchical clustering analysis of 40 overexpressed genes compared to other groups in each cluster. F: Top-ranked GOs based on overexpressed genes in each group (UMI>1, log₂FC>0.5, P<0.05, DAVID). G, representative gene expression specifically regulated compared to other groups. Data is represented by the depth of color (log2 expression (UMI). F-H, (DEGs) GOs showing more pronounced expression in Og-neural stem cells compared to the analysis of 2D-neural stem cells (vs 2D-NSCs, log₂FC >1, padj<0.01) were analyzed using bulk (H) and single-cell RNA-seq datasets (I). The expression of 'cell-cell adhesion' was increased in both bulk- and single-cell RNA-seq data, and thus heatmap analysis was performed (J).

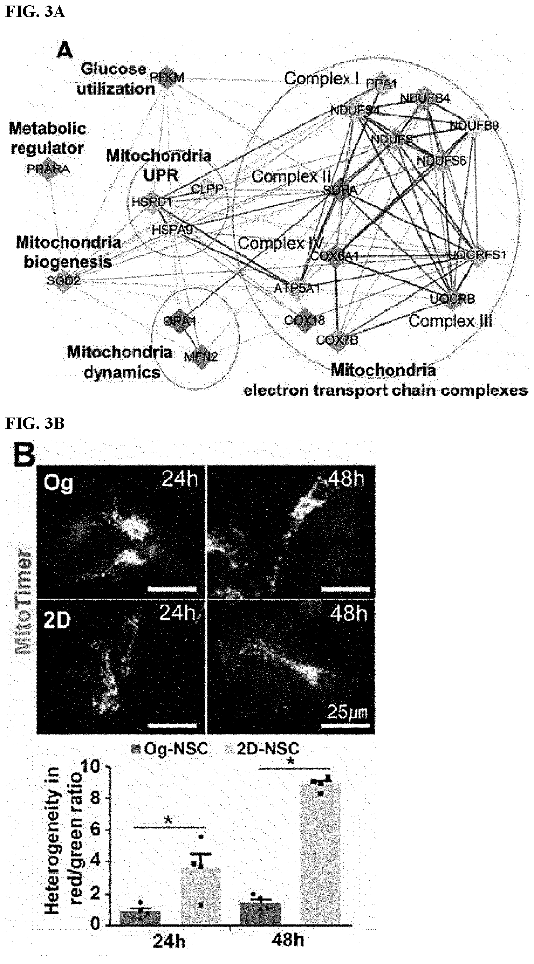

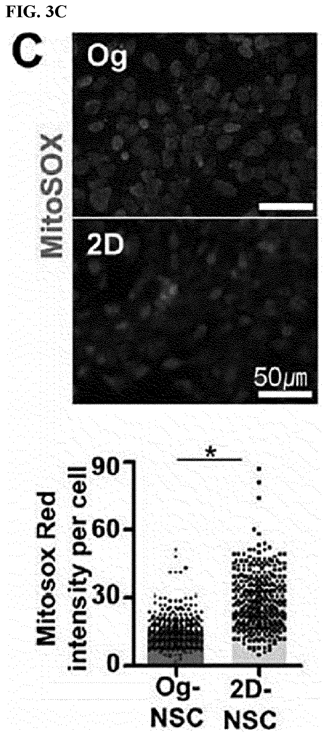

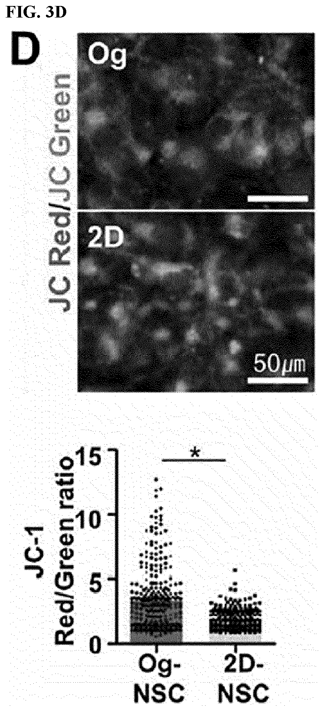

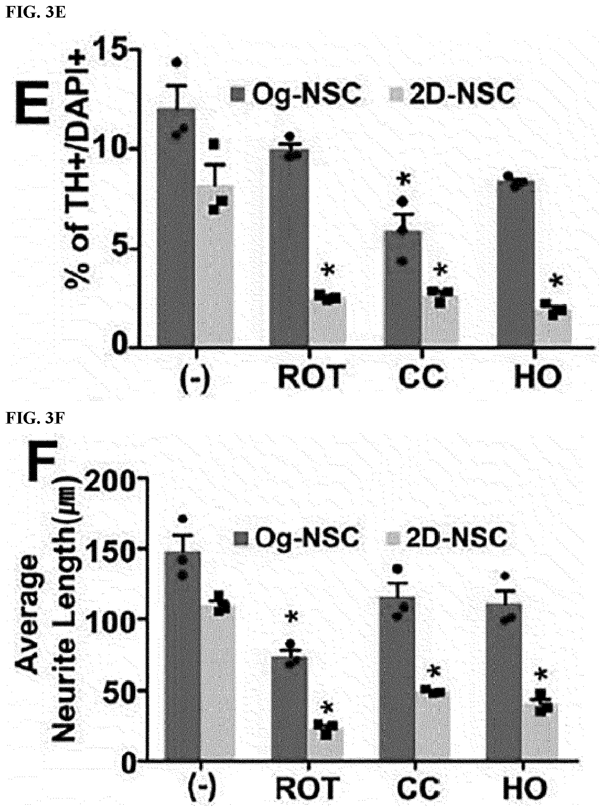

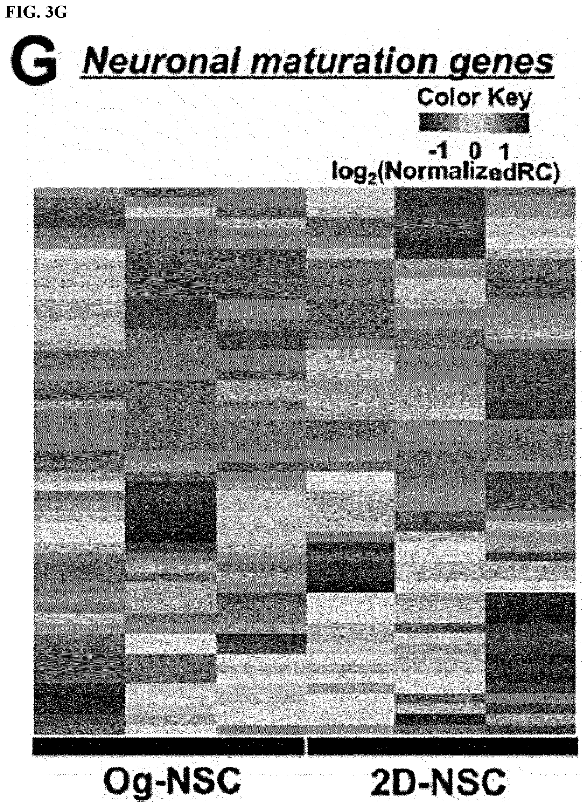

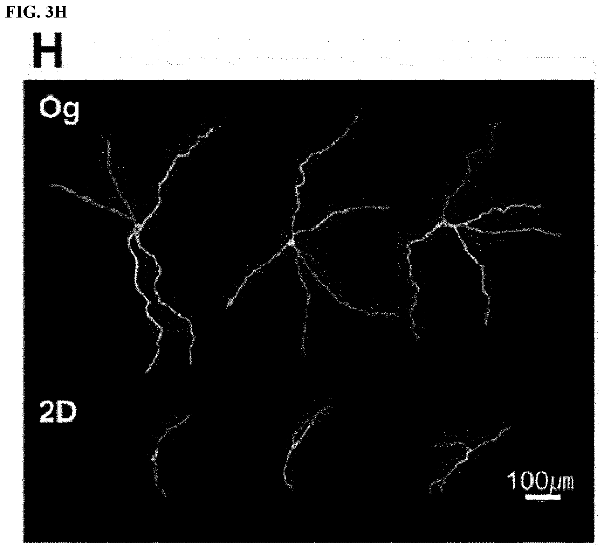

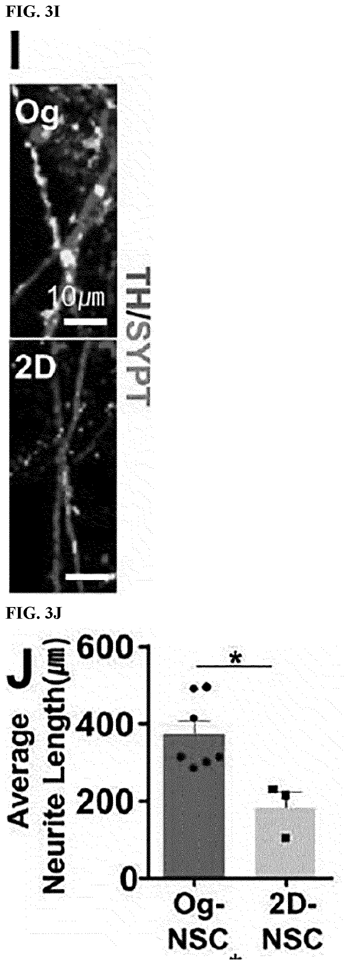

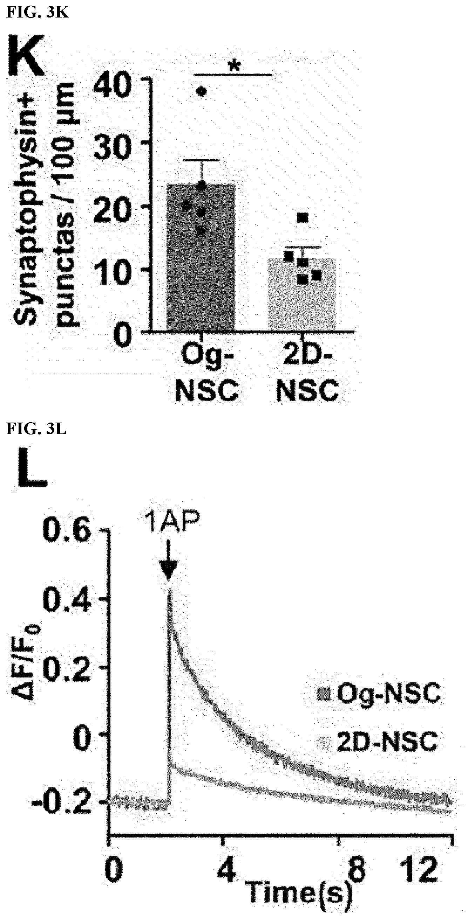

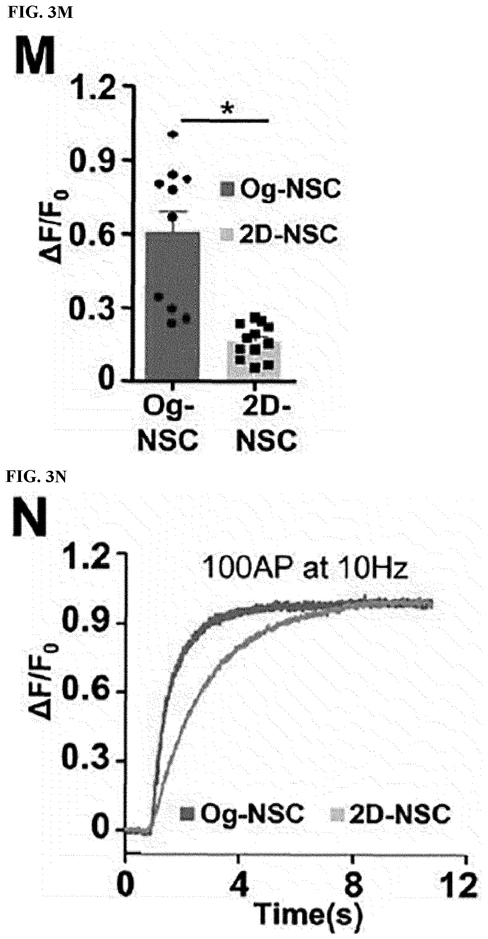

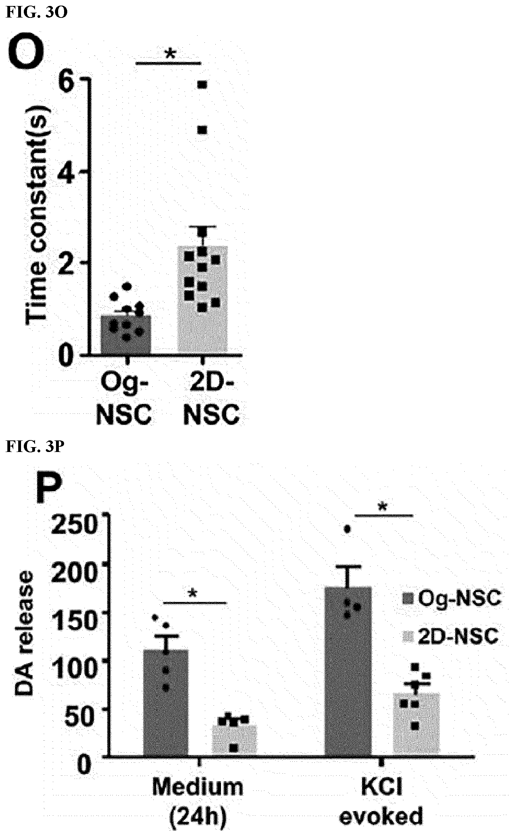

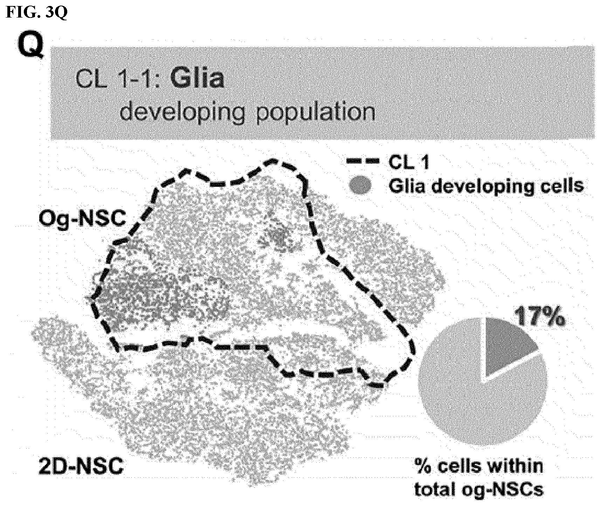

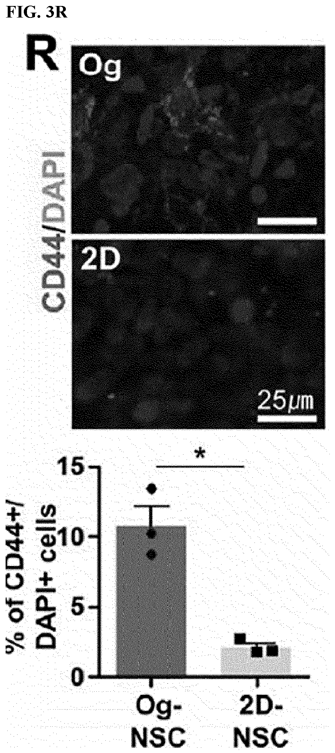

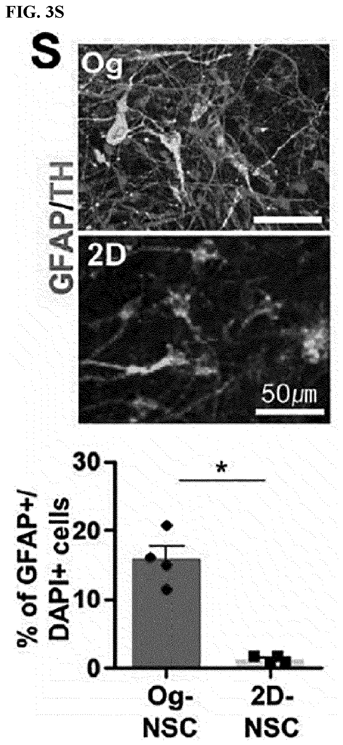

FIG. 3 shows a result obtained by analyzing the maturity and functionality of Og-NSCs and dopaminergic neurons in terms of mitochondrial function, and a result showing the difference in neuron maturity between the two groups. In conclusion, it shows that Og-NSCs can be differentiated into a healthier and more mature form compared to the NSCs produced in the past. [A-F: Neurons derived from organoid neural stem cells (Og-NSCs) maintain mitochondria in a healthy condition even under a mitochondria toxin experiment. A: STRING analysis of mitochondria factors overexpressed in Og-NSCs (vs 2D-NSCs). B: Analysis of mitochondrial synthesis (Mitochondrial biogenesis (MitoTimer)). ^{∗}p=0.0000005 (48hr), 0.024 (24hr), n=4 cultures. C: mitochondrial ROS (MitoSox), D, mitochondrial membrane potential (JC-1). ^{∗}p=1.9e-46, independent cultures, 310-314 cells (C), p=3.5e-21, 283-311 cell analysis results from three or more independent experiments (D), Student's t-test. E, F, Analysis of survival of dopaminergic neurons differentiated for 4 days under mitochondria toxin treatment. Cell survival/death (E), anaylsis in length of dopaminergic neuron (F). ROT, rotenone; CC, cccp; HO, H₂O₂ ^{∗}p=0.013-0.165 (E), p=0.000032-0.11 (F) n=3, Student's t-test. G-P: Analysis of maturity and functionality of dopaminergic neurons. G, Heatmap analysis of Og-NSCs expression-specific genes (vs 2D-NSCs). H, High-maturity dopaminergic neurons derived from Og-NSCs representatively analyzed by the Neurolucida analysis program. J, Analysis of length of dopaminergic neurons which were differentiation-induced for 12 days. The length of TH+ fibers from 23-58 cells/culture was measured. ^{∗}p=0.013, n= 7 (Og), 3 (2D) independent cultures, t-test. I, K, Analysis of synapse of dopaminergic neurons. The number of neurons that actually form synapses was analyzed by synaptophysin staining. (27-43 TH+ fibers/culture) ^{∗}p=0.03, n= 5 cultures for each group. L-O, Analysis of Ca²⁺ influx based on neuron function. L, 1AP-producing neurons from Og-NSCs and 2D NSCs were observed. Og-NSCs (red) and 2D-NSCs (black). Arrow 1AP point. M, mean values of 1 AP response amplitudes: [Ca²⁺ influx]Og = 0.60 ± 0.09 (n=10), [Ca²⁺ influx]2D = 0.16 ± 0.02 (n=12). N, Ca²⁺ influx traces by stimulation. O, mean values of Ca²⁺ influx kinetics during 100AP response: [τ of Ca²⁺ influx]3D=0.85s ± 0.11 (n=10), [τ of Ca²⁺ influx]2D=2.35s ± 0.43 (n=12). ^{∗}p=0.0009 (M), p=0.005 (O), Student's t-test. P, Pre-synaptic DA secretion, DA levels were measured as dopamine secretion in the differentiation day 13~15 medium with the conditioned medium for 24 hours. KCl-depolarization was induced for 30 minutes. ^{∗}p=0.0006-0.0009, n=4 (Og), 5-6 (2D) independent cultures. Q, R, The ability of Og-NSCs to differentiate into astrocytes. The astrocyte progenitor cell population was observed in cluster 1 of Single RNA-seq (Q). The CD44+ astrocyte progenitor cell population was included in neural stem cell population (R), and after differentiation, astrocytes (GFAP) were differentiated together with dopaminergic neurons (TH) (S). It was observed in Og-NSC group and rarely in 2D-NSC group. ^{∗}p=0.004 (R), p=0.0003 (S), n=3-4 independent cultures. Scale bars, 10 µm (I), 25 µm (B, R), 50 µm (C, D, S), 100 µm (H)].

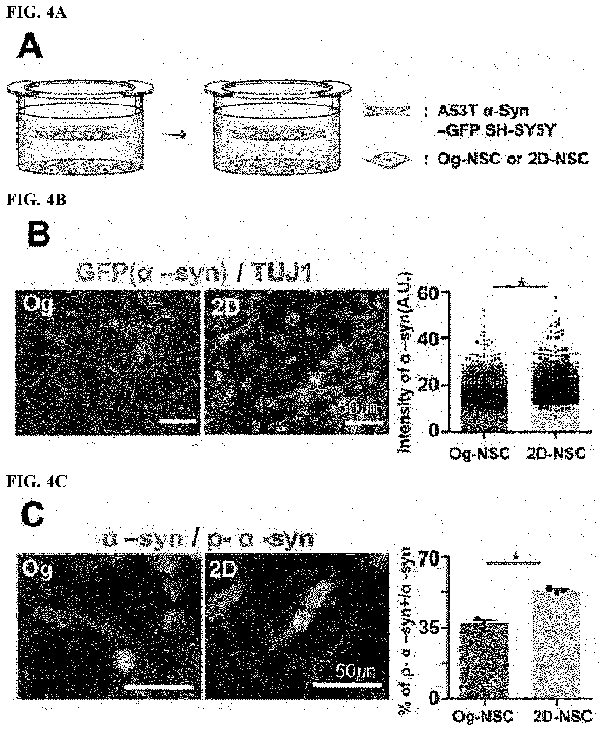

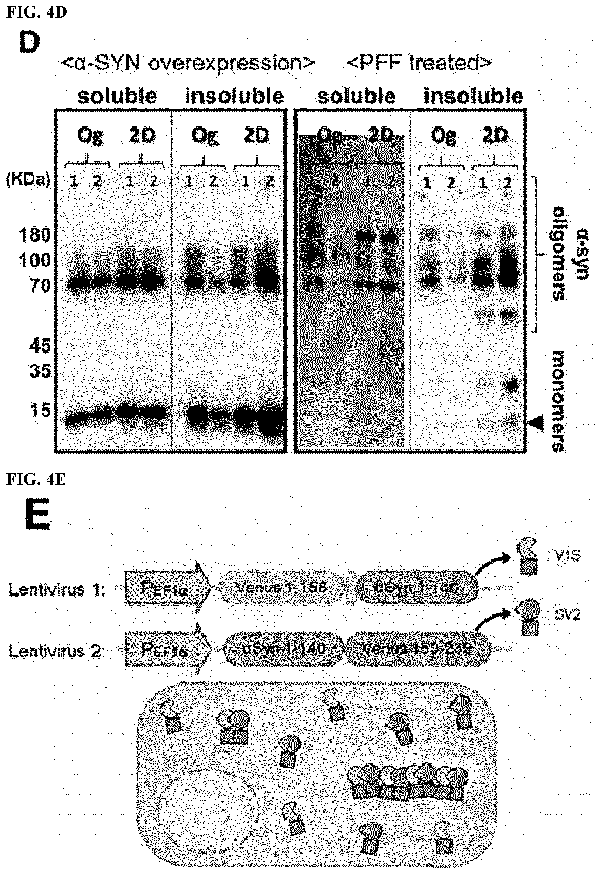

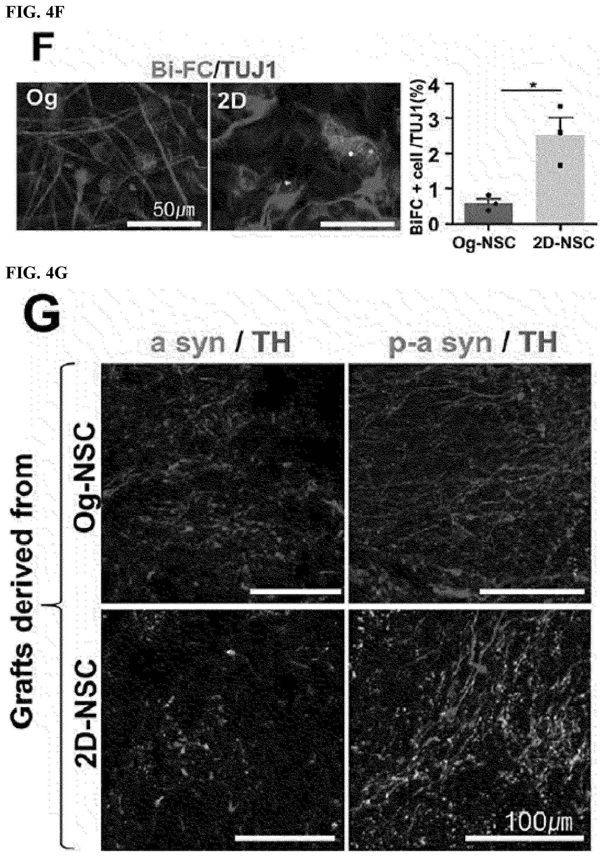

FIG. 4 shows that when observed in a model of α-synucleinopathy, which is a biological characteristic of Parkinson's disease, the neural stem cells induced by the method of the present invention are cells in a state in which α-synuclein, the etiology of severe Parkinson's disease, is not well propagated, and thus it can be said to be advantageous when used as a cell therapeutic agent for Parkinson's disease [The neurons derived from organoid-derived midbrain-type neural stem cells (Og-NSCs) were observed to have relatively less α-syn propagated. The neurons may be more resistant to α-syn disease environments. A-D, Observation of the characteristics that α-syn penetrates less into neurons. Og-NSCs (vs 2D-NSC). A, A dual chamber system capable of observing the transfer of α-syn between cells. B: α-syn intensities (GFP) were compared in 768 (Og)- and 740 (2D)- treated cells. ^{∗}p=0.000004. It was observed that α-syn penetrated relatively less in Og-NSCs. C-F: When α-syn oligomerization, which could be the etiology of Parkinson's disease, was induced by an overexpression system using virus, the p129-α-syn levels, which could be the etiology, were particularly observed relatively low in Og-NSCs. (C), Western blot analysis of α-syn oligomers after induction of virus overexpression (left) or optionally after PFF (preformed α-syn fibril) treatment (right). It was observed that α-syn oligomers, which could be the main etiology of Parkinson's disease, occured less in Og-NSCs (D), Bi-FC system (E, F). Bi-FC-green expressed inclusions were relatively less observed in Og-NSCs. This indicates that even the same amount of α-syn has a low probability of aggregation and pathogenesis (F). *significance at p=0.001 (C) and 0.02 (F), n= 3 independent experiments. G, After transplantation of Og-NSCs (left hemisphere) and 2D-NSCs (right hemisphere) into PFF-a syn model animals, respectively, histological analysis was performed. The animals were induced into the Parkinson's disease model with PFF and α-syn AAV virus for 2 weeks and sacrificed 1 month after transplantation of each cell. Relatively less malignant α-syn (p129-α-syn) was observed at the site of transplantation of Og-NSCs, and healthy dopaminergic neurons were observed. Scale bars, 50 µm (B, C, F), 100 µm (G)].

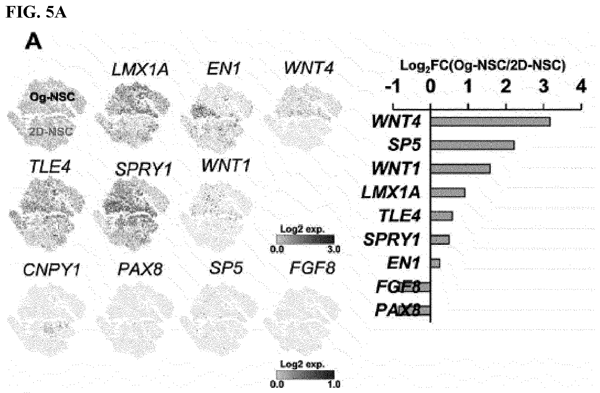

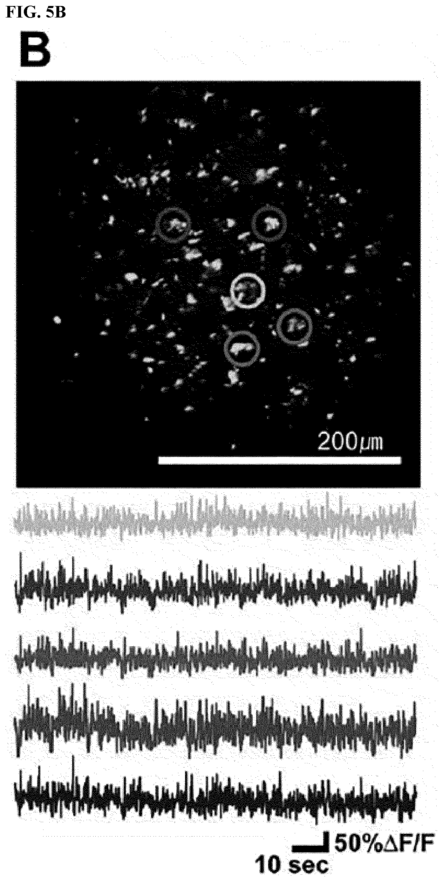

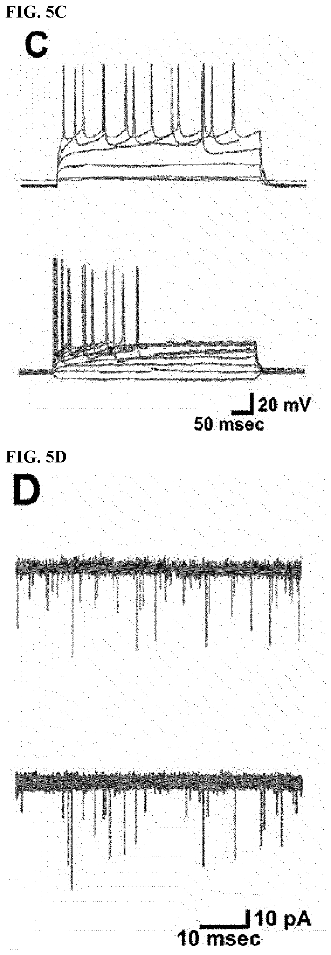

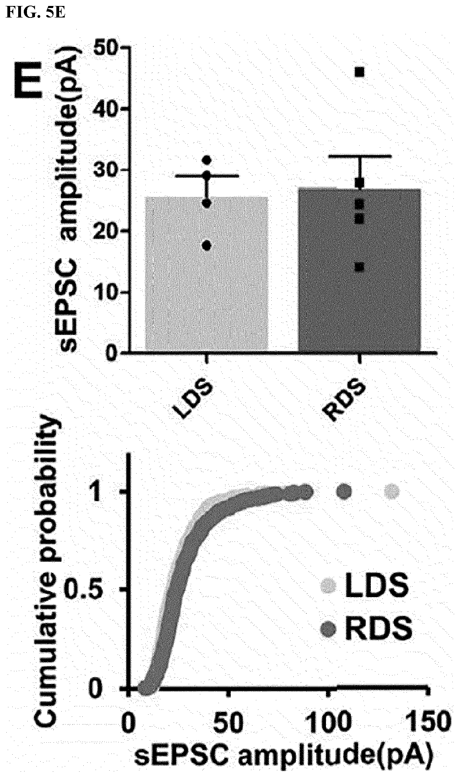

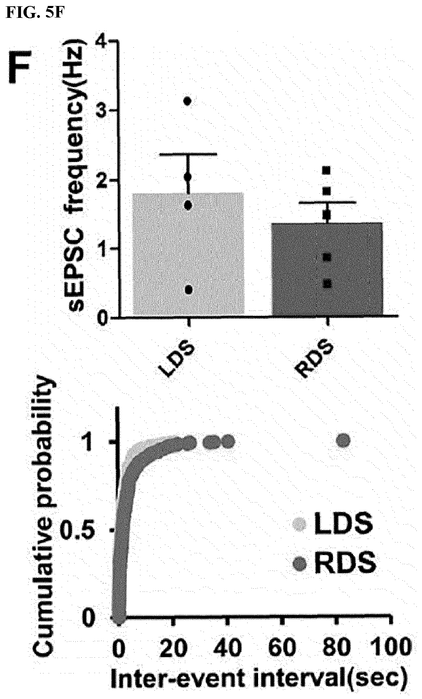

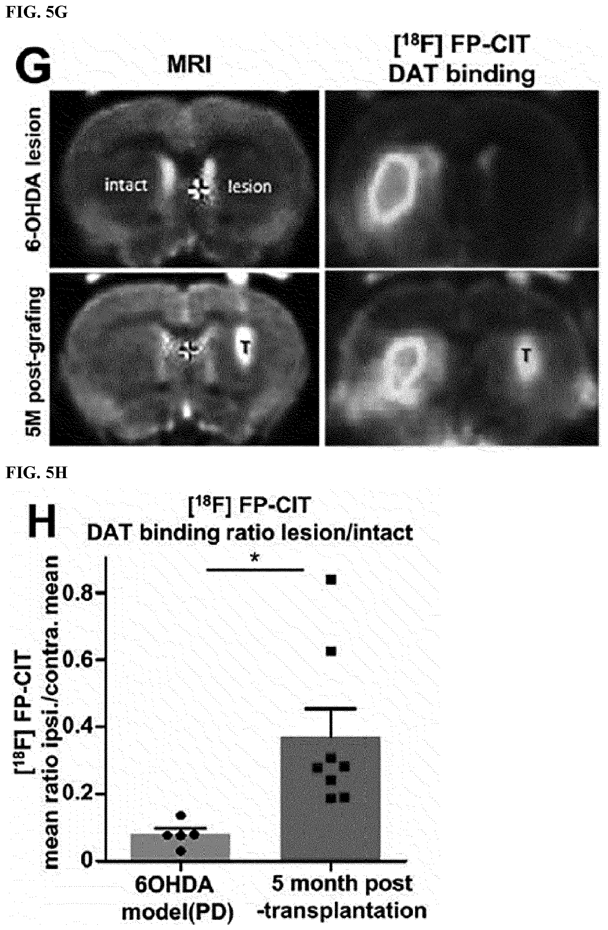

FIG. 5 shows an *in vivo* prediction study in various aspects for the results that may appear after transplantation of Og-NSCs [A: As a result of analyzing the expression of factors reported as important factors for success in transplantation of dopamine neurons from single cell RNA-seq data, Og-NSCs showed relatively high expression. (vs 2D-NSCs). The result value is expressed as the color density. It is expressed as log2 (expression of unique molecular identifier (UMI) values) (left) and as a ratio average of log2 Og/2D expression values (right). B: Results of images observed by two-photon fluorescence endomicroscopy by transplanting pSyn-GCaMP6s-expressing neurons into dorsal striatum (top). ROIs (regions of interest) of different colors of GCaMP6s fluorescence are indicated by circles in the upper image of the figure (B, bottom). C-F: Action potential firing and synaptic transmission at Og-NSC-derived transplantation sites. Og-NSCs differentiated from pDll1-GFP-hESCs and expressing fluorescence in neural stem cells were transplanted into the striatum of a hemi parkinsonian model animal. Recordings of whole-cell patch clamp from animal brain slices 1.5 months after transplantation. Intrinsic excitability was observed by depolarizing current injection of transplanted GFP+ cells (C), and sEPSCs were recorded with a -70 mV holding potential (D). The mean amplitude (E) and frequency (F) of sEPSCs from Og-NSC derived transplantation sites were compared with those of normal cells from non-transplanted sites (left). n=5 (graft) and 4 (control, unlesioned) from 2 rats. G, H: After 5 months of transplantation of Og-NSCs into 6-OHDA Parkinson's disease-induced animals, ¹⁸F FP-CIT DAT binding was analyzed by MRI and PET scans. ^{∗}p=0.021, n=8 (graft) and 5 (control, lesioned). T: transplantation site, scale bar, 200 µm.

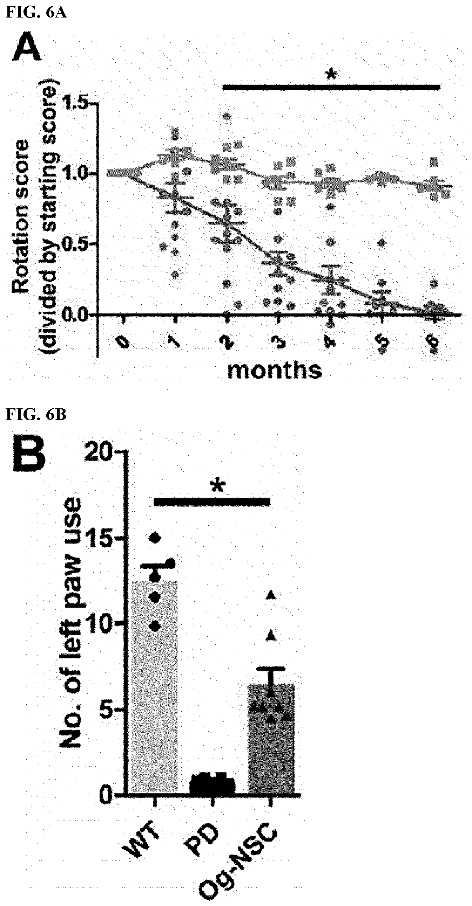

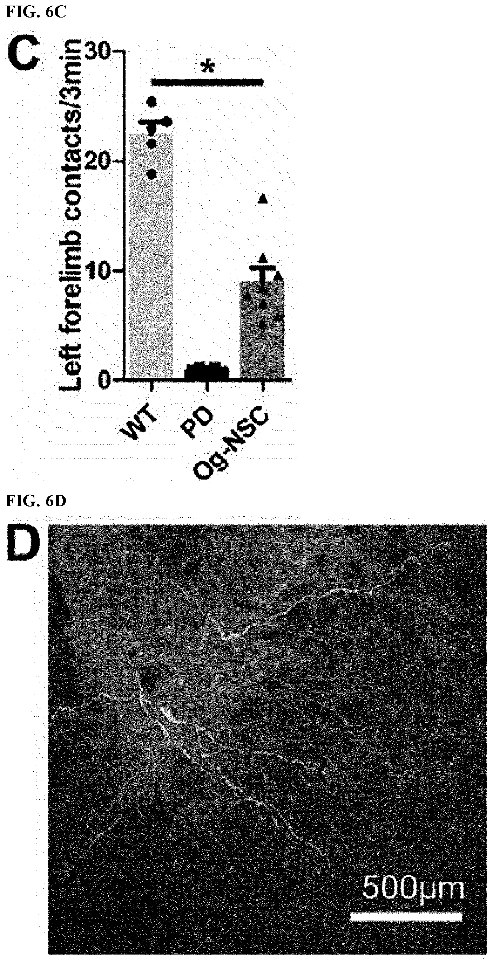

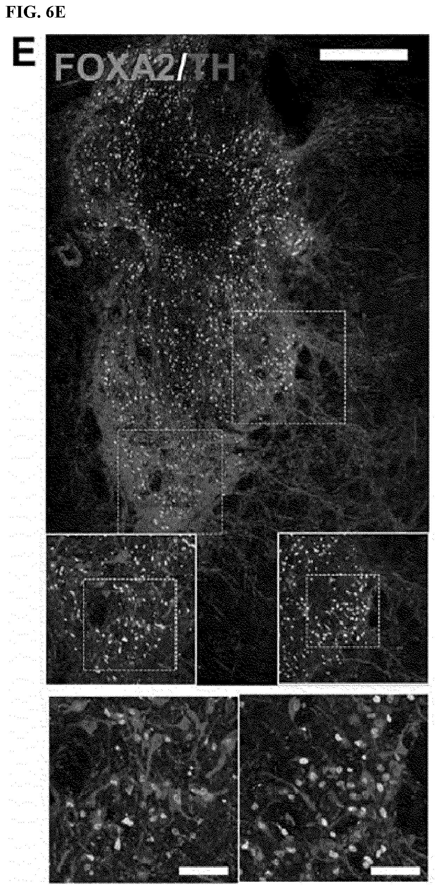

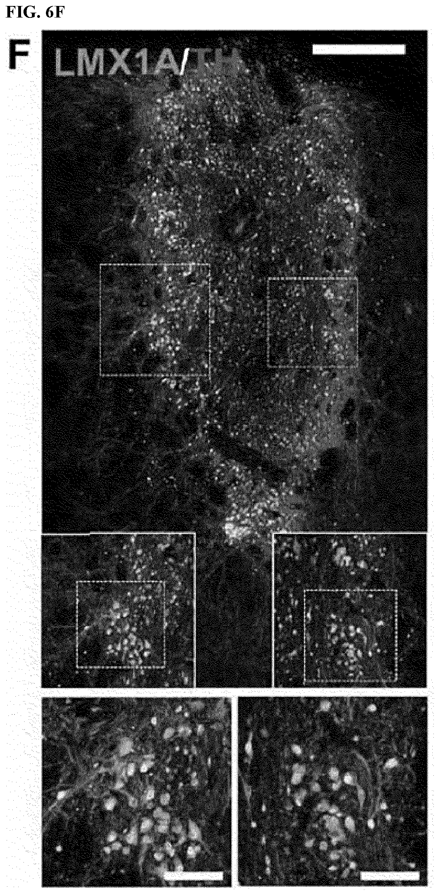

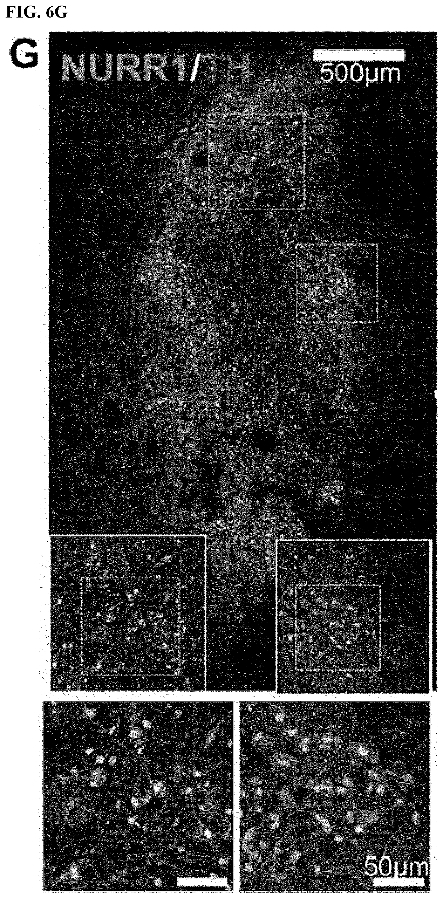

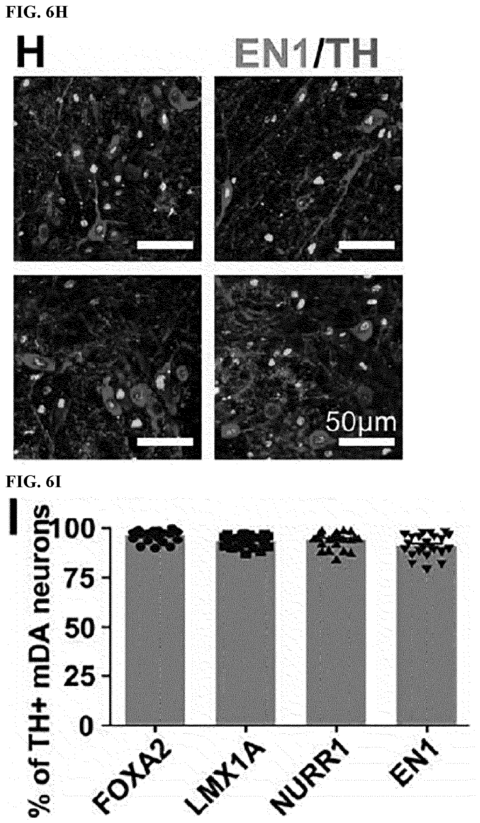

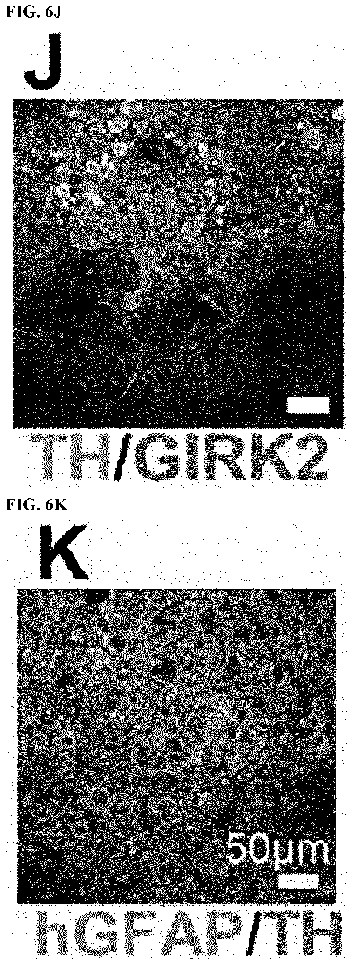

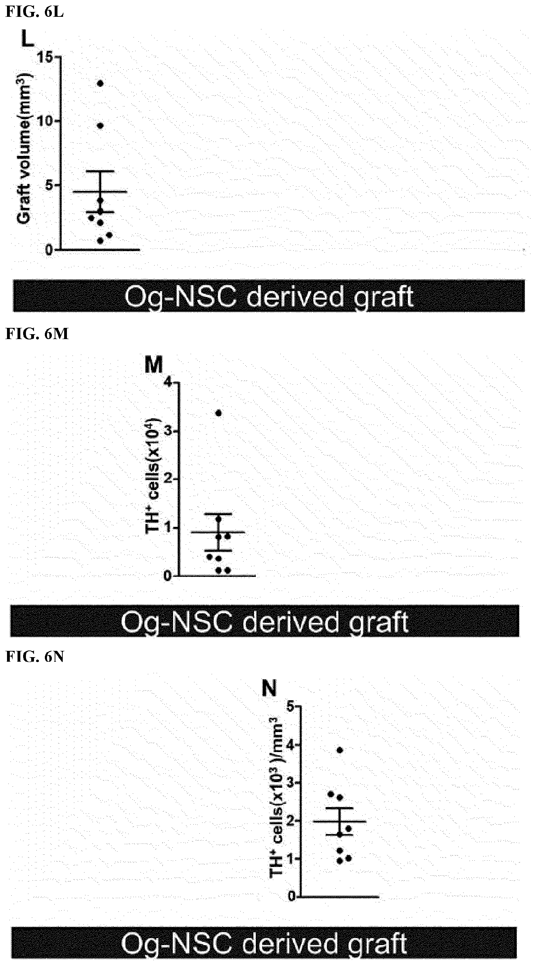

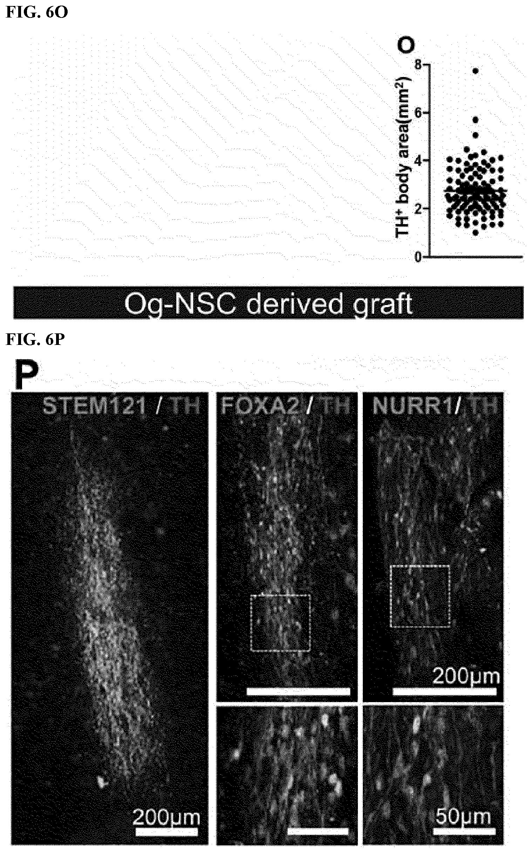

FIG. 6 shows a result observed after transplantation of Og-NSCs into an actual Parkinson's disease model animal [Og-NSCs were transplanted into 6-OHDA-inducing hemi parkinsonian model rats (A-O) and also transplanted into Philippine monkeys (P). A-C: Behavioral analysis after transplantation of Parkinson's disease animals. A: Six months after transplantation, amphetamine-induced rotation scores were observed. At the starting time point, 14 Parkinson's disease model animals were transplanted with Og-NSCs, and amphetamine-derived behavioral recovery scores were analyzed monthly for 1-6 months after transplantation. When 6 animals had a behavioral recovery score >60% for 1-4 months, the animals were sacrificed for analysis. Recovery is expressed as a percentage (%) thereof based on behavioral score analysis data before transplantation. 7 animals in the physiological saline transplantation group were used as a control group. (Analysis of sham-(PBS-injected) controls (n=7) was ^{∗}p=7.8e-10-0.037, one-way ANOVA.) Step adjustment tests were performed 6 months after transplantation as another behavioral experiment in an Og-NSC-transplanted animal model, ^{∗}p=5.58e-07-0.0006, one-way ANOVA, Tukey's post hoc analysis (B). In addition, cylinder tests were performed, ^{∗}p=1.63e-08-0.000424, one-way ANOVA, Tukey's post hoc analysis (C) (n=8). D: In a Og-NSC-transplanted animal model, six months after transplantation, the morphology of dopamine neurons was observed with a Neurite tracing (Neurolucida) image, and as a result, it was confirmed that they were differentiated into a mature form. E-O: Tissue morphological analysis was confirmed by fluorescence staining. E-G: Confirmation of TH+/FOXA2+, TH+/LMX1A+, TH+/NURR1+ dopaminergic neurons at the transplantation sites. The lower enlarged image confirms that the transplanted dopaminergic neurons are true midbrain-type neurons. I: As a result of fluorescence staining with midbrain-type factors, % expression was confirmed at the transplantation site of 8 animals (the number of 13,697 TH+ cells was analyzed). L-P: The transplantation site was calculated by volume (L), the number of TH+ neurons (M), density (N), body size (O) 6 months after transplantation (n=8 rats). P, One month after transplantation into Og-NSC-transplanted adult Philippine monkeys (cynomolgus), among TH+ dopamine neurons, the transplanted dopaminergic neurons expressing STEM121, which indicates that they are the transplanted cells derived from human cells, and expressing FOXA2 and NURR1 as midbrain-type markers were confirmed. Scale bars, 50 µm (E-G bottom, H, J, K, P bottom), 200 µm (P top), 500 µm (D-G)].
FIG. 7 shows the generation and property of midbrain-like organoids from hPSCs [A: A schematic diagram of the experimental procedure for generating midbrain-like organoids. B-D, Phase contrast images of organoids over days of *in vitro* (DIV) culture. Scale bar, 200 µm. E-O: Representative immunofluorescence images of nerve structures formed in organoids. The organoids were cryosectioned *in vitro* on the indicated days and stained with the indicated antibodies. V, ventricle; VZ, ventricular zone; IZ, intermediate zone; MZ, mantle zone, scale bars, 100 µm (E-P), 200 µm (B-D)].
FIG. 8 shows that the maintenance of midbrain neural stem cells is efficiently improved by controlling the treatment time of FGF8, which is important for midbrain neurogenesis, by the method of the present invention [A: FGF8b treatment scheme of protocols A and B. B: RNA-seq data for expression of ventral midbrain (VM)-specific and hypothalamus-specific markers in organoid (Og)-NSCs calculated by two different FGF8b treatment schemes. C: Representative images of EN1+/TH+ mDA neurons induced with protocols A and B. Scale bar (50 µm)].

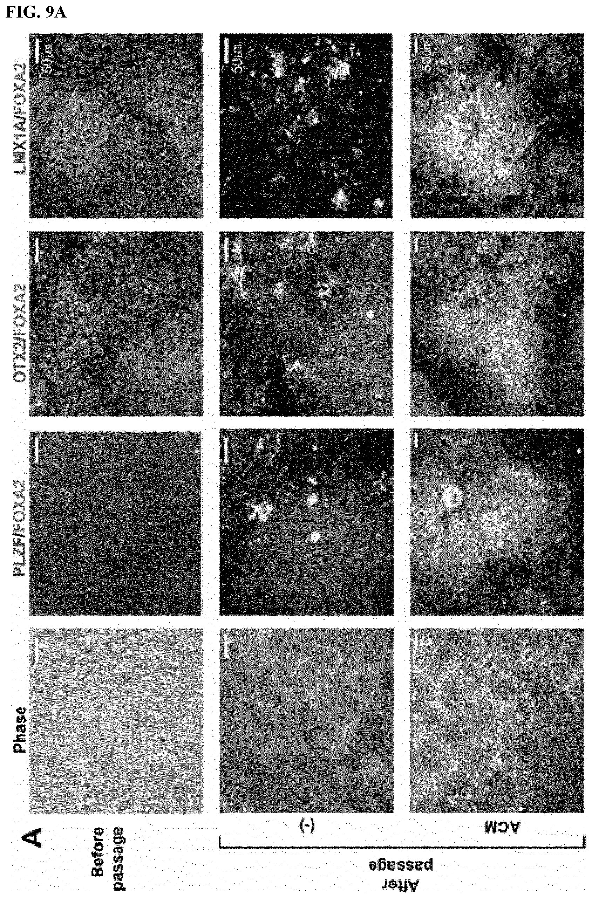

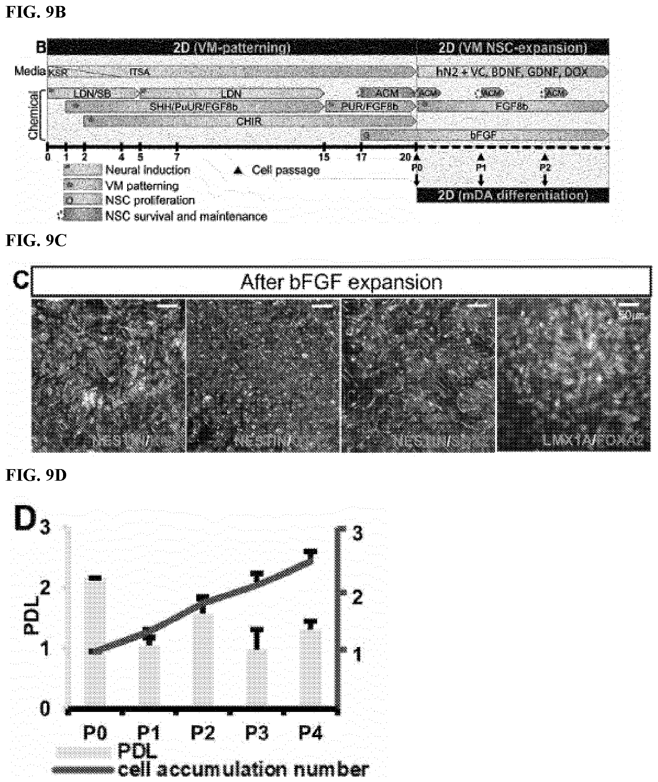

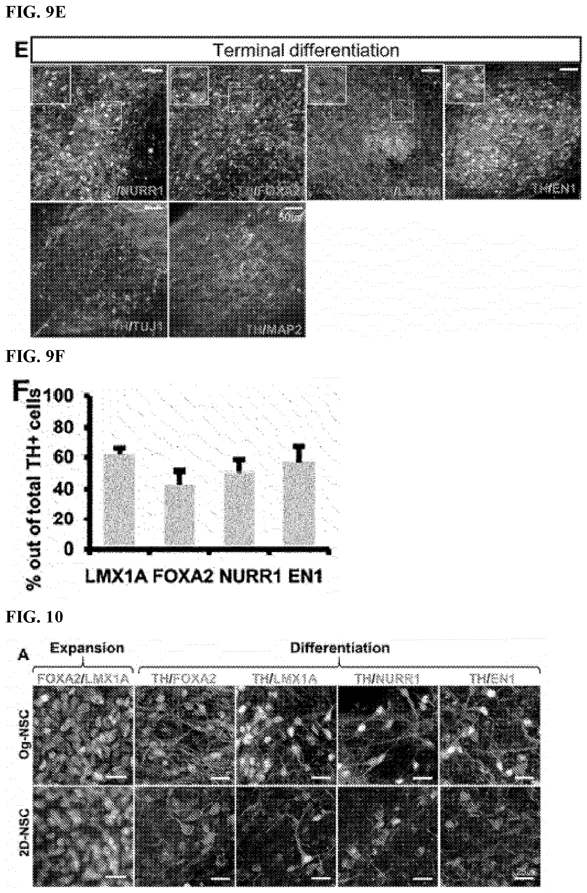

FIG. 9 shows 2D-NSC generation from ventral midbrain (VM) neural stem/progenitor cells (NSCs) or hPSCs in a 2D culture environment [A: Requirement of astrocyte conditioned medium (ACM) treatment during cell passage of hPSCs differentiated in 2D. In order to prepare NSCs, hPSCs differentiated with VM-patterning chemical treatment (before top, upper panel) in the absence (-) or presence (+) of ACM were isolated and plated (after passage). Images of phase contrast and immunofluorescence staining of cells expressing the indicated midbrain-specific factors are presented. B: Schematic overview of the 2D culture-based hPSC-NSC/mDA neuron differentiation protocol. C: Immunofluorescence staining 2 weeks after VM-specific NSC marker expression NSC expansion in 2D-NSCs. D: Cell expansion of 2D-NSCs expressed by population doubling level (PDL, bar) and cumulative PDL (line). E-F: mDA neurons were generated from Og-NSCs at 12-15 days after differentiation. Midbrain-specific marker expression in DA neurons (% of TH+ cells) was quantified from three independent experiments (F). Scale bar, 50 µm].
FIG. 10 shows representative images of neural stem/progenitor cells (NSCs) and mDA neurons expressing midbrain-related markers in organoid- (top) and 2D-based cultures (bottom) [scale bar, 50 µm].

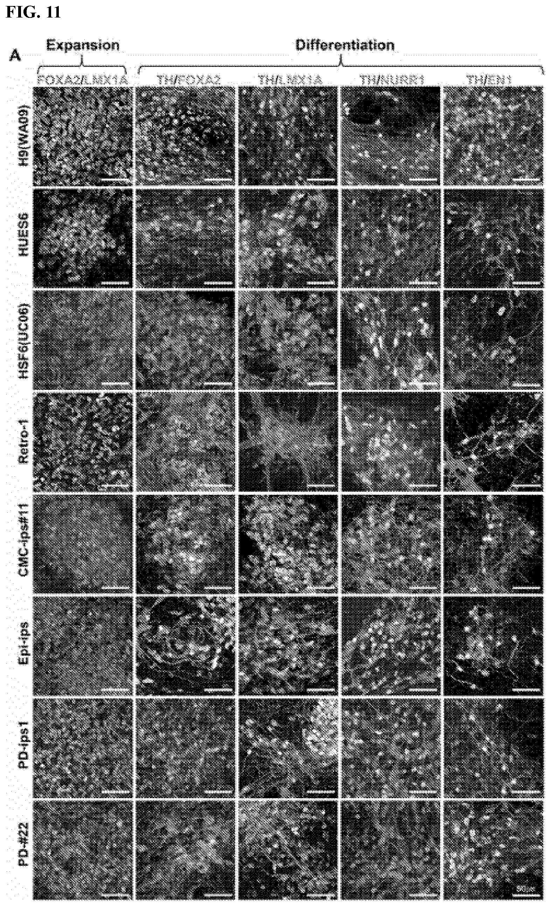

FIG. 11 illustrates a result showing that the developed protocol is a protocol that is universally applicable to human embryonic stem cells/induced pluripotent stem cells and various stem cell lines [Ventral midbrain (VM) neural stem/progenitor cells (NSCs) and mDA neurons expressing midbrain-specific markers were generated across all hPSC lines tested. 3 hESC and 4 hiPSC lines were induced to differentiate into VM-type NSCs using the organoid protocol outlined in FIG. 1A. NSCs were expanded with 2-3 cell passages and final differentiation from NSCs into mDA neurons was induced. It depicts representative immunostaining images for FOXA2 +, LMX1A + NSCs in NSC stage (expansion) and TH+ mDA neurons expressing the midbrain markers FOXA2, LMX1A, NURR1 and EN1 at 7-12 days after terminal differentiation. Scale bar, 50 µm].

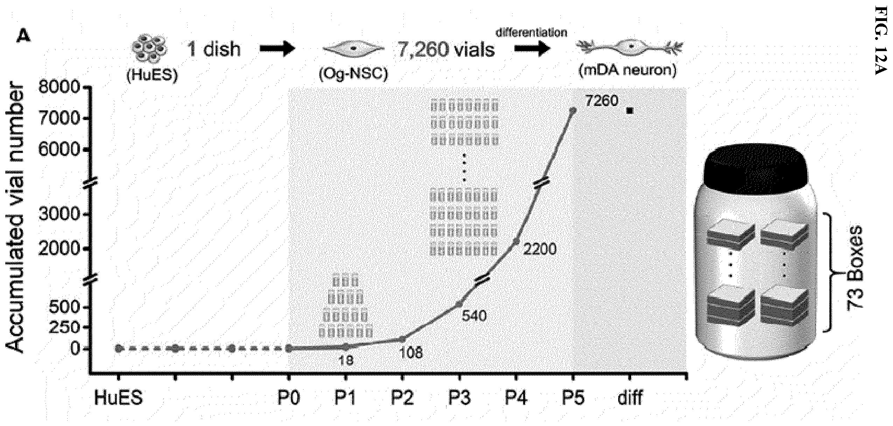

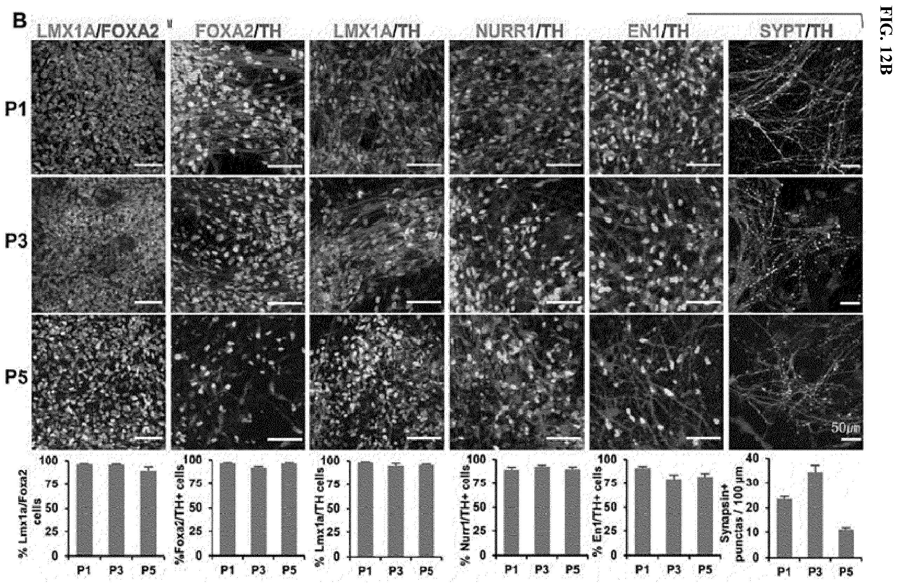

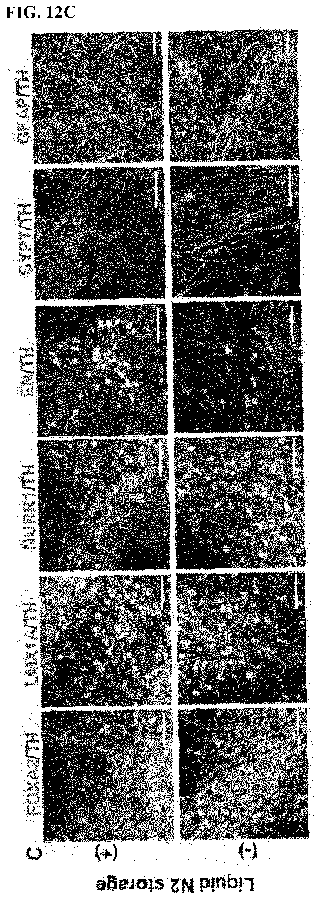

FIG. 12 illustrates a result showing that organoid (Og)-neural stem/progenitor cells (NSCs) are expandable to human mDA neurons, and in terms of amount or maintaining its cell biological properties, can be supplied enough to be used as a universal cell therapeutic agent. [A: Schematic overview of an organoid-based protocol to generate 7,260 vials (9,075 dishes) of mDA neurons from 1 dish of undifferentiated hPSCs using NSC expansion with 5 cell passages. B: Maintenance of mDA neurogenic capacity of Og-NSCs during multiple NSC passages. The upper drawing depicts representative immunostaining images of FOXA2+, LMX1A+ NSCs at NSC stage (expansion) and TH+ mDA neurons expressing the midbrain markers FOXA2, LMX1A, NURR1 and EN1 at 10-18 days after terminal differentiation. Midbrain marker expression in NSC and mDA neurons was quantified in the lower graph. Independent experiments (n = 3). C: Storage of Og-NSCs in liquid N₂ without altering the mDA neurogenic potential. Representative immunostaining images of mDA neurons differentiated from H9 Og-NSCs with (+) and without (-) liquid N₂ storage are depicted. Scale bar, 50 µm].

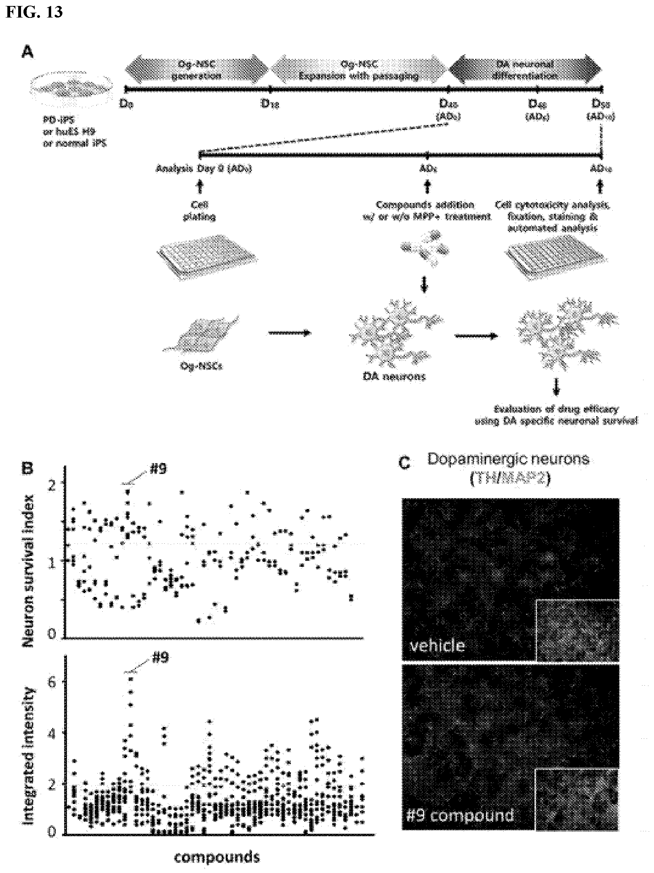

FIG. 13 shows the screening of candidate compounds of PD drugs using mDA neurons derived from PD-iPSCs [A: A schematic diagram of the experimental procedure. hiPSCs derived from PD patients (PD-ips1) were differentiated into mDA neurons using an organoid-based protocol. Cell survival of PD-iPSC-mDA neurons was evaluated in the presence of compounds using MTT analysis and immunofluorescence intensity-based counting of TH- positive DA neurons. A high content screening (HCS) analysis using Image Express Microconfocal (Molecular Devices, San Jose, CA) was used for TH intensity-based mDA neuron survival analysis. B: Examples of screening data obtained by MTT analysis (top) and TH+ intensity measurement (bottom). Yellow line, mean value of a control group (vehicle). C: Representative TH/MAP2 images of PD-iPSC derived mDA neurons at day 10 using vehicle (top) and compound #9].
FIG. 14 summarizes the development of mDA neurons and the properties of the cells in developmental midbrain (VM) of organoid (Og) neural stem/progenitor cell (NSC) differentiation according to subculture. Terminal differentiation of Og-NSCs was induced by the withdrawal of bFGF and the presence of neurotrophic factors BDNF, GDNF and cAMP Immunocytochemical analysis was performed on the indicated differentiation days using antibodies specific for proliferating cells (Ki67), mature neurons (MAP2), DA neurons (TH) and midbrain (FOXA2, LMX1A, NURR1). Scale bar, 50 µm.
FIG. 15 shows single cell RNA expression patterns of neural stem/progenitor cell (NSC)- (A), pluripotent cell- (B) and cell senescence-specific genes (D) in organoids (Og) and 2D-NSCs [low expression levels of the multipotent genes SSEA4 and TRA1-60 were further evaluated by FACS analysis (C)].
FIG. 16 shows a detailed protocol for isolating cerebral cortex neural stem cells using 3D cerebral cortex organoids.
FIG. 17 shows that neural stem cells are obtained using 3D cerebral cortex organoids [left graph: Suitability of the differentiation protocol was confirmed through expression of pax6, a cerebral marker. right: In the upper photograph, before chopping of organoids, the formation of organoids in the correct form was confirmed with a photograph. In the lower photograph, after chopping, they were confirmed to be grown in the form of normal neural stem cells].
FIG. 18 shows the final established direct differentiation protocol from 2D and 3D derived culture into midbrain-neural stem cells [A: Establishing the stepwise technology of direct differentiation from embryonic stem cells into midbrain-neural stem cells using human embryonic stem cells, securing of astrocyte progenitor cells, and then terminal differentiation into astrocytes, B: Protocol for securing cerebral cortex derived astrocytes. (A) differs from (B) in its patterning method].
FIG. 19 shows the preparation of astrocytes using a direct differentiation protocol from 2D- and 3D- derived culture into midbrain-neural stem cells. It shows that the cells derived by the method of the present invention are healthier and have improved marker maintenance compared to the conventional two-dimensional culture method.
FIG. 20 shows a result obtained by analyzing the properties of cerebral cortex-type and midbrain-type astrocytes cultured *in vitro.* It shows that midbrain derived astrocytes are superior in genetic analysis in terms of the actual support function of neurons between the two astrocytes.
FIG. 21 shows a result obtained by analyzing the properties of cerebral cortex-type and midbrain-type astrocytes after transplantation *in vivo.* It shows that midbrain derived astrocytes are superior in genetic analysis in terms of the actual support function of neurons between the two astrocytes.
FIG. 22 shows the change of microglia into high-quality M2 type cells from transplantation of astrocyte progenitor cells *in vivo.* It can be expected that they will have great utility since the environment can be improved when transplanting astrocytes in the future.
FIG. 23 shows a result of an adaptive culture of other types of induced pluripotent stem cells using the xeno-free system. This is a basic result because it is important to apply the xeno-free system to various cell lines when actually developing a cell therapeutic agent.
FIG. 24 shows confirmation of the protocol application from other types of induced pluripotent stem cells (CMC-iPSC#11) into the midbrain-type astrocyte group (left) and confirmation of differentiation of normal neurons (right).
FIG. 25 shows a result obtained by confirming the protocol application of other types of induced pluripotent stem cells (CMC-iPSC#11).
FIG. 26 shows a result obtained by confirming the protocol application through efficient differentiation induction using TGF-β and LIF.
FIG. 27 shows a result obtained by analyzing the physiological properties of the generated midbrain astrocytes.
FIG. 28 shows a result obtained by observing changes in microglia using a conditioned medium for midbrain-type astrocytes. Through this, the anti-inflammatory effect by astrocytes was verified *in vitro.*
FIG. 29 shows a result obtained by confirming the possibility of inhibiting death factors with an astrocyte conditioned medium after induction of inflammation. Through this, the anti-inflammatory effect by astrocytes was verified *in vitro.*
FIG. 30 shows a result obtained by confirming the anti-inflammatory effect of an astrocyte conditioned medium after induction of inflammation using RT-PCR. Through this, the anti-inflammatory effect by astrocytes was verified *in vitro.*
FIG. 31 shows a result obtained by analyzing RNA expression related to non-transplanted sites and inflammatory response after transplantation of astrocytes into the striatum. Through this, the anti-inflammatory effect by astrocytes was verified *in vivo.*
FIG. 32 shows the expression of the M1 marker iNOS after transplantation of astrocytes *in vivo.* It was confirmed that less inflammation occurred compared to the neural stem cell transplantation site.
FIG. 33 shows the expression of the M1 marker CD11b after transplantation of astrocytes *in vivo.* It was confirmed that less inflammation occurred compared to the neural stem cell transplantation site.
FIG. 34 shows the expression of the M1 marker CD16.32 after transplantation of astrocytes *in vivo.* It was confirmed that less inflammation occurred compared to the neural stem cell transplantation site.
FIG. 35 shows the expression of the M2 marker CD206 after transplantation of astrocytes *in vivo.* It was confirmed that less inflammation occurred compared to the neural stem cell transplantation site.
FIG. 36 shows a detailed protocol for isolating hypothalamus neural stem cells using 3D organoids.
FIG. 37 shows a result obtained by securing 3D hypothalamus organoids and confirming the expression of a marker suitable for this. As specific markers, the expression of nestin, Rax, and Sox2 occurs well.
FIG. 38 shows a result obtained by confirming that hypothalamus neural stem cells which are capable of being proliferated are obtained [A: Securing neural stem cells capable of being proliferated up to passage 4 expressing 97% nestin/Rax, a hypothalamus neural stem cell marker; B: Confirmation of the expression of hypothalamus-related genes in hypothalamus neural stem cells].
FIG. 39 shows a result obtained by confirming the differentiation of normal neurons and astrocytes after induction of differentiation of hypothalamus neural stem cells.
FIG. 40 shows a result obtained by confirming the differentiation into cells responsive to leptin after induction of differentiation from hypothalamus neural stem cells. This function analysis shows the functional characteristics of hypothalamus cells well, and the expression of NPY and AGRP, hypothalamus neuron markers, is increased after differentiation. Therefore, it was confirmed that patterning into desired hypothalamus neural stem cells was well performed both functionally and in terms of marker expression.
FIG. 41 shows that hypothalamus neural stem cells developed by using 3D organoids express more marker gene groups *in vivo* than those developed in a two-dimensional 2D environment.
FIG. 42 shows a result obtained by confirming the possibility of *in vivo* transplantation of hypothalamus neural stem cells developed by using 3D organoids.

### Best Mode for Carrying out the Invention

Hereinafter, the present invention will be described in more detail.

The present invention relates to a method of patterning and chopping 3D organoids prepared from human pluripotent stem cells, culturing the stem cells or progenitor cells, and inducing the differentiation thereof to obtain a large amount of finally differentiated cells.

As used herein, the term "pluripotent stem cell (PSC)" refers to a stem cell capable of inducing differentiation into any type of cell constituting the body, and the pluripotent stem cell includes an embryonic stem cell (ESC) and an induced pluripotent stem cell (Ipsc, dedifferentiated stem cell).

As used herein, the term "organoid" refers to a 'mini-organ like' made to have a minimum function using stem cells, and is characterized in that it is made in a three-dimensional structure and may create an environment similar to an actual body organ even in a laboratory. That is, "organoid" refers to a cell having a 3D stereostructure, and refers to a model similar to organs such as nerves and intestines prepared through an artificial culture process that is not collected or acquired from animals and the like. The origin of the cells constituting it is not limited. The organoid may have an environment that is allowed to interact with the surrounding environment in the process of cell growth. Unlike 2D culture, 3D cell culture allows cells to grow in all directions *ex vivo.* Accordingly, in the present invention, the 3D organoid may be an excellent model for observing the development of therapeutic agents for diseases and the like by almost completely mimicking the organs that actually interact *in vivo.*

An organoid may generally be prepared by culturing human pluripotent stem cells. Specifically, it may differentiate from induced pluripotent stem cells derived from Parkinson's disease into neuroectodermal spheres, or differentiate into intestinal organoids through stepby-step differentiation from induced pluripotent stem cells derived from Parkinson's disease into definitive endoderm and hindgut.

As used herein, the term "differentiation" refers to a phenomenon in which the structure or function of cells is specialized during division and proliferation of cells and growth of the entire individual. In other words, it refers to a process of a change into a suitable form and function of cells, tissues, and the like of organisms in order to perform their respective given roles. For example, differentiation may include a process in which pluripotent stem cells are transformed into ectoderm (cerebral cortex, midbrain, hypothalamus, etc.), mesoderm (yolk sac, etc.) and endoderm cells, as well as a process in which hematopoietic stem cells are transformed into red blood cells, white blood cells, platelets, and the like, that is, a process in which progenitor cells express specific differentiation traits.

The existing method of inducing differentiation directly from human pluripotent stem cells to differentiate them into the corresponding cells has a problem in reproducibility depending on the cell line or laboratory (experiment environment), and does not achieve stable expression of maintenance factors. For example, in the case of midbrain dopamine neurons, unlike the actual *in vivo* environment, astrocytes do not exist together, and there is a limit to the amount of final cells that may be obtained through one differentiation. As in one embodiment of the present invention, by patterning and preparing organoids capable of sufficiently containing cerebral astrocyte progenitor cells, midbrain astrocyte progenitor cells, midbrain dopaminergic neural stem cells, or hypothalamus neural stem cells, respectively (target cell enriched), that is, securing organoids containing the maximum amount of each target cell, and chopping the organoid tissues to culture the corresponding stem cells or progenitor cells, as compared to the two-dimensionally differentiation-induced cell population, they may be more similar to the cells isolated from the actual brain, their properties may be well maintained, and it is possible to obtain differentiation into a cell population in which viability is obtained.

As used herein, the term "patterning" refers to the preparation of an organoid such that, when preparing an organoid, the cell population with the fate having the property of the origin tissue of the cell to be finally extracted from among the brain detailed tissues in this way, such as midbrain-type pattern, cerebral cortex-type pattern, or hypothalamus-type pattern, is contained as a plurality of cell populations (target cell enriched). In addition, patterning markers include Lmx1a, Foxa2, Nurr1, and En1 for midbrain-type patterning; Pax6 and Foxg1 for cerebral cortex-type patterning; and Rax and Nkx2.2 for hypothalamus-type patterning, and the like.

Therefore, in the present invention, the finally differentiated cells may be obtained in a large amount at once by chopping the prepared 3D organoids, and separating stem cells capable of being proliferated from the organoids and culturing the same to proliferate them.

According to one embodiment of the present invention, it includes a method, which chops the 3D organoids prepared from human pluripotent stem cells to obtain a large amount of stem cells or progenitor cells and finally differentiates them, comprising:
1) proliferating and culturing human pluripotent stem cells to prepare 3D organoids;
2) patterning and chopping the prepared 3D organoids; and
3) culturing and proliferating stem cells or progenitor cells in the cells extracted from the chopped organoids, and inducing the differentiation thereof to obtain a large amount of the finally differentiated cells.

The stem cell may be an ectodermal stem cell (skin and nerve cells), a mesodermal stem cell (muscle and bone cells), or an endodermal stem cell (digestive and respiratory cells). Specifically, it may be a midbrain dopaminergic neural stem cell or a hypothalamus neural stem cell.

The progenitor cell may be a cerebral astrocyte progenitor cell or a midbrain astrocyte progenitor cell, but is not limited thereto.

The cell may be a neuron or an astrocyte, but is not limited thereto.

More preferably, the human pluripotent stem cells and stem cells are cultured using vitronectin. In this regard, it is appropriate to use vitronectin at 0.3 to 0.7 µg/cm². Up to now, according to the report of CellStemCell of Parmar group (A. Kirkeby et al., Predictive Markers Guide Differentiation to Improve Graft Outcome in Clinical Translation of hESC-Based Therapy for Parkinson's Disease. Cell Stem Cell 20, 135-148 (2017).), Nature Protocol (S. Nolbrant, A. Heuer, M. Parmar, A. Kirkeby, Generation of high-purity human ventral midbrain dopaminergic progenitors for in vitro maturation and intracerebral transplantation. Nature protocols 12, 1962-1979 (2017)), which has reported the latest research on mass-producing dopaminergic neurons for transplantation, it was reported that it is very important to use Lamin521 (BioLamina LN-521) at 0.5 µg/cm² for proliferation and culture of human embryonic stem cells and to use Lamin511 (BioLamina LN-511) at 1 µg/cm² after differentiation into midbrain neurons. In contrast, in the Examples of the present invention, vitronectin (Gibco A31804) at 0.5 µg/cm² was used for both culturing of human embryonic stem cells and culturing of midbrain neural stem cells. In terms of the coating unit price of a 60 mm dish generally used for culture, the price of Lamin521 for culturing human embryonic stem cells is 7,282 won/dish, the price of vitronectin in the present invention is 1,480 won/dish, and the price of Lamin511 for culturing midbrain neural stem cells is 23,832 won/dish, whereas the price of vitronectin continuously used in the present invention is 1,480 won/dish. Even if only the coating cost is taken into consideration, the method of the present invention has an effect of saving 5 times when culturing human embryonic stem cells and 16 times when culturing midbrain neural stem cells.

As used herein, the term "large amount" means that it is possible to obtain about 1,000 to 7,200 vials of frozen cells when introduced from the start of one culture dish of pluripotent stem cells used for the first time although there is a difference depending on each patterned cell population, and refers to an amount increased by about 1,000 to 14,000 times when one or two culture dish cells are generally frozen in one vial. In particular, it includes not only simple quantitative proliferation, but also maintenance of properties.

As used herein, the term "glia" refers to a cell that occupies the largest portion of cells present in the brain, and refers to an "astrocyte." The astrocyte is also referred to as an microglia and is involved in the protection of neurons and supply of nutrients and inflammation, and the microglia are cells responsible for inflammation in the brain.

The astrocytes may be obtained by differentiation from embryonic or adult stem cells and may be also obtained by isolation from midbrain (ventral midbrain), cerebral cortex (cortex) or lateral ganglionic eminence (striatum anlage) in mammals.

"Neural stem/precursor cells (NSCs/NPCs)" are isolated and cultured from brain tissue in developing or adult brain tissue, and the cultured NPCs may be used for mass formation of dopamine neurons for research and drug screening.

A "nerve cell" is a cell of the nervous system, and the term "neuron" or "neuron cell" may be used interchangeably.

"Dopamine (DA) nerve cell" refers to a nerve cell expressing tyrosine hydroxylase (TH). In the present invention, "dopaminergic nerve cell," "dopamine neuron," "DA," and the like are used interchangeably. Dopamine nerve cells are specifically located in the midbrain substantia nigra, and stimulate the striatum, limbic system, and neocortex *in vivo* to control postural reflexes, migration, and reward-related behaviors.

The present invention also includes a cell therapeutic agent comprising cells obtained by the above method.

"Cell therapeutic agent" is a drug used for treatment, diagnosis, and prevention with cells and tissues prepared through isolation, culture, and special modification from a subject (U.S. FDA regulations). It refers to a drug used for the purpose of treatment, diagnosis, and prevention through a series of actions such as proliferating or sorting living autologous, allogeneic, or xenogeneic cells *ex vivo,* or changing the biological properties of cells in other ways to restore the function of cells or tissues. Cell therapeutic agents are largely classified into somatic cell therapeutic agents and stem cell therapeutic agent according to the degree of cell differentiation.

As used herein, the term "subject" may be a vertebrate to be treated, observed or experimented, preferably a mammal, for example, cattle, pigs, horses, goats, dogs, cats, rats, mice, rabbits, guinea pigs, human, and the like.

As used herein, the term "treatment" refers to any action that inhibits, alleviates, or advantageously alters a clinical situation related to a disease. In addition, treatment may mean increased survival compared to the expected survival rate if not receiving treatment. Treatment simultaneously includes prophylactic means in addition to therapeutic means.

The cell therapeutic agent of the present invention exhibits a therapeutic effect on a disease selected from the group consisting of a neurodegenerative disease, an inflammatory degenerative disease, and a metabolic disease.

The neurodegenerative disease may include, for example, one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease, and Pick disease, but is not limited thereto.

The inflammatory degenerative disease may include one selected from the group consisting of dementia, Lewy body dementia, frontotemporal dementia, white matter degeneration, adrenoleukodystrophy, multiple sclerosis, and Lou Gehrig's disease, but is not limited thereto.

The metabolic disease may include one selected from the group consisting of diabetes mellitus, obesity, and a metabolic disorder, but is not limited thereto.

The cells obtained by the method of the present invention may be applied as a cell therapeutic agent, and may be formulated by further including a pharmaceutically acceptable carrier. As used herein, the term "pharmaceutically acceptable carrier" refers to a carrier or diluent that does not considerably stimulate the organism and does not inhibit the biological activity and properties of the administered component. In the present invention, the pharmaceutically acceptable carrier that may be included in a cell therapeutic agent may be used without limitation as long as it is known in the art, such as a buffering agent, a preservative, an analgesic agent, a solubilizer, an isotonic agent, a stabilizer, a base, an excipient, a lubricant, and the like. The cell therapeutic agent of the present invention may be prepared in the form of various formulations according to commonly used techniques. The cell therapeutic agent of the present invention may be administered through any route as long as it may induce migration to the diseased site. In some cases, a method of loading the stem cells into a vehicle equipped with a means for directing the lesion may be also considered. Therefore, the cell therapeutic agent of the present invention may be administered through several routes including topical (including buccal, sublingual, dermal and intraocular administration), parenteral (including subcutaneous, intradermal, intramuscular, instillation, intravenous, intraarterial, intraarticular and intracerebrospinal fluid) or transdermal administration, and is preferably administered directly to the site of disease. In one embodiment, the cells may be administered to an individual by suspending the drug in a suitable diluent, and the diluent is used to protect and maintain the cells and to facilitate use when injected into a target tissue. The diluent may include physiological saline, a phosphate buffer solution, a buffer solution such as HBSS, cerebrospinal fluid, and the like. In addition, the pharmaceutical composition may be administered by any device to allow the active substance to migrate to the target cell. A preferred mode of administration and preparation are injections. Injections may be prepared using aqueous solutions such as physiological saline, Ringer's solution, Hank's solution or sterilized aqueous solution, vegetable oils such as olive oil, higher fatty acid ester such as ethyl oleic acid, and non-aqueous solvents such as ethanol, benzyl alcohol, propylene glycol, polyethylene glycol or glycerin, and the like. For mucosal penetration, a non-penetrating agent known in the art suitable for a barrier to pass through may be used, and may further include a pharmaceutical carrier such as ascorbic acid, sodium hydrogen sulfite, BHA, tocopherol, EDTA, and the like as a stabilizer for preventing deterioration, and an emulsifier, a buffering agent for pH adjustment, and a preservative for inhibiting the growth of microorganisms such as phenylmercuric nitrate, thimerosal, benzalkonium chloride, phenol, cresol, and benzyl alcohol.

The present invention also provides a drug screening method using the cells or progenitor cells obtained by the above method.

Important features of the cells obtained by the present invention include the possibility of securing the production of a large amount of cells, the maintenance of their properties even during cryopreservation, the possibility of maintaining the same cell population for a long period of time, and differentiation more similar to those of cells derived from a living body. This property is particularly suitable for simultaneous screening of multiple drugs, which requires a large amount of cells in the same state and is the key to securing the same cells for a long period of time for repeated analysis thereof. It is very suitable for screening cells because a cell population with the same features in which key markers are maintained may be used continuously from the beginning to the end of the screening operation.

The drug is a drug for treating a disease selected from the group consisting of a neurodegenerative disease, an inflammatory degenerative disease, and a metabolic disease, and exhibits a therapeutic effect on the disease selected from the group consisting of a neurodegenerative disease, an inflammatory degenerative disease, and a metabolic disease.

The neurodegenerative disease may include, for example, various nervous system diseases including Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease, and Pick disease, but is not limited thereto.

The inflammatory degenerative disease may include one selected from the group consisting of dementia, Lewy body dementia, frontotemporal dementia, white matter degeneration, adrenoleukodystrophy, multiple sclerosis, and Lou Gehrig's disease, but is not limited thereto.

The metabolic disease may include one selected from the group consisting of diabetes mellitus, obesity, and a metabolic disorder, but is not limited thereto.

All technical terms used in the present invention, unless otherwise defined, have the same meaning as commonly understood by one of ordinary skill in the art to which the present invention belongs. In addition, although preferred methods and samples are described herein, ones similar or equivalent thereto are also included in the scope of the present invention. The contents of all publications described herein as a reference are incorporated herein by reference.

### Mode for Carrying out the Invention

Hereinafter, the present application will be described in detail through examples. The following examples are only for illustrating the present application, and the scope of the present application is not limited to the following examples.

### [Example]

### Example 1: Differentiation from human induced pluripotent stem cells into mDA (midbrain dopamine) neurons using midbrain organoids

### [Experimental method]

### Culturing of human embryonic stem cells or human induced pluripotent stem cells

hESCs and hiPSCs were cultured based on the hESC research guidelines approved by the institutional review board (IRB) of Hanyang University (Seoul, Republic of Korea). The hESCs and hiPSCs used in this experiment are shown in Table 1 below.

**[Table 1]**

| **hESC line** | | | | | | | | |
|---|---|---|---|---|---|---|---|---|
| **Name used** i**n this paper** | | **Original name** | | | K**aryotype** | | **Establishing institute** | |
| HSF6 | | UC06 | | | Female (46, XX) | | UCSF | |
| H9 | | WA09 | | | Female (46, XX) | | Wi cell | |
| HUES6 | | HUES6 | | | Female (46, XX) | | Harvard university (HSCI iPS Core) | |
| | | | | | | | | |

| **hiPSC line** | | | | | | | | |
|---|---|---|---|---|---|---|---|---|
| **Name used in this paper** | **Original name** | | **Source (Human)** | **Reprogramming factors** | | **Methods** | | **Establishing institute** |
| Retro-1 | Rv-hiPS 01-1 | | Newborn fibroblast | OCT4, SOX2, KLF4, MYC | | Retrovirus | | Harvard |
| Epi-ips | episomal-ips 1 | | Skin | OCT4, SOX2, KLF4, shp53 | | Episomal vector | | Hanyang Univ (Our own) |
| CMC-ips#11 | CMC-hipsc-011 | | Bone marrow blood | OCT4, SOX2, KLF4, MYC | | Sendai virus | | Catholic Univ. (Korea NIH SCRM) |
| PD-ips1 | PD patient derived ips-1 | | Skin | OCT4, SOX2, KLF4, shp53 | | Episomal vector | | Hanyang Univ (Our own) |
| PD-#22 | Corr PD-ips#22 | | Skin | OCT4, SOX2. KLF4, shp53 | | Episomal vector and genome edited | | Hanyang Univ. (Our own) |

For proliferation and maintenance of undifferentiation hESC/iPSC, the cells were cultured on Matrigel^{™} or on a vitronectin (Human; Gibco Fisher Scientific, Waltham, MA) (Gibco A31804; 0.5 µg/cm²)-coated 6 cm dish (Thermo Fisher Scientific, Waltham, MA) using mTESR-1 medium (Stemcell Technologies Inc., Vancouver, BC, Canada) in a CO₂ incubator set at 37°C without a feeder layer, and medium replacement was performed daily. The undifferentiated stem cells maintained their differentiation ability by replacing the medium daily, and were subcultured using Acutase (Stemcell Technologies Inc.) every 4 to 5 days.

### Preparation of midbrain-type neural stem cells using 3D organoid preparation method

Briefly, a system was used in which a midbrain-type 3D organoid was first prepared, cut into small pieces, and proliferated in a large amount in a culture dish in a state of midbrain-type neural stem cells.

hESCs and hiPSCs, which were normally cultured in an undifferentiated state, were detached using Accutase^{™} during subculture, and human embryonic stem cells in an undifferentiated state were aliquoted into a 96 well plate with a round bottom that does not stick to the bottom at 10,000 cells/well to form organoids once. In the medium composition of the first day, the generation of organoids was started with the additive conditions (SB431542, 10 µM; Noggin, 100 ng/ml; ascorbic acid, 200 µM; doxycyclin, 1 µg/ml; Y27632, 20 µM) corresponding to Day 0 of Table 2 in the seeding base medium of Table 4 below. The next day, the neural inducing base media (Table 3) was used as a medium, and development was induced by using a medium in which purmorphamine 2 µM, sonic hedgehog 100 ng/ml were additionally added to the components except for doxycyclin, 1 µg/ml, Y27632, 20 µM among the components added on the first day (see Table 2). The culture medium was the neural inducing base media until day 11, and development was induced by adding or subtracting the additives according to the concentration with reference to Table 2 for the addition components. The medium was replaced daily according to the date. Correct formation of organoids was confirmed by the formation of a cell mass on days 2 to 3, and was checked by buding into the outside of the cell before day 11. On day 11 of organoid differentiation, eight organoids formed one by one in a 96 well plate were grouped into each group and aliquoted and cultured into respective wells of a 6 well low binding plate. Development was induced under the midbrain patterning base media (Table 3) containing the components of Table 2 while rotating with an orbitary shaker (80 to 100 rpm). On day 18, one organoid was cut into about 4 to 10 pieces with a 30 G needle, treated with Accutase^{™} for 10 minutes, and then pipetted 5 times with a 1 ml pipette, and then Accutase^{™} was removed by centrifugation, and then 3 wells of organoid cells were center-plated in one 60 mm culture dish coated with poly-L ornithine (PLO; 15 µg/cm²)/fibronectin (FN; 1 µg/cm²) or vitronectin (VN; 0.5 µg/cm²).

**[Table 2]**

| | |
|---|---|
| Day 0 | Seeding Base Media (recipe below) + SB431542 (Tocris, #1614 10 µM) + Noggin (Peprotech, #120-10, 100 ng/ml) + Ascorbic Acid (Sigma, #A4544, 200 µM) + Doxycycline (Sigma, #D9891, 1 (µg/ml) + Y27632 (Sigma, #Y0503, 20 µM) |
| Day 1 | Neural Induction Base Media (recipe below) + SB431542 (Tocris, #1614 10 µM) + Noggin (Peprotech, #120-10, 100 ng/ml) + Purmorphamine (Calbiochem, #540223, 2 µM) + SonicHedgeHog (Peprotech, #100-45, 100 ng/ml) + Ascorbic Acid (Sigma, #A4544, 200 µM) |
| Day 2~4 | Neural Induction Base Media (recipe below) + SB431542 (Tocris, #1614 10 µM) + Noggin (Peprotech, #120-10, 100 ng/ml) + Purmorphamine (Calbiochem, #540223, 2 µM) + SonicHedgeHog (Peprotech, #100-45, 100 ng/ml) + CHIR99021 (Stemgent, #04-0004, 0.8 µM) + Ascorbic Acid (Sigma, #A4544, 200 µM) |
| Day 5~6 | Neural Induction Base Media (recipe below) + Noggin (Peprotech, #120-10, 100 ng/ml) + Purmorphamine (Calbiochem, #540223, 2 µM) + SonicHedgeHog (Peprotech, #100-45, 100 ng/ml) + CHIR99021 (Stemgent, #04-0004, 0.8 µM) + Ascorbic Acid (Sigma, #A4544, 200 µM) |
| Day 7~10 | Neural Induction Base Media (recipe below) + Noggin (Peprotech, #120-10, 100 ng/ml) + Purmorphamine (Calbiochem, #540223, 2 µM) + SonicHedgeHog (Peprotech, #100-45, 100 ng/ml) + Fgf8b (Peprotech, #AF-100-25, 100 ng/ml) + CHIR99021 (Stemgent, #04-0004, 0.8 µM) + Ascorbic Acid (Sigma, #A4544, 200 µM) |
| Day 11~14 | Midbrain Patterning Base Media (recipe below) + Noggin (Peprotech, #120-10, 100 ng/ml) + Purmorphamine (Calbiochem, #540223, 2 µM) + SonicHedgeHog (Peprotech, #100-45, 100 ng/ml) + Fgf8b (Peprotech, #AF-100-25, 100 ng/ml) + CHIR99021 (Stemgent, #04-0004, 1.5 µM) + Ascorbic Acid (Sigma, #A4544, 200 µM) |
| Day 15~16 | Midbrain Patterning Base Media (recipe below) + Purmorphamine (Calbiochem, #540223, 2 µM) + Fgf8b (Peprotech, #AF-100-25, 50 ng/ml) + CHIR99021 (Stemgent, #04-0004, 1.5 µM) + Ascorbic Acid (Sigma, #A4544, 200 µM) |
| Day 17 | Midbrain Patterning Base Media (recipe below) + Purmorphamine (Calbiochem, #540223, 2 µM) + Fgf8b (Peprotech, #AF-100-25, 50 ng/ml) + CHIR99021 (Stemgent, #04-0004, 1.5 µM) + bFGF (R&D systems, #233-FB, 20 ng/ml) + Ascorbic Acid (Sigma, #A4544, 200 µM) |
| Day 18 | Chop & Plating |

**[Table 3]**

| Seeding Base Media | Neural Induction Base Media | Midbrain Patterning Base Media |
|---|---|---|
| Neurobasal/N2 2X(1:1) (Neurobasal, Gibco, #21103049) | Neurobasal/N2 2X(1:1) (Neurobasal, Gibco, #21103049) | Human N2 (human N2 recipe as follows(1L) |
| (N2 2X recipe as follows(1L) | (N2 2X recipe as follows(1L) | D.W. 1L |
| D.W. 1L | D.W. 1L | DMEM-F12 12g |
| DMEM-F12 12g | DMEM-F12 12g | Sodium Bicarbonate 1.69g |
| Sodium Bicarbonate 1.69g | Sodium Bicarbonate 1.69g | D-glucose 1.55g |
| D-glucose 1,55g | D-glucose 1,55g | L-Glutamine 0.073g |
| L-Glutamine 0.073g | L-Glutanine 0.073g | Apo-transferrin 200mg |
| Apo-transferrin 200mg | Apo-transferrin 200mg | Insulin 312*µl* |
| Putrescine 200*µl* | Putrescine 200*µl* | Putrescine 100*µl* |
| Selenite 120*µl* | Selenite 120*µl* | Selenite 60*µl* |
| Progesterone 400*µl*) | Progesterone 400*µl*) | Progesterone 200*µl*) |
| MEM-NEAA (Sigma, #M7145, 1:100) | B27 w/o VitA (Gibco, #12587010, 1:50) | |
| â-mercaptoethanol (Sigma, #M6250, 0.1%) | Insulin (Gibco, #12585014, 0.31*µl*/ml) | |
| B27 w/o VitA (Gibco, #12587010, 1:50) | | |
| GlutaMAX (Gibco, #35050061, 1:100) | | |
| Insulin (Gibco, #12585014, 0.31*µl*/ml) | | |

### Mass proliferation of midbrain 3D organoid-derived midbrain-type neural stem cells

The organoid-derived midbrain-type neural stem cells were physically cut with a 30 G needle. After the first plating for subculture, 3 X 10⁶ cells were cultured in plane in a 60 mm culture dish coated with PLO/FN or PLO/VN using Accutase^{™} once a week for subculture when the cells were about 80 to 90% full. For the culture medium of midbrain-type neural stem cells, a medium in which mitogen (bFGF 20 ng/ml, FGF-8 50 ng/ml), BDNF (20 ng/ml), GDNF (20 ng/ml), ascorbic acid 200 µM, doxycyclin, 1 µg/ml were added to the midbrain patterning base medium of Table 4 was used as a proliferation medium. However, on the day of subculture, Y27632, 20 µM was added to this medium. At every subculture, 8 X 10⁴ cells per well were plated on a PLO/VN 24 well coated plate and proliferated for 2-3 days. When 80-90% cell confluency was reached, differentiation of NSCs was induced by fluorescence staining of midbrain-type markers or removal of mitogens (bFGF, FGF-8). For the differentiation medium, BDNF (20 ng/ml), GDNF (20 ng/ml), Ascorbic acid 200 µM, and cAMP 500 µM were added to the midbrain patterning base medium shown in Table 4 above to induce the differentiation of neurons for 12 days, and then it was checked whether the dopaminergic neuron and midbrain-type markers were coexpressed.

### Frozen storage of 3D organoid-derived midbrain-type neural stem cells

Since organoid-derived midbrain-type neural stem cells generally do not change their properties at least until passage 5, the cells can be thawed and used without change in properties after frozen storage of the cells at every passage after the first passage. The frozen storage of the cells was made during subculture, the cells were detachd using Accutase^{™}, the number of cells was counted, and 6 X 10⁶ cells were dissolved and frozen in a freezing medium mixed with a proliferation medium and DMSO at a concentration of 10% in one freezing vial. The cells were stored frozen at -70°C for the first 24 hours and then stored in a cryogenic nitrogen tank the next day until use. If necessary, one vial was quickly thawed in a 37°C water bath, and the cells were cultured in plane in a 60 mm culture dish coated with PLO/FN or PLO/VN. For thawing, a neural stem cell proliferation medium was used, and on the day of thawing, Y27632, 20 µM was added to this medium.

### Immunocytochemistry

The cells were fixed with 4% paraformaldehyde in PBS (phosphate-buffered saline), cultured in a blocking solution (1% BSA/0.03% Triton X-100 in PBS), and then cultured together with the following primary antibodies. Tuj1 anti-rabbit (1:2000, Babco), TH anti-mouse (1:1000, Immunostar), GFAP anti-mouse (1:100, ICN Biochemicals), microtubule-associated protein 2 (MAP2, 1:200, Sigma). Secondary antibodies labeled with the following fluorescence molecules were used for visualization. Other antibodies are listed in Table 4. Alexa 488 (1:200, Invitrogen) and Cy3 (1:200, Jackson ImmunoResearch Laboratories). The stained sample was mounted in a Vectashield (Vector Laboratories) with DAPI mounting medium and photographed using an epifluorescence microscope (Leica).

**[Table 4]**

| Antibodies | Working dilution | Company | Notes |
|---|---|---|---|
| Mouse monoclonal Antibody (Ab) | | | |
| Oct4 | 1:200 | ¹Santa Cruz Biotechnology | Use only Cy3 2nd antibody |
| Pax6 | 1:100 | ²DSHB | |
| Cdh2 (N-cadherin) | 1:500 | Santa Cruz Biotechnology | |
| Neuron-specific class III betatubulin (TuJ1) | 1:500 | ³Covance | |
| Tyrosine Hydroxylase (TH) | 1:1000 | ⁴Immunostar | Incubation for 2 days at 4°C |
| Ki67 | 1:100 | ⁵Novocastra | |
| Pax5 | 1:50 | ⁶BD Biosciences | |
| human Neural Cell Adhesion Molecule (hNCAM) | 1:100 | Santa Cruz Biotechnology | |
| HuC/D | 1:100 | ⁷Chemicon | |
| Microtubule-Associated Protein 2 (MAP2) | 1:200 | ⁸Sigma | |
| Dopamine Transporter (DAT) | 1:100 | BD Biosciences | Blocking with 0.1% |
| | | | Saponin |
| Human Nuclei antigen (HN)1:1000 | | Chemicon | |

| Rabbit polyclonal Ab | | | |
|---|---|---|---|
| Nanog | 1:200 | Santa Cruz Biotechnology | |
| Nestin #130 | 1:50 | ⁹Dr Martha Marvin, Dr Ron McKay | |
| Sox2 | 1:100 | Chemicon | |
| TuJ1 | 1:2000 | Covance | |
| Pax2 | 1:100 | Covance | |
| TH1:500 | | ¹⁰Pel-freez | |
| GFAP | 1:400 | ¹¹DAKO | |
| Serotonin | 1:4000 | Sigma | |
| γ-aminobutyric acid (GABA) | 1:700 | Sigma | |
| GAD67 | 1:500 | Chemicon | |
| Vesicular monoamine transporter 2 (VMAT2) | 1:500 | Pel-freez | Use only cy3 2nd antibody |
| Nurr1 | 1:500 | Chemicon | 0.1% SDS treatment for 5 min before blocking |
| EN1 | 1:200 | Chemicon | Incubation for 2 days at 4°C |
| Calbindin1, 28 kDa (calbindin D28K) | 1:250 | Chemicon | |
| potassium inwardly-rectifying channel, subfamily J, member 6 (GIRK2) | 1:200 | Sigma | |
| Cleaved Caspase-3 | 1:500 | ¹²Cell signaling | |

### Cell transplantation and histological procedure in Parkinson's disease model animal

Experiments were performed according to NIH (National Institutes of Health) guidelines. Hemi-parkinsonian was induced in mature female Sprague-Dawley rats (220-250 g) by stereotaxic injection of 3 µl of 6-OHDA (6-hydroxydopamine, 8 µg/µl; Sigma) alone into the right substantia nigra (AP-4.8 mm, ML-1.8 mm, V-8.2 mm) and MFB (median forebrain bundle) (AP -4.4 mm, ML -1.2 mm, V -7.8 mm). The incisor bar was set to -3.5 mm, and the AP and ML coordinates were given relative to the bregma. The rats with 300 rotations/hour on the same side for this disorder were selected in the amphetamine-induced rotation test. For transplantation, rat E12 VM-NPCs were proliferated and mixed with Ctx-, VM-astrocytes, N + F-VM-astrocytes or E14 Ctx-NPCs (control group) in a 2:1 ratio. 3 µl of the mixed cells (1.5 × 10⁵ cells/(µl) was injected into two sites of the striatum (coordinates of AP, ML and V relative to bregma and dura: [1] 0.07, -0.30, -0.55; [2] -0.10, -0.40, -0.50; incisor bar set to 3.5 mm below zero), respectively, over 10 minutes under anesthesia induced by 100 µl/100 g (50 mg/ml) of Zoletil mixed with 100 µl/100 g (23.32 mg/ml) of Rompun. The needle (22 gauge) was left in place for 5 minutes after completion of each injection. The rats were administered daily with cyclosporine A (10 mg/kg, i.p.) from one day before transplantation for 1 month and maintained without immunosuppressants for the remainder of the post-transplantation period. Six months after transplantation, the animals were anesthetized and transdermally perfused with 4% paraformaldehyde. The brains were removed, soaked in 30% sucrose in PBS overnight, frozen in Tissue-Tek^{®} (Sakura Finetek, Torrance, CA, USA), and then sliced in a frozen microtome (Leica). Free-floating brain sections (thickness of 30 µm) were subjected to immunohistochemistry as described above and imaged by a confocal microscope (Leica). In experiments to test the host environment of transplanted brains, the animals were sacrificed one month after transplantation and sliced to a thickness of 1 mm on a rat brain slice matrix (ZIVIC Instruments, Pittsburgh, PA). In order to observe the changes after transplantation, the tissue at transplantation site was extracted. 8-12 regions of the graft-host interface (ca 2x2 mm)/graft were dissected, and qPCR analysis was performed. In addition, cells immunoreactive to neurotrophic and pro-inflammatory glia markers were counted along the graft-host interface of the freeze cleaved brain slices 7-10 days after transplantation.

### Behavior test

Animal behavior was evaluated using the apomorphine/amphetamine-induced rotation test as described in the paper by Y. H. Rhee et al. (LIN28A enhances the therapeutic potential of cultured neural stem cells in a Parkinson's disease model. Brain: α journal of neurology 139, 2722-2739 (2016)). Apomorphine (Sigma) was injected subcutaneously at a dose of 0.5 mg/kg and observed while rotating for 60 minutes. The results are expressed in a unit of final number of revolutions/60 minutes.

### Animal PET/MRI imaging and analysis

PET-MRI fusion imaging was performed using a nanoScanPET/MRI system (IT, Mediso, Hungary). In order to keep mice warm, 0.2 mL of FP-CIT was administered intravenously via tail vein 6.5±1.0 MBq, and the rats were maintained under anesthesia (1.5% isoflurane in 100% O₂ gas). MR brain imaging obtained T1 images weighted with gradient echo (GRE) 3D sequences (TR = 25 ms, TE_{eff}= 3.4, FOV = 50 mm, matrix = 256x256), and T2 images weighted with fast spin echo (FSE) 3D sequences (TR = 2400 ms, TE_{eff}= 110, FOV = 50 mm, matrix = 256x256) acquired during the FP-CIT uptake period. A 20 minute static PET image was acquired with a 1-3 concordance in a single field of view within the MRI range. Body temperature was maintained with heated air of the animal bed (Multicell Mediso, Hungary), and pressure-sensitive pads were used to induce respiration. PET images were reconstructed by Tera-Tomo 3D in full detector mode with all corrections and high normalization and 8 repetitions. Three-dimensional VOI (volume of interest) analysis of the reconstructed images was performed by using the InterView Fusion software package (Mediso, Hungary) and applying standard uptake value (SUV) analysis. The VOIs were fixed with 2 mm spheres in the coronal images, and VOIs for the striatum were derived. The SUV of each VOI site was calculated using the following equation. SUVmean = (Bq/cc × tumor radioactivity in the tumor volume of interest in body weight)/ radioactivity injected.

### High-throughput whole transcriptome RNA sequencing

For RNA, total RNA was isolated using a Trizol reagent (Invitrogen), and then the removal of rRNA was performed using the Ribo-Zero Magnetic kit (Epicentre Inc., Madison, WI, USA). Library construction was performed using the SENSE mRNA-Seq Library Prep Kit (Lexogen Inc., Vienna, Austria). High-throughput sequencing was performed as pairedend 100 sequencing using the HiSeq 2000 (Illumina, San Diego, CA, USA). RNA-Seq read values were mapped using TopHat software to obtain bam alignment files. Read counts mapped to transcription regions were extracted from alignment files using BEDTools (v2.25.0) and R/Bioconductor (version 3.2.2; R Development Core Team, 2011). The alignment files were used to assemble transcriptomes, estimate their abundance, and detect differential expression of genes, linc RNAs or isoforms. FPKM (fragments per kb of exon per million fragments) was used to determine the expression level of the gene region. Global normalization was used for comparison between samples. Genetic classification is based on searches submitted to DAVID (http://david.abcc.ncifcrf.gov/).

GO and KEGG pathway analysis was performed using DAVID Bioinformatics Resources version 6.8. GO and KEGG pathway analysis of the rich categories gave p-value up to 0.05.

### 10× Genomics single cell RNA sequencing (scRNA-seq) and data analysis

Single cell transcription factor regulation analysis (scRNA-seq) was performed using Chromium Controller (10 × Genomics, San Francisco, CA) program after experiments of Chromium Single cell Gene Expression Solution and Chromium Single cell 3' GEM, Library and Gel Bead Kit v2 (10× Genomics). Cells separated into 15,000 single cells were placed on Single cell A chip with Master mix solution. In each single cell, transcription factors inside the cell were reverse transcribed and RNA barcoded by emulsion of oligo dT encoded UMIs. The barcoded libraries were sequenced with the Illumina HiSeq 4000 platform (Macrogen Inc., Seoul, Korea). The results were analyzed with Cell Ranger (v2.1.1, 10× Genomics). For 2D-NSCs analysis, 12,166 cells with 2D_cellRanger averaged 57,343 reads and 2,284 genes per cell (among 24,055 total genes) were analyzed, and for Og-NSCs analysis (with 3D_cellRanger), 13,739 cells averaged 39,191 reads and 2,353 genes per cell (among 23,860 total genes) were analyzed. Cellular transcription factor expression was expressed as UMI count. Cell diversity analysis was performed by k-means clustering for t-SNE (t-distributed stochastic neighbor embedding) analysis (performed by Loupe Cell Browser ver. 3.0.1, 10× Genomics, Inc.). The top 40 genes with increased expression showing a significant difference in expression were analyzed by hierarchical clustering analysis method, and each cluster was divided based on this. For GO and KEGG pathway analysis of Og-NSCs, 580 differentially upregulated genes (UMI >1, log2FC>0.5, P<0.05) were analyzed. Genetic analysis was mainly performed using DAVID (http://david.abcc.ncifcrf.gov/). Network analysis was performed using Cytoscape (version 3.7.1+JAVAv8, provided by NIGMS).

### Analysis of mitochondrial function

The regenerative dynamics of mitochondria was analyzed using integrated portions of young (green) and old (red) MitoTimer proteins (Ferree, A.W., Trudeau, K., Zik, E., Benador, I.Y, Twig, G., Gottlieb, R.A., and Shirihai, O.S. (2013). MitoTimer probe reveals the impact of autophagy, fusion, and motility on subcellular distribution of young and old mitochondrial protein and on relative mitochondrial protein age. Autophagy 9, 1887-1896; Hernandez, G., Thornton, C., Stotland, A., Lui, D., Sin, J., Ramil, J., Magee, N., Andres, A., Quarato, G., Carreira, R.S., et a/. (2013). MitoTimer: a novel tool for monitoring mitochondrial turnover. Autophagy 9, 1852-1861). The pMitoTimer vector (Addgene52659; Addgene, Watertown, MA) was injected into NSCs cells by electroporation (NEPA21, Nepagene, Japan) and expressed, and then 2 days later, mitochondria generated by red versus green fluorescence were investigated. Mitochondria ROS and membrane potential were measured with MitoSox (Thermo Scientific Inc., Waltham, MA) and NucleoCounter3000 (NC3000; Chemometec, Allerod, Denmark), respectively. ROS generation was analyzed for induction after treatment with H₂O₂ (500 µM) 2 hours before analysis. In order to study the toxicity resistance of mitochondria, mitochondria were treated with 10 µM Rotenone, 4 µM CCCP or 250 µM H₂O₂ for 1 hour and analyzed by TH fluorescence staining.

### Analysis of DA release

The presynaptic activity of DA neurons was determined by measuring the level of DA neurotransmitter released from the differentiated VM-NPC culture. The medium cultured for 24 hours (days 12-13 of differentiation) was collected, and the DA level was measured using an ELISA kit (BA E-5300, LDN). In addition, the DA release induced by membrane depolarization was evaluated by culturing (day 12 of culture) in a fresh N2 medium culture for 30 minutes in the presence or absence of 56 mM KCl. The induced DA release was calculated as the DA level with KCl minus the DA release without KCl.

### Analysis of intercellular α-syn transduction

### Confirmation of intercellular α-synuclein aggregate transfer by factors secreted from astrocytes

An analysis experiment was conducted using a co-culture with cells overexpressing A53Tα-syn-EGFP in SH-SY5Y (Professor Sang Myun Park, Ajou University) cells derived from neuroblastoma.

SH-SY5Y cells, which were induced to differentiate using RA for 5 days and induced to express A53Tα-syn-EGFP, were co-cultured for 24 hours with neural stem cells induced to differentiate for 10 days in a dual chamber system, and the transfer of A53Tα-syn-EGFP was compared by the level of GFP expression. The transfer (transmission) of α-synuclein was confirmed by immunostaining (immunoncytochemistry) through the brightness of α-syn-GFP using an antibody.

### Detection of pathological α-syn aggregates

During differentiation, the medium was treated (8 µg/ml of medium) with PFF (preformed fibrils), α-synuclein aggregates, to induce α-synuclein aggregation, and the level of α-synucelin aggregation was analyzed after 20 days of differentiation. Alternatively, overexpression was induced using lentiviruses expressing α-syn (pEF1α-α-syn) and analyzed. Immunostaining (immunoncytochemistry) using thioflavin S (staining method to measure protein aggregation; Sigma Aldrich) and α-synuclein antibody and protein electrophoresis (Western blot) were used to confirm the presence, and the aggregation was observed through a change in protein size.

### Bi-FC (Bimolecular fluorescence complementation)

Og-NSCs or 2D-NSCs were induced to express lentiviruses expressing Venus1-α-syn (V1S; N-terminal of α-syn), α-syn-Venus2 (SV2; C-terminal of α-syn), and the aggregated α-syn in dopaminergic neurons that were differentiated for 25 days was analyzed by GFP.

### In vivo α-syn propagation into transplanted grafts

Female SD (Sprague Dawley) rats (225-250 g) were injected into the striatum with AAV expressing human hα-syn (AAV2-CMV-hα-syn, 2× 10¹³ genome copies) and PFF (10 µg) to induce the expression and aggregation. anteroposterior (AP), -0; mediolateral (ML), - 3.0; dorsoventral (DV), -5.0. Two weeks later, Og-NSCs and 2D-NSCs were transplanted onto both sides of the striatum, respectively. One month after transplantation, the transfer of α-syn to transplanted cells was analyzed by immunohistochemistry (a-syn and p129-α-syn).

### Two-photon imaging of GCaMP6 expressing neurons in grafts

GCaMP6 (Addgene, # 40753) was generated under the control of the synapsin promoter (pSyn-GCaMP6s) and used to transduce the culture *in vitro.* 2 ml/6-cm dish or 200 µl/well (24-well plate) and 10⁶ transduction units (TU/ml (60-70 ng/ml) were used for each transduction reaction. Packaging and production of AAV2-CMV-hα-syn were performed at the Korea Institute of Science and Technology (Seoul).

Og-NSCs at passage 3 were transduced with lentivirus (pSyn-GCaMP6) 3 days before transplantation. Thereafter, the cells were transplanted into 4 wild-type SD rats, and the rats were analyzed one month after transplantation. Two-photon imaging was performed using a commercial microscope system (SP-5, Leica, Germany) equipped with a Ti:Sapphire femtosecond laser (Chameleon Vision, Coherent Inc., USA) having a tunable wavelength from 680 to 1080 nm, 80 MHz repetition rate and 140 fs pulse width.

For *in vivo* brain imaging, a single GRIN lens (NEM-100-25-10-860-S-1.0p-ST, GRINTECH GmbH) with a diameter of 1.0 mm and a length of 9.20 mm was used. Under gas anesthesia, rats were mounted in a rat head holder with the surgically exposed brain facing up. The GRIN lens was fixed to an aluminum plate through a hole in the plate, and the plate was translated and the brain was inserted from above. A 10× dry objective lens (Leica HC PL FLUOTAR 10.0 × 0.30) was placed close to one end of the GRIN lens in order to couple the excitation laser from the microscope system. The excitation wavelength was set to 900 nm for the two-photon excitation of GCaMP6. Two-photon imaging was performed by scanning the excitation laser into the brain. The imaging field of view (FOV) and imaging speed were 387.50 µm x 387.50 µm with 512 × 512 pixels and 9.03 frames/s, respectively. Emission light from the brain was collected by the GRIN lens and coupled to the microscope system through the objective lens. The microscope system had four detection channels (Ch1 : 430-480 nm, Ch2 : 500-550 nm, Ch3 : 565-605 nm and Ch4 : 625-675 nm), and GCaMP6 fluorescence was collected in the second channel (Ch2). The excitation laser power is 58.08 mw.

### Calcium imaging analysis

The medium was treated with Fluo3-AM for 1 hour so that Fluo3-AM was absorbed into the cells, and washed 3 times with PBS so that no fluorescence remained in the medium. While observing fluorescence with a confocal microscope, the intensity of fluorescence was taken at an interval of 1 to 3 seconds, and ROI was measured for each cell in each timeframed photograph, and it was confirmed that the intensity of fluorescence was changed. It was gated by FACS as needed.

### Experiment result

### Preparation of midbrain-type organoids from human embryonic stem cells

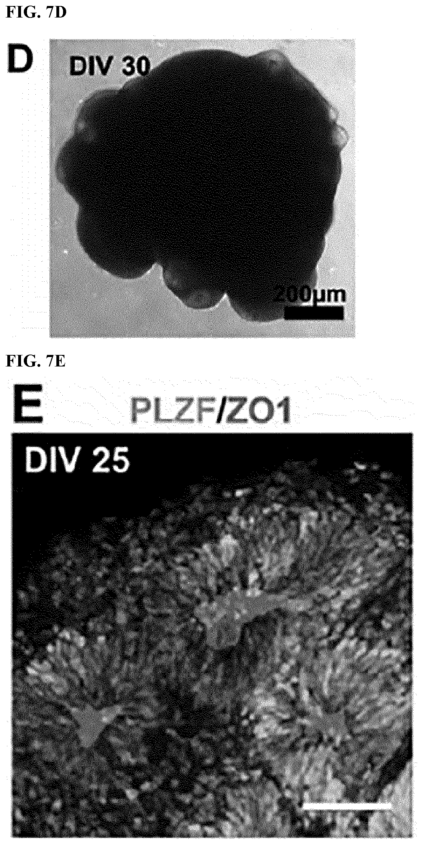

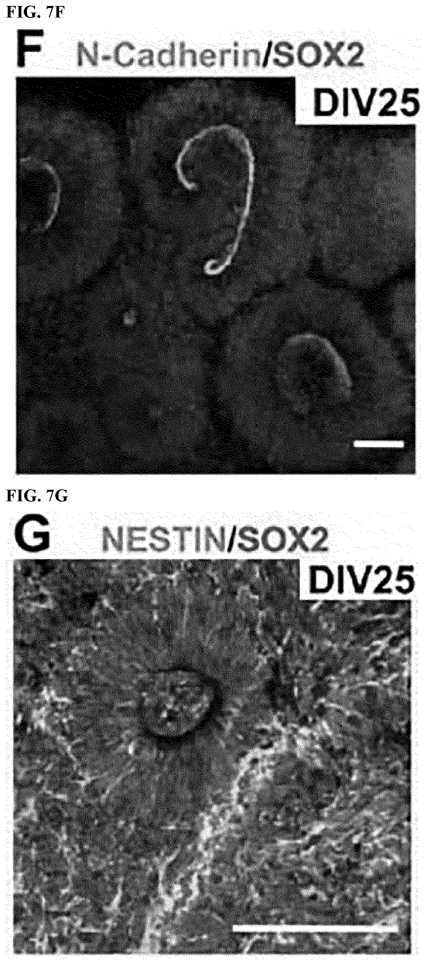

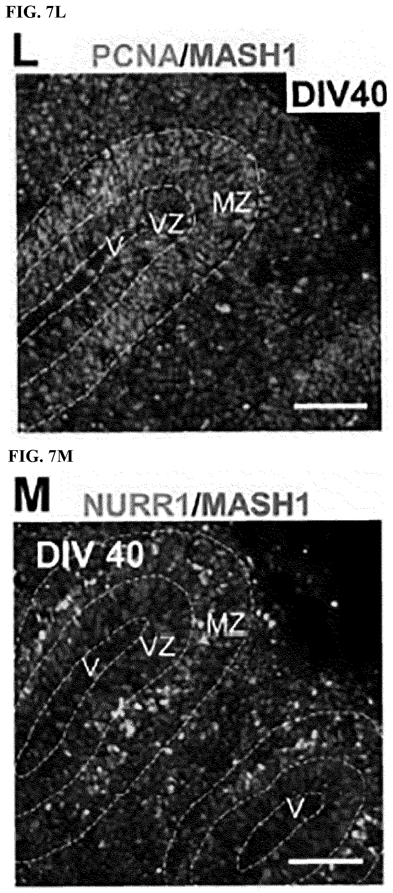

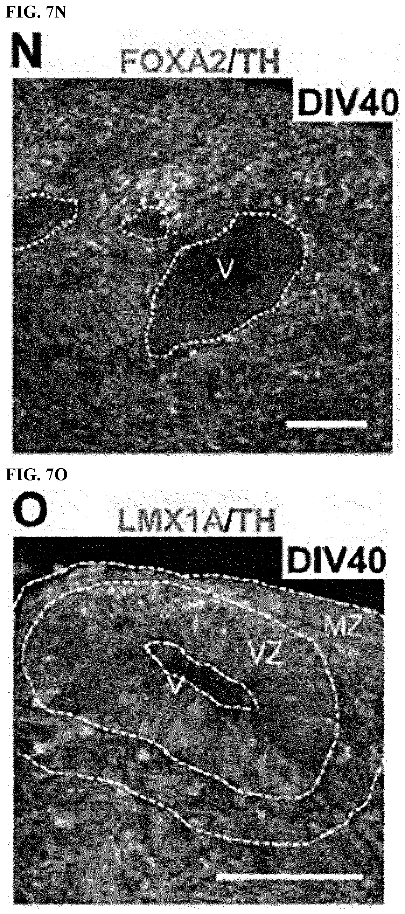

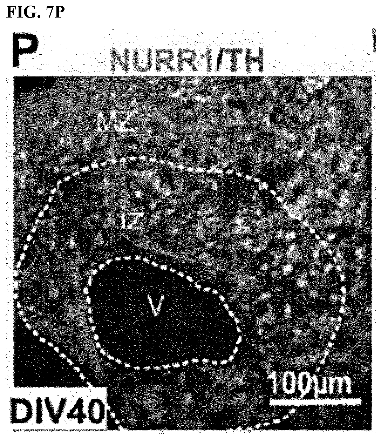

The conventional differentiation technology based on two-dimensional 2D culture does not represent a three-dimensional complex network in the actual brain *in vivo.* Only the development of a protocol based on three-dimensional generation can represent the tissue properties of the actual brain. Therefore, first of all, this research team first prepared an existing 3D midbrain-type organoid. In the early stage of development of the successfully prepared midbrain-type organoids, ZO-1 and N-cadherin were expressed along with PLZF+ and Sox2+, which are neural stem cell markers (FIGs. 7E and 7F). Anterior embryonic brain markers (OTX2) and ventral VM floor plate markers (FOXA2 and LMX1A) were expressed along with Nestin and Sox2, which can be referred to as general neural stem cell markers (FIGs. 7G to 71). When development was further induced, the neuron marker MAP2 was expressed as a layer outside the proliferation markers Ki67+ and SOX2+, and more specifically, like the brain layer of the living body, structures specifically expressed in PCNA+ (proliferative ventricular zone; VZ), MASH1+ (VZ, intermediate zone (IZ) and mantle zone (MZ)), and NURR1+ cells (IZ and MZ), respectively, were confirmed (FIGs. 7L and 7M). Characteristically, in IZ and MZ, FOXA2+, LMX1A+ and NURR1+ cells, which are representative midbrain specific factors, were expressed along with the dopaminergic neuron marker TH (tyrosine hydroxylase), confirming that they were true midbrain-type dopaminergic neurons (FIGs. 7N, 7O, and 7P).

### Isolation of midbrain-type neural stem cells (Og-NSCs) from midbrain-like organoids

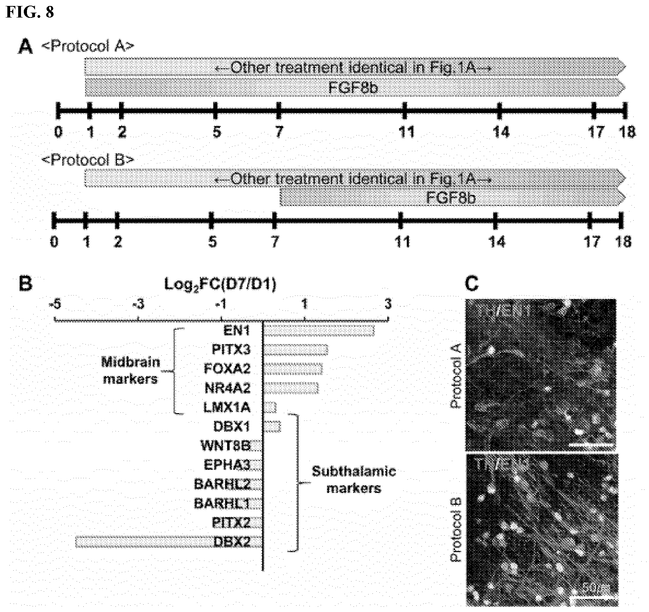

When used as a cell therapeutic agent for Parkinson's disease, a technology for isolating and culturing organoid-derived midbrain-type neural stem cells (Og-NSCs) as a more evolved form of neural stem cells was developed. As a previous study, an attempt was made to extract neural stem cells from organoids during days 10 to 35 of development, but the efficiency was not high. It is believed that it is possible to secure an appropriate amount of cells by increasing the ratio of neural stem cells in the organoid, and the protocol was modified in an effort to increase the amount of cells.
(1) Since the amount of stem cell population is automatically reduced when differentiation is continuously induced, the final developmental differentiation protocol to be performed from day 10 was stopped and improved in the direction of proliferation. (2) From day 17, it was modified by adding bFGF, which can specifically increase neural stem cells. (3) The proliferation of midbrain specific neural stem cells was induced by extending the treatment period of midbrain specific factors (SHH/Purmorphamine/CHIR999021) (from day 1 to day 18), that is, by extending the process of midbrain patterning. (4) Unlike the conventional method, the FGF8b treatment period started from day 7 (A in FIG. 8).

By controlling such a detailed time period, the development into the hypothalamus (sunthalamic), which is prone to contamination during induction of the midbrain, can be reduced, and the expression of EN1, one of the specific markers, can be significantly increased (FIG. 8). By applying these various modifications, proliferation and culture were performed in a 2D environment on day 18 of inducing the generation of midbrain-type organoids. Human vitronectin coated plates were cultured in a bFGF-supplemented hN2 medium. After isolation, cells contained 84-97% of organoid-derived neural stem cells (Og-NSCs). It was confirmed that the neural stem cell markers SOX2 and NESTIN were expressed, and as OTX2 midbrain-type markers, FOXA2 and LMX1A, which are very important for initial securing of dopaminergic neuron properties, were expressed (FIGs. 1B and 1C). By confirming the expression of PAX2 and PAX5 factors around the neural stem cells, it was confirmed that they developed into true midbrain-type cells (FIG. 1B). It was observed through the expression of midbrain specific markers such as FOXA2+ and LMX1A+ (FIG. 12), that organoid-derived midbrain-type neural stem cells (Og-NSCs) can proliferate while faithfully maintaining their properties during subculture (FIG. 1D). We attempted to prepare VM-type NSCs in 2D hESC culture. Treatment of medium conditioned by VM-like astrocytes (astrocyte conditioned medium, ACM) was important for cell survival and midbrain marker expression during cell passage (FIG. 9A). With ACM treatment and modification to enrich the NSC population described above, the present inventors were also able to prepare FOXA2+/LMX1A+VM-NSC capable of being expanding from the differentiation of hESCs in the conventional 2D culture (FIG. 9).

### Organoid-derived midbrain-type neural stem cells (Og-NSCs) can be differentiated into complete dopaminergic neurons that fully express midbrain-type markers through the final differentiation process

Through the final differentiation process, midbrain-type Og-NSCs can be efficiently differentiated into complete dopaminergic neurons (FIGs. IE and 1F). Og-NSC-derived dopaminergic neurons simultaneously expressed FOXA2 (95%), LMX1A (96%), NURR1 (91%), and EN1 (86%), midbrain-type markers, along with TH, a dopamine neuron marker. On the other hand, in the conventional 2D culture environment, low midbrain-type factor expression of about 44-61% was shown (FIGs. 9E, 9F, and 10). Moreover, this organoid-derived midbrain-type neural stem cell isolation protocol is applicable to a variety of human embryonic stem cells and induced pluripotent stem cells (FIGs. 1G and 11). The success rate of 111 out of 117 experiments that were performed with 8 human pluripotent stem cells (3 hESCs and 5 hiPSCs) was a considerably high success rate. On the other hand, the 2D method was much more difficult to apply, so a success rate of 28 out of 63 was recorded (FIG. 1H).

The H9 hESC line showing the highest midbrain-specific factor expression was mainly used throughout this study. This is because the protocol developed from organoids is very stable, and it seems that only a small amount undergoes the process of cell death during general processes such as cell isolation, proliferation, and differentiation. This, in contrast to the 2D method, showed a significant percentage of cell death during the procedure. In actual culture, analysis of ethidium heterodimer-stained dead cells (FIG. 1I) and cleaved caspase 3+ dead cells (FIG. 1J) and analysis of Annexin+/PI+ dead cells through FACS analysis after cell isolation showed stable results with relatively few numbers (FIG. 1K). Senescent cells (pre-apoptotic stage) represented by β-galactosidase+ cells were also shown to be significantly less in Og-NSC culture (FIG. 1L). Overall, the newly secured protocol can more stably secure midbrain-type neurons compared to the existing protocol.

### Og-NSC cultured cells can be used as platform cells for new drug development

Og-NSCs can be patterned in the midbrain-type and differentiated into complete dopaminergic neurons after differentiation, and can maintain their properties for more than 5 passages (FIGs. 1D, 12A, and 12B). Arithmetically, 7,260 vials (9,075 culture dishes) of Og-NSCs can be secured from one culture dish (FIG. 12A). In addition, the cells can be stored in a nitrogen tank without loss of their function (FIG. 12C). As a result, the cells are actually being used by a leading pharmaceutical company (Chong Kun Dang Pharm., Seoul, KOREA) and for development of new drug candidates for Parkinson's disease (FIG. 13). In collaboration with the pharmaceutical company (Chong Kun Dang Pharm., Seoul, KOREA), the expandability and storability properties of the Og-NSC culture system was subjected to a bulk screening of candidate chemicals in order to develop PD drugs using mDA neurons from hESC (H9) and PD-hiPSC (PD-ips1) cell lines (FIG. 13).

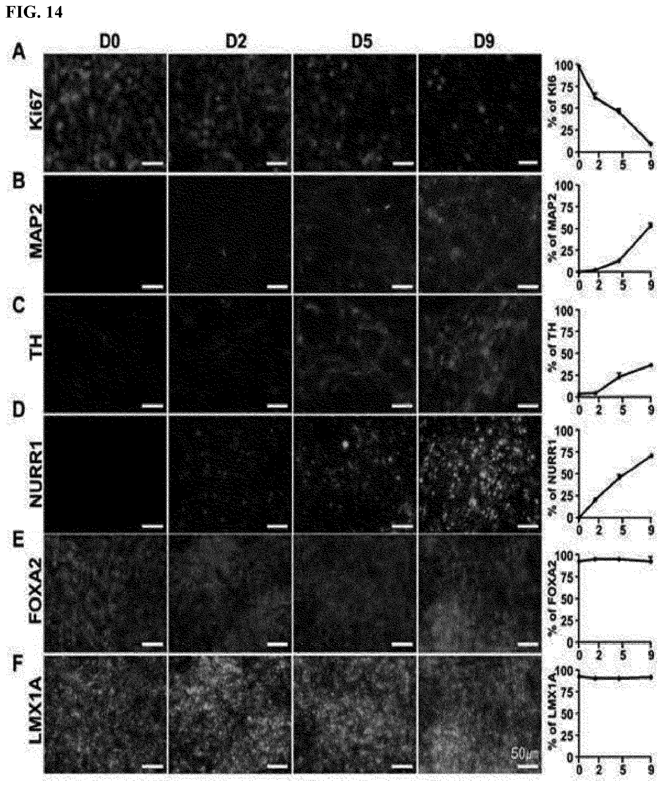

Og-NSCs in an undifferentiated state perfectly express an appropriate midbrain-type neural stem cell marker (FIGs. 1B, 1C, 14A, 14E, and 14F). Prior to differentiation, the cells do not express any differentiation neuronal/DA neuronal markers (MAP2, TH) (B and C in FIG. 14). After induction of the final differentiation, the expression of the proliferation marker Ki67+ is reduced, and morphological changes such as neurite lengthening occur as a property of neurons (A to C in FIG. 14). As in the developmental stage of actual midbrain-type neural stem cells, NURR1, a specific marker, starts to be expressed from the progenitor cell stage and continues to be expressed until post-differentiation (from day 2 of differentiation to the stage of final differentiation) (D in FIG. 14). Actually, in accordance with the biological rhythm of development in the midbrain, FOXA2 and LMX1A were continuously expressed, and after differentiation, the expression of LMX1A+ showed a tendency to slightly decrease (E and F in FIG. 14). NURR1, FOXA2, and LMX1A, important markers of midbrain-type cells, were well expressed overlaid on the finally differentiated dopaminergic neurons (FIGs. IE to 1G). These findings collectively indicate that *in vitro* NSC differentiation recapitulates physiological mDA neuron development in VM, and may be useful as an *in vitro* model to study human mDA neuron development that did not exist.

### Analysis of transcriptional regulation properties of Og-NSCs using bulk and single cell RNA sequencing

In order to analyze molecular biological transcriptional regulation of Og-NSCs, RNA sequencing (RNA-seq) analysis was performed. Organoid-derived midbrain-type neural stem cells (Og-NSCs) and neural stem cells (2D-NSCs) generated by the conventional differentiation method were compared and analyzed for differences in transcription regulation factors in the existing human fetal and adult brain tissues (Carithers, L.J., Ardlie, K., Barcus, M., Branton, PA., Britton, A., Buia, S.A., Compton, C.C., DeLuca, D.S., Peter-Demchok, J., Gelfand, E.T., et al. (2015). A Novel Approach to High-Quality Postmortem Tissue Procurement: The GTEx Project. Biopreserv Biobank 13, 311-319.; Jo, J., Xiao, Y., Sun, A.X., Cukuroglu, E., Tran, H.D., Goke, J., Tan, Z.Y., Saw, T.Y., Tan, C.P., Lokman, H., et al. (2016). Midbrain-like Organoids from Human Pluripotent Stem Cells Contain Functional Dopaminergic and Neuromelanin-Producing Neurons. Cell Stem Cell 19, 248-257.). As in previous reports (Jo, J., Xiao, Y., Sun, A.X., Cukuroglu, E., Tran, H.D., Goke, J., Tan, Z.Y., Saw, T.Y., Tan, C.P., Lokman, H., et al. (2016). Midbrain-like Organoids from Human Pluripotent Stem Cells Contain Functional Dopaminergic and Neuromelanin-Producing Neurons. Cell Stem Cell 19, 248-257.), PCA (principal component analysis) and Spearman's correlation analysis showed that midbrain-type organoids were remarkably similar to human fetal brains (FIGs. 2A and 2B). Og-NSCs are similar to midbrain-type organoids and maintain their characteristics (Spearman's correlation co-efficiencies: 0.79-0.97). In addition, it can be seen that Og-NSCs are similar to an actual fetal midbrain (0.70-0.85), so that the Og-NSCs extracted from actual neural stem cells maintain their characteristics even in the process of isolation and subculture.

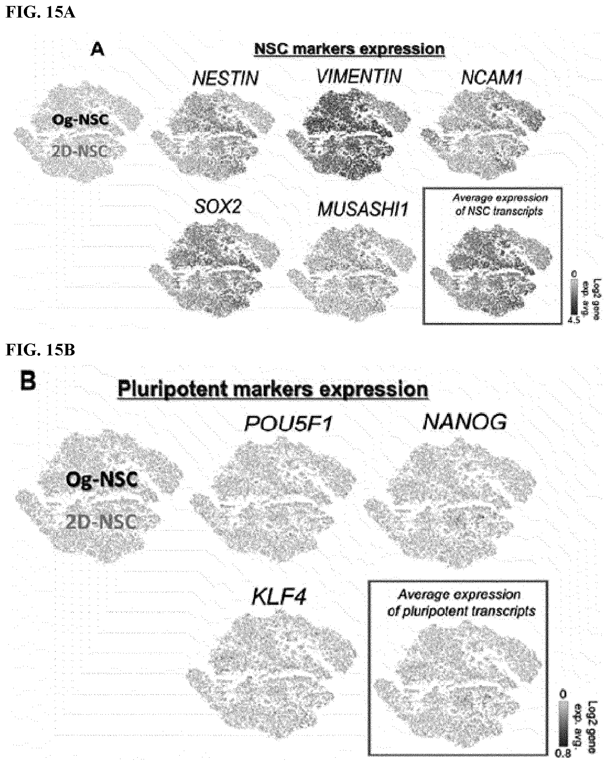

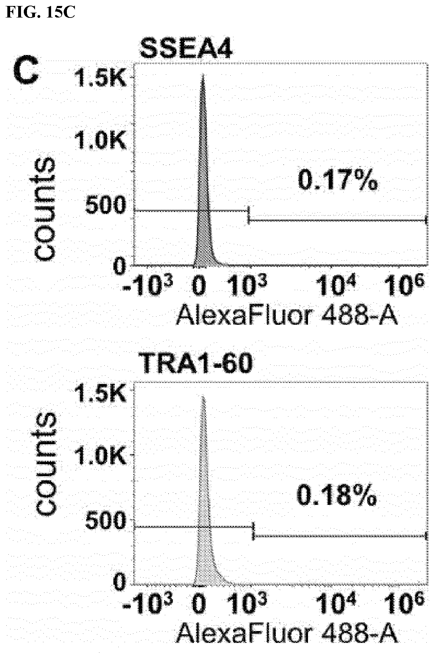

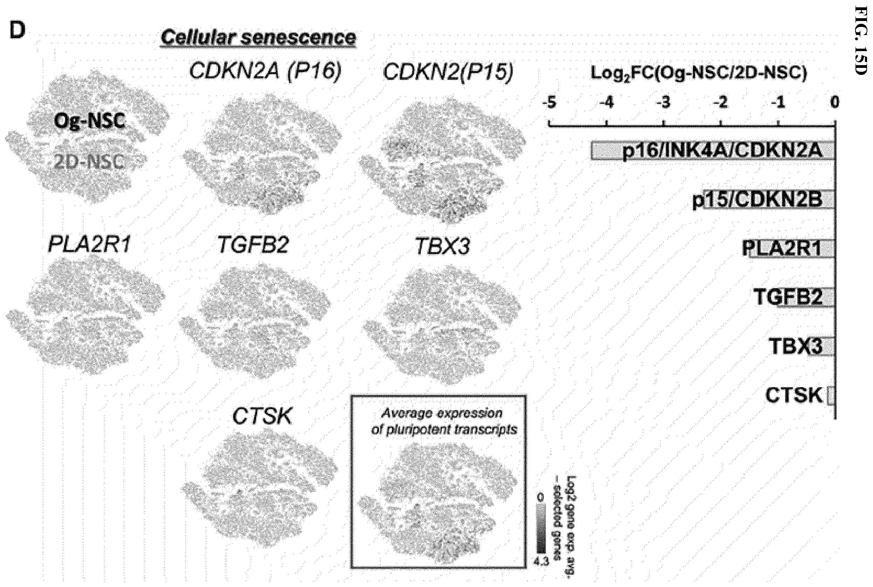

In order to confirm the properties of each individual cell in the neural stem cell colony, single-cell RNA-seq was performed (Og-NSCs : a total of 13,793 cells analyzed; 2D-NSCs : 12,166 cells ;. On average, there were 3.8 × 10⁵ post-normalization reads per cell and 2,227 genes detected in each individual cell). On average, there were 3.8 × 10⁵ post-normalization reads per cell and 2,227 genes detected in each individual cell. As expected, ∼ 99.7% of Og-NSCs and the conventional 2D-NSC cells expressed the neural stem cell markers NESTIN, VIMENTIN, NCAM1, SOX2, NOTCH1, and MUSASHI1 (FIG. 15A). POU5F1, NANOG, and KLF4, markers of totipotent cells, were expressed only in 0.09%, 0.45%, and 0.91% of Og-NSC cells, and even in the cells expressing the markers, the expression levels of the markers were insignificant (average unique molecular identifiers (UMI) were <0.005) (FIG. 15B). Although most of them were neural stem cells, the conventional 2D-NSCs showed a relatively slightly high expression of totipotent cells (0.19%, 2.69%, and 1.24%, respectively, with an average UMI of <0.031). Since there are no cells simultaneously expressing the three markers for both Og-NSCs and 2D-NSCs, this shows that only neural stem cells can be isolated and cultured. Through FACS analysis, it was confirmed that there was almost no expression of TRA-1-60 and SSEA-4 (FIG. 15C). Previously, it was analyzed that the number of senescent cells (pre-apoptotic stage) was also significantly reduced in Og-NSC culture (FIG. 1L), and the number of cells expressing apoptosis genes was also very small (FIG. 15D). In particular, a very low proportion of Og-NSC cells compared to 2D-NSC cells expressed p16/INK4a, which is an important characteristic for cell aging/senescence and reduction of stem cell repair ability in brain (Molofsky, A.V, Slutsky, S.G., Joseph, N.M., He, S., Pardal, R., Krishnamurthy, J., Sharpless, N.E., and Morrison, S.J. (2006). Increasing p16INK4a expression decreases forebrain progenitors and neurogenesis during ageing. Nature 443, 448-452.; Nishino, J., Kim, I., Chada, K., and Morrison, S.J. (2008). Hmga2 promotes neural stem cell self-renewal in young but not old mice by reducing p16Ink4a and p19Arf Expression. Cell 135, 227-239.) and other tissues (Sousa-Victor, P., Gutarra, S., Garcia-Prat, L., Rodriguez-Ubreva, J., Ortet, L., Ruiz-Bonilla, V, Jardi, M., Ballestar, E., Gonzalez, S., Serrano, A.L., et al. (2014). Geriatric muscle stem cells switch reversible quiescence into senescence. Nature 506, 316-321.; Wang, J., Lu, X., Sakk, V, Klein, C.A., and Rudolph, K.L. (2014). Senescence and apoptosis block hematopoietic activation of quiescent hematopoietic stem cells with short telomeres. Blood 124, 3237-3240.).

The Og-NSCs cell populations analyzed by t-SNE (t-distributed stochastic neighbor embedding) analysis can be classified into three clusters (FIGs. 2C to 2E). In order to confirm the properties of each cell cluster, GO (gene ontology) analysis of the gene group with increased expression was performed (UMI>1, log₂FC>0.5, P<0.05) (FIG. 2F). Cluster 3 occupies 25% (Og-NSC) and 12% (2D-NSC) (FIG. 2D). As a result of genetic analysis, it is a cell population that has already been destined to be a neuron, and contains a significant number of differentiation factors. In the case of the gene group overexpressed in cluster 1, a large number of gene groups important for the development of the substantia nigra (SN), where dopamine neurons are generated, are expressed (ENO3, RSPO2, FGF9, LDHA, NR4A2, INA, LMX1A, OTX2, HSPA5, SYNGR3, RAD1, UCHL1, EN1, NDUFS3, and SEC16A). Therefore, cluster 1 can be said to be the most complete cell population for patterning into the midbrain. In particular, 71% of Og-NSCs cells and 13% of the existing 2D-NSCs cells belong to cluster 1. As a result of this genetic analysis, it was also shown that Og-NSC culture had a much more midbrain-type patterning, and it was possible to stably secure a cell population without contamination of totipotent cells and without apoptosis.

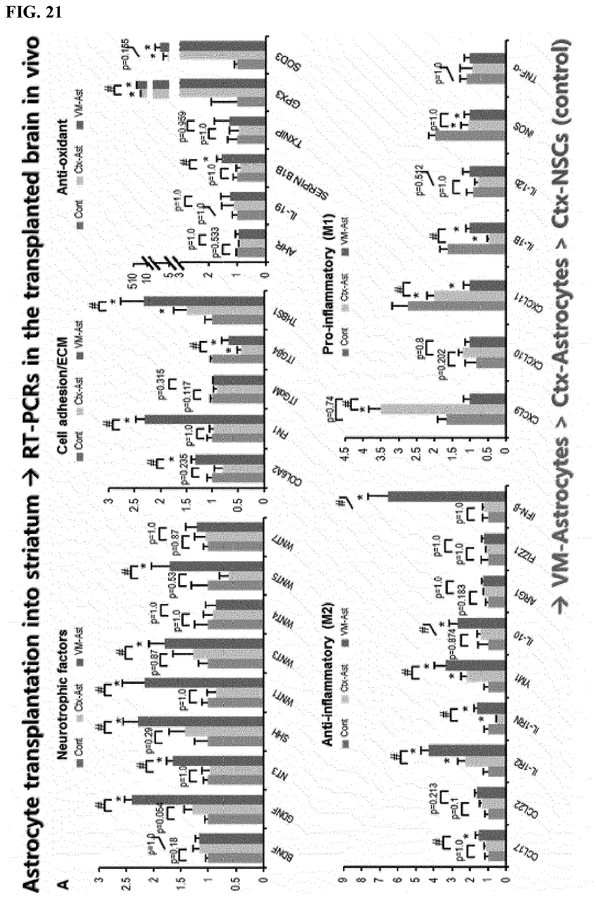

Through GO and KEGG analysis, bulk and single cell RNA-seq data (Og-NSCs vs 2D-NSCs) was analyzed in more detail (FIGs. 2H and 21). According to the seq data of the two methods, the gene group that is relatively frequently expressed in Og-NSCs (compared to 2D-NSCs) is a gene group of cell adhesion and ECM that emphasizes the importance of the development of stem cells and maintenance of function. By seq data of the two methods, the expression of this gene group, which is highly expressed in both methods, was studied by unsupervised hierarchal clustering (FIG. 2J). Its special functions were also described (Table 5).

**[Table 5]**

| **Cell-cell adhesion** | | |
|---|---|---|
| **Gene ID** | **Alias** | **Functions** |
| HSP90AB1 | Heat Shock Protein HSP 90-Beta | involved in signal transduction protein folding and degradation |
| DBN1 | Developmentally-Regulated Brain Protein | a cytoplasmic actin-binding protein which plays a role in the process of neuronal growth |
| SERBP1 | SERPINE1 mRNA Binding Protein 1 | plays a role in the regulation of mRNA stability |
| CCT8 | Chaperonin Containing TCP1 Subunit 8 | involved in the transport and assembly of newly synthesized proteins |
| RDX | Radixin | a cytoskeletal protein important in linking actin to the plasma membrane |
| KIF5B | Kinesin Family Member 5B | micrombule-dependent motor required for normal distribution of mitochondria and lysosomes |
| MACF1 | Microtubule-Actin Crosslinking Factor 1 | acts as a positive regulator of Wnt receptor signaling pathway |
| EIF4G2 | Eukaryotic Translation Initiation Factor 4 Gamma 2 | plays a role in the switch from cap-dependent to IRESmediated translation during mitosis |
| HNRNPK | Heterogeneous Nuclear Ribonucleoprotein K | plays an important role in p53/TP53 response to DNA damage |
| HSPAS | Heat Shock 70-kDa Protein 8 | plays a pivotal role in the protein quality control system |
| ZC3H15 | Zinc Finger CCCH-Type Containing 15 | protects DRG1 from proteolytic degradation |
| BZW1 | Basic Leucine Zipper and W2 Domains 1 | enhances histone H4 gene transcription |
| RSL1D1 | Ribosomal L1 Domain Containing 1 | regulates cellular senescence through inhibition of PTEN translation |
| HSP90AB1 | Heat Shock Protein HSP 90-Beta | involved in signal transduction protein folding and degradation |
| DBN1 | Developmentally-Regulated Brain Protein | a cytoplasmic actin-binding protein which plays a role in the process of neuronal growth |

| **Neuron maturity** | | |
|---|---|---|
| **Gene ID** | **Alias** | **Functions** |
| (continued) | | |
| CADPS | Calcium-dependent secretion activator I | calcium-binding protein involved in exocytosis of vesicles |
| P2RX7 | P2X purmoceptor 7 | receptor for ATP that acts as a ligand-gated ion channel |
| CADPS2 | Calcium-dependent secretion activator 2 | regulates the exocytosis of synaptic and dense-core vesicles in neurons and neuroendocrine cells |
| SYT10 | Synaptotagmin 10 | calcium sensors in the regulation of neurotransmitter release and hormone secretion |
| UNC13C | Unc-13 Homolog C | plays a role in vesicle maturation during exocytosis |
| RIMS1 | Regulating synaptic membrane exocytosis protein 1 | synaptic vesicle protein that regulates synaptic vesicle exocytosis |
| PCLO | Protein piccolo | regulates the cycling of synaptic vesicles Similar role to Bassoon RIMS12 |
| KCNMB3 | Calcium-activated potassium channel subunit beta-3 | modulates the calcium sensitivity and gating kinetics of KCNMA1 |
| SCN1A | sodium channel voltage-gated type I. alpha subunit | mediates the voltage-dependent sodium ion permeability of excitable membranes |
| KCND2 | Potassium voltage-gated channel subfamily D member 2 | mediates transmembrane potassium transport in excitable membranes |
| ANK3 | Ankyrin-3 | plays key roles in cell motility, activation and proliferation |
| SCN2A | sodium channel voltage-gated type II. alpha subunit | responsible for the generation and propagation of action potentials in neurons and muscle |
| SCN9A | sodium channel voltage-gated type 9. alpha subunit | mediates the voltage-dependent sodium ion permeability of excitable membranes |
| GPER1 | G protein-coupled estrogen receptor 1 | stimulates cAMP production calcium mobilization and tyrosine kinase Src |
| MYOSA | Myosin-Va | a class of actin-based motor proteins involved in cytoplasmic vesicle transport |
| GRIK1 | Glutamate receptor ionotropic kainate 1 | mediates excitatory neurotransmission and is critical for normal synaptic function |
| AKAP9 | A-kinase anchor protein 9 | required to maintain the integrity of the Golgi apparatus |
| GABBR2 | Gamma-aminobutyric acid (GABA) B receptor. 2 | regulates the release of neurotransmitters and the activity of ion channels |
| PMCHL1 | Pro-Melanin Concentrating Hormone-Like 1 | pseudogene found in fetal newborn and adult brain |
| KCNQ5 | Potassium voltage-gated channel subfamily KQT member 5 | associates with KCNQ3 to form a potassium channel |
| PCDHB16 | Protocadherin beta-16 | plays a critical role in the establishment and function of specific cell-cell neural connections |
| DLG2 | Disks large homolog 2 | part of the postsynaptic protein scaffold of excitatory synapses |
| PCDHB9 | Protocadherin beta-9 | play a critical role in the establishment and function of specific cell-cell neural connections |
| PTPRD | Receptor-type tyrosine-protein phosphatase delta | promotes neurite growth, and regulating neurons axon guidance |
| CEP89 | Centrosomal protein 89 | plays a role in mitochondrial metabolism |
| PCDHB3 | Pforocadherin beta-3 | plays a critical role in the establishment and function of specific cell-cell neural connections |
| GRIN2A | Glutamate [NMDA] receptor subunit epsilon-1 | ionotropic glutamate receptor |
| PDYN | Prodynorphin | preproproteins of ligands for the kappa-type of opioid receptor |
| HOMER1 | Homer protein homolog 1 | plays a role in synaptic plasticity |
| GRM5 | Metabotropic glutamate receptor 5 | involved in the regulation of neural network activity and synaptic plasticity |
| ATXN3 | Ataxin-3 | deubiquitinating enzyme involved in protein homeostasis maintenance |
| GRIA2 | Glutamate ionotropic receptor AMPA type subunit 2 | function as ligand-activated cation channels |
| LRP6 | Low-density lipoprotein receptor-related protein 6 | a co-receptor with LRP5 for transducing signals by Wnt proteins |
| RIT2 | GTP-binding protein Rit2 | binds and modulates the activation of POU4F1 |
| HTR2A | 5-Hydroxytryptamine Receptor 2A | 5-HT_{2A} receptor |
| OPRD1 | 3-opioid receptor | inhibits neurotransmitter release by reducing calcium ion currents |
| DCC | Deleted in Colorectal Carcinoma | mediates axon guidance of neuronal growth cones toward sources of netrin 1 ligand |
| KIF5B | Kinesin Family Member 5B | microtubule-dependent motor required for normal distribution of mitochondria and lysosomes |
| LMX1A | LIM Homeobox Transcription Factor 1 Alpha | plays a role in the development of dopamine-producing neurons during embryogenesis |
| PTPRO | Receptor-type tyrosine-protein phosphatase O | plays a role in the inhibition of cell proliferation and facilitation of apoptosis |
| SHH | Sonic hedgehog | key inductive signal in patterning of the ventral neural tube |
| EPHA5 | EPH Receptor A5 | functions as an axon guidance molecule during development |
| WNT3 | Wnt Family Member 3 | functions in the canonical Wnt signaling pathway |
| ISPD | Isoprenoid Synthase Domain Containing | catalyzes the formation of CDP-ribitol nucleotide sugar from D-ribitol 5-phosphate |
| SOS1 | SOS Ras Rac Guanine Nucleotide Exchange Factor 1 | guanine nucleotide exchange factor for RAS proteins. membrane proteins |
| SPTBN1 | Spectrin Beta. Non-Erythrocytic 1 | actin crosslinking and molecular scaffold protein |
| OPHN1 | Oligophrenin 1 | Rho-GTPase-activating protein |
| CNTN4 | Contactin 4 | axon-associated cell adhesion molecule |
| UNC5D | Unc-5 Netrin Receptor D | plays a role in cell-cell adhesion and cell guidance |
| SPTA1 | Spectrin Alpha. Erythrocytic 1 | links the plasma membrane to the actin cytoskeleton |
| CHL1 | Cell Adhesion Molecule L1 Like | plays a role in nervous system development and in synaptic plasticity |
| FEZ2 | Fasciculation and Elongation Protein Zeta 2 | involved in axonal outgrowth and fasciculation |
| MYH10 | Myosin Heavy Chain 10 | plays a role in cytokinesis cell shape |
| SPTB | Spectrin Beta Erythrocytic | plays a role in cell membrane organization and stability |

In addition, the gene group that is specifically highly expressed in the Og-NSC cell population is 'SN development' and 'negative regulation of cell death' (FIG. 21) related to midbrain patterning. In the analysis of single cell RNA-seq analysis, in particular, it was found that the gene group related to the biological function of mitochondria was largely regulated in the Og-NSCs group (mitochondrial function, ER and protein folding, and autophagy). This can emphasize the importance because the mitochondria are closely related to the organ that is typically affected in the context of neurodegenerative diseases closely related to cell senescence and death. The correlation can be inferred even in KEGG analysis through this data as it is ranked as the top change in 'Parkinson's disease,' 'Huntington's disease,' and 'Alzheimer's disease,' which are representative neurodegenerative diseases (FIG. 21).

The function of mitochondria is very important in the differentiation of human embryonic stem cells and induced pluripotent stem cells. In particular, the mitochondria of neural stem cells are known to be very important in the progression and pathogenesis of neurodegenerative diseases. Mitochondrial genes were all regulated in Og-NSCs, including those related to mitochondrial generation and dynamics, in two different bulk and single-RNA-seq analyses compared to cells induced by conventional methods (FIG. 3A). Similar to genetic analysis, it can be analyzed with Mitotimer, and it was found that the Og-NSCs cell population was much healthier and functionally superior than the conventional 2D-NSCs (FIG. 3B). The healthy and functional superiority of Og-NSCs can also be seen by mitochondria ROS analysis (MitoSox, FIG. 3C). In addition, since the Og-NSC group also had a high mitochondrial membrane potential, it was shown to be superior in all aspects (JC-1, FIG. 3D). In addition to improved mitochondria turnover and antioxidant capacity, mDA neurons differentiated from Og-NSCs showed greater resistance to apoptosis (FIG. 3E) and neurite degeneration (FIG. 3F) induced by various mitochondria toxins than those derived from 2D-NSCs.

### Dopaminergic neuron mDA neurons differentiation-induced from Og-NSCs coexist with astrocytes, resulting in improved neuronal function and maturation

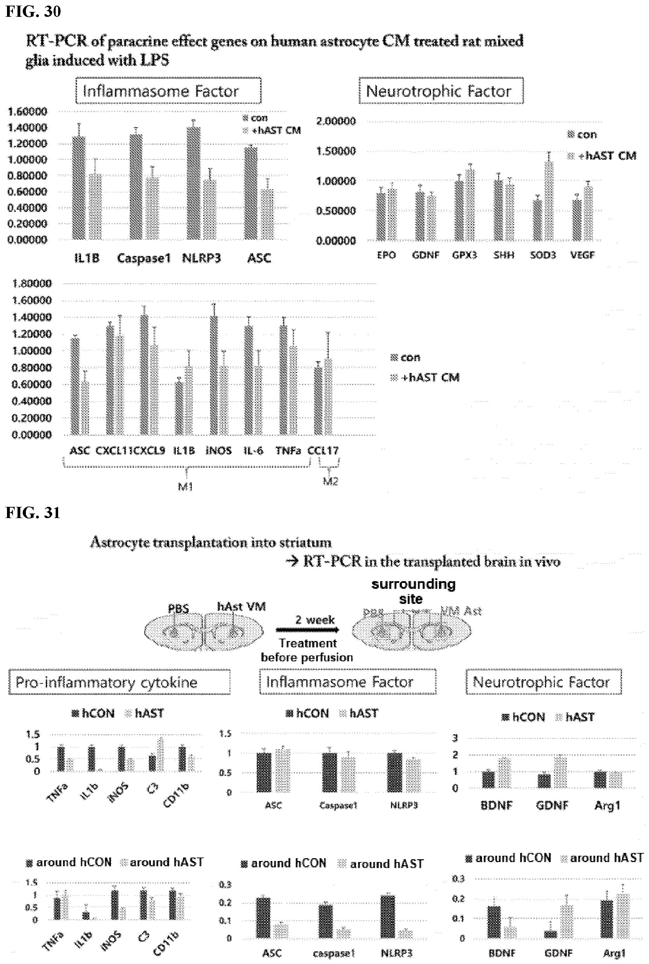

The transcriptional regulation of genes actually shows early changes before the shape of the cell population changes. For example, in actual genetic analysis (in differentially expressed genes (DEGs)), the change that can be seen from neural stem cells is one that can be seen from the embryonic stem cell stage. In bulk RNA-seq analysis, genes related to neuronal maturation were highly expressed in Og-NSCs (vs 2D-NSCs) cells (FIGs. 2H and 3G). As in the gene expression profile, compared to their 2D counterparts, midbrain-type dopamine neurons differentiation-induced from Og-NSCs showed a more mature morphology after neuron differentiation (FIGs. 3H and 3J), and showed a more mature morphology in terms of synapse formation (FIGs. 31 and 3K), action potential-induced Ca²⁺ dynamics (FIGs. 3L to 3O), pre-synaptic dopamine neuron function evaluated by depolarization induced dopamine secretion (FIG. 3P).

In all currently existing hPSC-mDA differentiation protocols, mDA neurons are generated without astrocyte differentiation. Similarly, no astrocyte differentiation was found in cultured 2D-NSCs (FIGs. 3R and 3S). Interestingly, 17% of the cells in the subpopulation of Og-NSCs cluster 1 (midbrain-type SN generating cell population, cluster with higher SN developmental gene expression) showed a characteristic of high expression of genes (EIF2B5, MT3, PLP1, CDK6, SCL1A3, HES1, HES5, and SOX8) related to glia development in common (FIG. 3Q). It was shown that Og-NSC culture contained a VM (SN)-type glia progenitor population. Consistently, some of the cells in Og-NSC culture expressed CD44 (FIG. 3S), which is specifically found in neurogenetic progenitor cells (FIG. 3R) that are differentiated into astrocytes during peripheral differentiation. These cells express CD44, a marker of astrocyte progenitor cells (FIG. 3R), and thus can be expected to be differentiated into astrocytes during final differentiation in the future (FIG. 3S). It can be seen that, like the actual pattern of midbrain development, mDA neurons are generated along with GFAP+ astrocytes (FIG. 3S).

Therefore, mDA neurons were generated along with GFAP + astrocytes in the differentiated culture as in the midbrain *in vivo* (FIG. 3S). In addition to the general neurotrophic action of astrocytes, VM-type astrocytes are known to exert specific dopaminotrophic functions superior to astrocytes from other brain regions.

Considering the cytokine secretion ability of astrocytes based on previous reports, astrocytes can be expected to have a positive effect when dopaminergic neurons are generated. Based on this information, as shown in FIGs. 1E to 1L and 3E to 3P, adjacent astrocytes can exert neurotrophic support for mDA neuron maturation, survival, pre-synaptic function and midbrain-specific factor expression.

### Dopaminergic neurons differentiated from Og-NSCs are less prone to α-syn oligomerization and pathogenic propagation.

In order to prevent the propagation of α-synucleinopathy existing in the original patient's brain into transplanted cells, and for the long-term success of cell therapy for Parkinson's disease, it is important to secure the α-syn propagation resistance of cells to be used (transplanted) as a cell therapeutic agent. Toxic α-syn pathogenic propagation is associated with intercellular transfer of α-syn, and α-syn aggregation is also associated with transfer into transplanted cells. In order to measure the intercellular transfer ability of α-syn, Og-NSCs and 2D-NSCs were analyzed using a dual chamber system (Choi, Y.R., Cha, S.H., Kang, S.J., Kim, J.B., Jou, I., and Park, S.M. (2018b). Prion-like Propagation of alpha-Synuclein Is Regulated by the FcgammaRIIB-SHP-1/2 Signaling Pathway in Neurons. Cell reports 22, 136-148), respectively. GFP-labeled α-syn (a protein overexpressed in SH-SY5Y neuronal cells) was placed on the chamber, and the cultured Og-NSC (2D-NSC)-derived neurons were placed under the chamber, and uptake and expansion was observed after protein transport (FIG. 4A). As a result, the fluorescence intensity of GFP-labeled α-syn was observed to be significantly lower in Og-NSC derived neurons (FIG. 4B).

In addition to α-syn transfer, the pathogenic propagation of α-syn into transplanted cells, which is another important issue, was analyzed by toxic α-syn aggregates. The degree of aggregation was compared and analyzed in an environment in which the amount of α-syn inside the cell is increased or in a condition in which α-syn is introduced from the outside (exogenous α-syn fibril seed).

Og-NSCs and 2D-NSCs cell populations were infected with a lentivirus overexpressing α-syn, and oxidative stress was induced with H₂O₂ after differentiation of dopaminergic neurons, and toxic α-syn aggregation and propagation were analyzed. Phosphorylated α-syn at serine 129 (p129-α-syn), which may be a toxic α-syn, or GFP+ α-syn transfer was observed to be less in Og-NSCs (FIG. 4C). Therefore, toxic α-syn aggregation was also observed to be less in the differentiated Og-NSC cell population (FIG. 4D, left). In addition, as a result of observing toxic α-syn aggregation by preparing the condition in which α-syn is introduced from the outside (exogenous α-syn fibril seed) by injection of α-syn fibril (PFF), it was found that toxic α-syn aggregation was higher than in the conventional 2D-NSCs (FIG. 4D, right), and toxic α-syn aggregation under various conditions was also observed to be less in the Og-NSC cell population. Next, toxic α-syn aggregation was observed by a Bi-FC (bimolecular fluorescence complementation) system. This system is a system for observing α-syn by showing intense fluorescence when the amino (N) terminus (VIS) or carboxy (C) terminus (SV2) Venus fragment is expressed and aggregated, respectively (FIGs. 4E and 4F). Oxidative stress and mitochondria destruction are known to induce protein misfolding and aggregation of α-syn. The relative resistance of Og-NSC-derived dopaminergic neurons to α-synucleinopathy can be attributed to low oxidative and mitochondrial stress (FIGs. 3B to 3F). In addition, the inclusion of astrocytes in the differentiated Og-NSC cell population can also reduce toxic α-syn aggregation to an original function of astrocytes, and thus it can be seen that it contributes to the reduction of α-synucleinopathy. Overall, Og-NSCs derived neurons have relatively inhibited toxic α-syn propagation, which was confirmed by the study of α-syn inside and outside the cell.

Finally, Og-NSCs or 2D-NSCs were transplanted into the brain tissue *in vivo,* and α-syn propagation and aggregation in the actual brain environment were observed. Human specific α-syn overexpressing AAV virus and PFF were injected into the rat striatum to induce Parkinson's disease caused by α-syn disease. After two weeks, Og-NSCs and 2D-NSCs were transplanted into the model animal striatum, respectively, and differences within the *in vivo* brain environment were observed. As a result of analysis of the transplanted tissue after one month, under a serious α-synucleinopathy environment, the conventional 2D-NSC-derived transplantation site had almost no surviving dopaminergic neurons (FIG. 4G, lower panel), Lewy bodies in which α-syn+ and p129-α-syn+ were co-expressed were observed, and neurites were hardly differentiated. Therefore, it showed that the conventional cell population was not suitable as a cell therapeutic agent. On the other hand, in the case of the newly developed Og-NSC cell population derived transplantation site, the shape of the neurons was much healthier and there was no serious neurite degeneration (FIG. 4G, upper panel). Moreover, toxic α-syn+ (p129-α-syn+) aggregation was observed to be less at various sites in neurons. In the case of patients with Parkinson's disease, even after transplantation of a cell therapeutic agent, the propagation of patient derived α-syn into transplanted cells (host-to-graft α-syn propagation) has been a serious problem during patient survival (11 to 16 years after transplantation). In the case of animal models, α-syn propagation aggregation is observed at a much faster period of about one month. Although it does not fully reflect the patient's brain environment, the results of *in vitro* and *in vivo* assays show that Og-NSCs derived cells are more resistant to toxic α-syn propagation and aggregation.

### Og-NSC transplantation provides healthy dopaminergic neurons in Parkinson's disease animal model

Finally, the possibility of Og-NSCs derived cell population as a cell therapeutic agent was observed after transplantation in a model animal system in which Parkinson's disease was induced by 6-OHDA (6-hydroxydopamine). Recently, there has been a study on genes that can expect the success after transplantation of human embryonic stem cell derived cells. As expected, as a result of single cell RNA-seq analysis, it was found that this gene group was expressed relatively highly in the newly developed Og-NSCs cell population (compared to the conventional 2D cell population) (FIG. 5A). The synaptic plasticity of transplanted dopaminergic neurons is very important for cell survival and success of transplantation in the future. In order to analyze their function as neurons, GFP fluorescence-labeled GCaMP6s (calcium indicator protein, which emanates green fluorescence by Ca++ binding) was expressed in the Og-NSCs cell population and then transplanted into the brain striatum. The function of the neurons was observed by two-photon endomicroscopy at one month after transplantation without special stimulation to the neurons (FIG. 5B, top). In the transplanted Og-NSCs cells, spontaneous signal transduction through neurons was observed. Ca²⁺ transients (ΔF/F) show an action potential frequency of 2-5 Hz of neurons (FIG. 5B, bottom), which is similar to that of dopaminergic neurons that originally occurred in SNpc (substantia nigra pars compacta) of the brain tissue. In order to further confirm the synaptic plasticity of the transplanted Og-NSC cells, Og-NSCs were prepared from a human embryonic stem cell population expressing the gene with GFP attached to delta-like canonical Notch ligand 1 ( *Dll1;* pDll1-GFP-hESC). Since they are fluorescently expressed only in differentiated neurons, only cells expressing fluorescence were analyzed after cell transplantation into a Parkinson's disease model animal. After one and a half months, in the patch clamp analysis, action potentials (FIG. 5C) and spontaneous excitatory postsynaptic currents (sEPSCs) (FIG. 5D) were observed in the transplanted GFP-expressing neurons. The synaptic plasticity of transplanted neurons was not significantly different from that of normal cells without lesions on the other side (FIGs. 5E and 5F), which indicates that, even after transplantation of Og-NSCs, neurons had a functionally perfect morphology. In order to find out whether the transplanted dopaminergic neurons are fully mature and have perfect synaptic functions such as dopamine secretion, dopamine uptake by dopamine transporters was observed by [¹⁸F]FP-CIT PET scan. As a result, the striatum of Parkinson's disease animals showed low dopamine uptake (2% of the intact hemisphere), but relatively recovered up to 40% of the normal level 5 months after transplantation of Og-NSCs cell population (FIGs. 5G and 5H).

Og-NSCs cell populations were transplanted into a Parkinson's disease animal model, and behavioral studies were performed. As a result, the amphetamine-induced rotational behavior analysis showed a consistent recovery effect. At ~6 months of transplantation, behavioral recovery (rotation scores) in 11 of 14 animals was >50% of that before transplantation. In the case of 8 animals observed at 6 months after transplantation, all animals showed >79% results (FIG. 6A). In the transplanted animals, the recovery effect was also shown in the stepping test (FIG. 6B) and the cylinder test (FIG. 6C).

6 months after transplantation of Og-NSCs into a model animal, the results were analyzed. Healthy dopaminergic neuron tissue was confirmed at the transplantation site (graft volume: 4.50 ± 1.56 mm3, TH+ cells: 9031 ± 3773 cells, n=8 from 6 rounds of independent transplantation experiments, FIGs. 6D to 6O). No consistent specific proliferation was observed at the transplantation site. This is the same as the differentiation into Og-NSCs derived dopaminergic neurons *in vitro* (FIGs. 2H, and 3G to 31). In particular, the transplanted dopaminergic neurons maturely form synapses with cells in the original brain tissue and extend normal neurites (FIG. 6D). Although midbrain specific factors are very important for the survival and maturation of dopaminergic neurons, it is known that their expression is not maintained well after transplantation. For this reason, most of the existing papers did not show the expression of the entire midbrain-type factors in the tissue after transplantation, but showed only the image of the maximum FOXA2/TH+ expressing cells. However, the transplanted cell population derived from Og-NSCs developed by the present protocol expresses all important midbrain-specific factors. Even 6 months after transplantation, 96.7% (3,106/3,196, FOXA2), 93.8% (3,338/3,549, LMX1A), 95.0% (3,496/3,678, NURR1), 91.5% (2,989/3,274, EN1) of major midbrain-specific factors are co-expressed with TH+, a marker of dopaminergic neurons (from 8 animals; FIGs. 6E to 61). In particular, GIRK2, a marker of A9 nigral dopaminergic neurons, was expressed (FIG. 6J), and as with the main characteristics of A9 nigral dopaminergic neurons, the transplanted dopaminergic neurons had a mature morphology that extends neurites in multiple directions (FIGs. 6E to 6G and 6H, the lower drawing and FIG. 6P), and they also extended mature neurites with the original brain tissue cells (FIG. 6D). Human GFAP (hGFAP)-expressing astrocytes are clustered around the transplanted dopaminergic neurons (FIG. 6K), which indicates that the transplanted Og-NSC-derived astrocytes help dopaminergic neurons to form synapse by extending neurites and to help the maturation of the cells. It is the same as the previous co-transplantation of astrocytes showed similar effects. In order to confirm the effect in primates, the Og-NSCs derived cell population was transplanted into the striatum of two adult Philippine monkeys, and one month later, mature dopaminergic neurons co-expressing FOXA2 and NURR1, important midbrain-type markers, were identified (FIG. 6P).

### Discussion

Cultured organoids may be applied to tissue development and human disease modeling studies and may be also a source of donor transplantation in regenerative medicine. However, because transplantation of brain organoids into deep brain regions is not feasible without damaging host brain tissue, transplantation of brain organoids will not be applicable for the treatment of brain disorders. Moreover, the therapeutic outcome of transplanted brain organoids should be able to establish new neural networks in which self-organized structures in transplanted organoids interact with the host brain in a very precise manner. In contrast, transplanted organoids from other tissues (liver, intestine, kidney, pancreas, etc.) may exert therapeutic functions as isolated functional units (liver buds, pancreatic islets, renal nephrons, etc.) after transplantation. Therefore, although organoid transplantation may have the potential to treat other extra-CNS disorders, direct transplantation of brain organoids to treat brain disorders is unreasonable.

All tissue developments are achieved through sequential generation processes, proliferation of tissue-specific stem/progenitor cells and their differentiation into tissue-specific cells. Cultured tissue-specific stem/progenitor cells can provide donor cells for regenerative medicine as well as bioassay platforms for developmental studies and drug screening. However, the induction of NSC cultures with appropriate VM-patterning from hPSCs was not achieved in previous studies. The present inventors realized that the difficulty in preparing VM-specific NSC cultures was due to the instability of VM region specificity in the culture. Since maintenance of VM-specific marker expression is very sensitive to cell density, it is easily lost during the cell isolation and replating process required for NSC preparation (FIG. 9A).

Therefore, the hPSC-mDA neuron protocol was developed with very high cell densities or specific plate coating materials such as Laminin-511 or 111 (Biolamina, LN-511, LN-111). Various efforts using various cell dissociation conditions (Acutase, collagenase, Ca++, Mg++-free HBSS), cell survival cytokines (TGFβ, doxycycline, cAMP, ascorbic acid, Y27632), chemicals that induce open epigenetic conditions (5-azacytidine, VPA, TSA), β-estradiol or DAPT (Notch inhibitor) failed to solve the problem.

In a recent study (Song, J.J., Oh, S.M., Kwon, O.C., Wulansari, N., Lee, H.S., Chang, M.Y, Lee, E., Sun, W., Lee, S.E., Chang, S., et al. (2018). Cografting astrocytes improves cell therapeutic outcomes in a Parkinson's disease model. The Journal of clinical investigation 128, 463-482.), the present inventors observed that factors secreted from cultured astrocytes, particularly factors derived from VM tissues, exert strong trophic effects to promote cell survival and midbrain-specific factor expression in primary NSC and mDA neuron cultures.

Therefore, the 2D-NSC culture protocol used this study could be developed through the treatment of conditioned medium prepared from cultured astrocytes (ACM) (FIG. 9). However, the induction of 2D-NSC culture still had the aforementioned stability and reproducibility issues. In contrast, NSCs with stable VM marker expression were readily isolated and cultured from rodent embryonic VM tissues, stimulated to isolate human VM-specific NSCs from hPSC derived midbrain-like organoids, and had a constructed structure and region specificity in an environment similar to the physiological structure of the embryonic brain.

Organoid-based formulations have had great success with respect to stability of VM-patterns in NSC cultures with faithful midbrain-specific marker expression, as well as general culture stability with very low levels of apoptosis, senescence, oxidation and mitochondrial stress. Another notable observation was that Og-NSCs contain an astrocyte progenitor cell population with a VM-specific gene expression profile in which Og-NSCs are differentiated into astrocytes mixed with mDA neurons. In contrast, mDA neuron differentiation was induced without astrocyte differentiation in all existing methods for hPSC-mDA differentiation, and consequently, there were no astroglia in differentiated mDA neuron cultures. Therefore, in the absence of astroglial support, nerve maturity and function could not be expected to be identical to that of differentiated mDA neurons in their *in vivo* counterparts. Indeed, insufficient maturation and function have been proposed as critical drawbacks in the utility of mDA neurons differentiated from hPSC in *in vitro* disease modeling and therapy. In contrast, under nutritional support from mixed astrocytes, Og-NSC-derived mDA neurons exhibited an improved set of improved synapse maturation, functionality, resistance to toxic insult, and long midbrain specific factor expression *in vivo* and *in vivo* after transplantation. All these properties of Og-NSCs ultimately contribute to superior and long-term therapeutic efficacy after transplantation in a reproducible manner. In addition, we have shown that organoid-derived NSCs are an expandable cell source needed for the preparation of donor cells for therapy, as well as bioassay platforms for drug screening, developmental studies and disease modeling.

Based on the present invention, organoid-based methods may become a common next-generation strategy to prepare tissue specific stem/progenitor cells with strong therapeutic potential for CNS as well as non-CNS disorders.

In other words, there is no doubt that the cells of organoids will be healthier compared to the two-dimensional culture and that excellent results will be obtained during cell therapy. Since it is not desirable to directly transplant organoids in the form of 'mass' due to the characteristics of brain tissue, the present invention can be said to be the most appropriate model of a cell therapeutic agent for the nervous system.

### Example 2: Differentiation from human pluripotent stem cells into cerebral cortex neural stem cells using cerebral cortex organoids

### [Experimental procedure]

### Culturing of human pluripotent stem cells (hESCs/hiPSCs)

hESCs and hiPSCs were cultured based on the hESC research guidelines approved by the institutional review board (IRB) of Hanyang University (Seoul, Republic of Korea).

The hESCs and hiPSCs used in this experiment are shown in Table 6 below.

**[Table 6]**

| **hESC line** | | | | | | | | |
|---|---|---|---|---|---|---|---|---|
| **Name used in this paper** | | **Original name** | | | **Karyotype** | | **Establishing institute** | |
| HSF6 | | OC06 | | | Female (46, XX) | | UCSF | |
| H9 | | WA09 | | | Female (46, XX) | | Wi cell | |
| HUES6 | | HUES6 | | | female (46, XX) | | Harvard university(HSCI iPS Core) | |

| **hiPSC line** | | | | | | | | |
|---|---|---|---|---|---|---|---|---|
| **Name used in this paper** | **Original name** | | **Source (Human)** | **Reprogamming factors** | | **Methods** | | **Establishing institute** |
| Retro-1 | Rv-hiPS 01-1 | | Newborn fibroblast | OCT4, SOX2, KLF4, MYC | | Retrovirus | | Harvard |
| Epi-ips | episomal-ips1 | | Skin | OCT4, SOX2, KLF4, shp53 | | Episomal vector | | Hanyang Univ. (Our own) |
| CMC-ips#11 | CMC-hipsc-011 | | bone narrow blood | OCT4, SOX2, KLF4, MYC | | Sendal virus | | Catholic Univ. (Korea NIH SCRM) |
| PD-ips1 | PD patient derived ips-1 | | Skin | OCT4, SOX2, KLF4, shp53 | | Episomal vector | | Hanyang Univ. (Our own) |
| PD-#22 | Corr. PD-ips#22 | | Skin | OCT4, SOX2, KLF4, shp53 | | Episomal vector and genome edited | | Hanyang Univ. (Our own) |

For proliferation and maintenance of undifferentiation hESC/iPSC, the cells were cultured on Matrigel^{™} or on a vitronectin (Human; Gibco Fisher Scientific, Waltham, MA) (Gibco A31804; 0.5 µg/cm²)-coated 6 cm dish (Thermo Fisher Scientific, Waltham, MA) using mTESR-1 medium (Stemcell Technologies Inc., Vancouver, BC, Canada) in a CO₂ incubator set at 37°C without a feeder layer, and medium replacement was performed daily. The undifferentiated stem cells maintained their differentiation ability by replacing the medium daily, and were subcultured using Acutase (Stemcell Technologies Inc.) every 4 to 5 days.

### Preparation of three-dimensional cerebral cortex organoids

Human embryonic/induced pluripotent stem cells were detachd from the dish using Acutase, placed in a neuron differentiation induction medium, and injected into a low attachment 96-well round bottom plate (Corning, Corning, NY) at 150 µL of 10,000 cells per well (day 0 of differentiation). The composition of the neuron differentiation induction medium is shown in the table below. For the first seeding, KSR-hES media (DMEM 40 ml, KSR 10 ml, 2-mercapto 20 µl, MEM-NEAA 500 µl, GlutaMAX 500 µl) and bFGF (4 ng/ml), ROCK inhibitor Y27632 (20 µM), doxycyclin 1 µg/ml were added. The cells were treated with Y27632 only for 24 hours on the first day. On the second day, B27 without retinoic acid; Insulin (15.6 µl/50 ml); SB431542 (10 µM); LDN (10 µM); and A-83 (10 µM) were added to induce a full-scale nervous system differentiation. Between day 2 and day 3, only 1/2 of the basal medium was used as N2: Neurobasal (1:1, Gibco), and then the entire basal medium was used as N2: Neurobasal (1:1, Gibco) until spread on a plate after chopping in 2D state on day 18. The entire medium was replaced whenever the medium composition was changed. From day 8, bFGF (20 ng/ml) and EGF (20 ng/ml) were added.

Organoids were formed by culturing according to the medium shown in the table below until day 18.

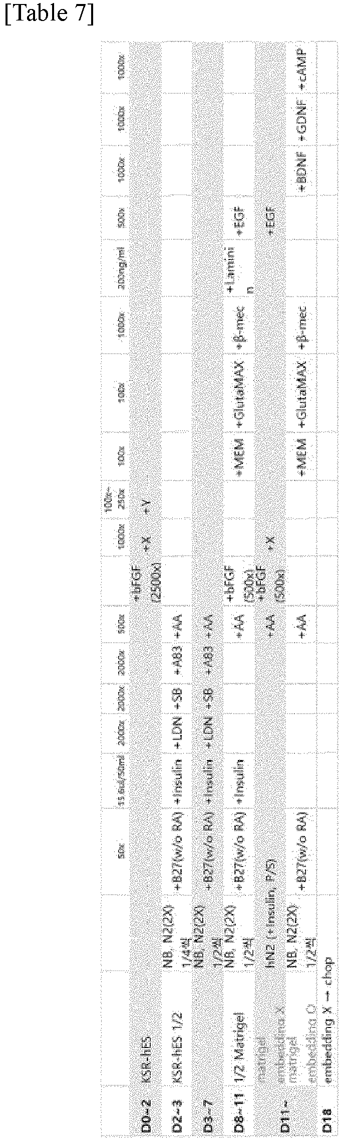

### Isolation of cerebral cortex neural stem cells and astrocyte progenitor cells from cerebral cortex organoids and differentiation into neurons and astrocytes

On day 18, the organoids were digested with Acutase at 37°C for 7 minutes, and then chopped into pieces with a 30 gauge needle, and spread on vitronectin-coated 6 cm plates (Corning, cells from 24 organoids/plate). At this point, the cells were at the neural stem cell (NSC) stage, capable of being proliferated, and subcultured every 5-7 days. The medium used for NSC culture was an N2 expansion medium, and the composition was as follows (IX N2 supplement containing 200 µM ascorbic acid, 20 ng/ml EGF, 20 ng/ml bFGF, and 1 µg/ml doxycycline). The medium was replaced daily and treated with Y27632 at a concentration of 5 µM for one day at the first conversion to two-dimensional culture and at each subculture. The final differentiation into neurons was induced with an N2 differentiation medium (10 ng/ml BDNF, 10 ng/ml GDNF, 200 µM ascorbic acid).

### Isolation and culture of cerebral cortex astrocyte progenitor cells from cerebral cortex organoids

If the passage of the prepared cerebral cortex-type Og-NSCs is further increased according to the actual brain development stage, it is possible to culture Og-astrocyte progenitor cells mainly containing astrocyte progenitor cells after passage 9 without any change in medium composition, etc. Passage 9 to passage 11 can be used in a healthy state. As in cerebral cortex-type Og-NSCs, the proliferation medium was an N2 expansion medium, and the composition was IX N2 supplement containing 200 µM ascorbic acid, 20 ng/ml EGF, 20 ng/ml bFGF, and 1 µg/ml doxycycline. The medium was replaced daily and treated with Y27632 at a concentration of 5 µM for one day at the first conversion to two-dimensional culture and at each subculture. Like organoid-derived astrocyte progenitor cells in the midbrain (see Example 3), cells in the proliferative stage with an older passage are used for transplantation studies. For analysis, the cell population with a high passage with the above final differentiation medium takes on the characteristics of astrocytes.

### Confirmation of differentiation into cerebral cortex neurons and astrocytes

Cerebral cortex-type neurons were prepared, and through this, cerebral cortex-type patterning was preferentially carried out to secure astrocyte progenitor cells through several subcultures. After induction of differentiation from human embryonic stem cells into the nervous system, the confirmation thereof was preferentially performed by the confirmation of patterning, and the expression of cerebral cortex-type markers such as PAX6 and FOXG1 was confirmed. In addition, when the astrocyte progenitor cells were developed through several subcultures, the expression of astrocyte-type factors such as GFAP, AQP4, ALDH1L1, and GLAST was confirmed. The markers can be confirmed through a process such as an expression confirmation simultaneously using real time PCR or immunostaining method after RNA extraction.

### [Result]

### Preparation of 3D organoids

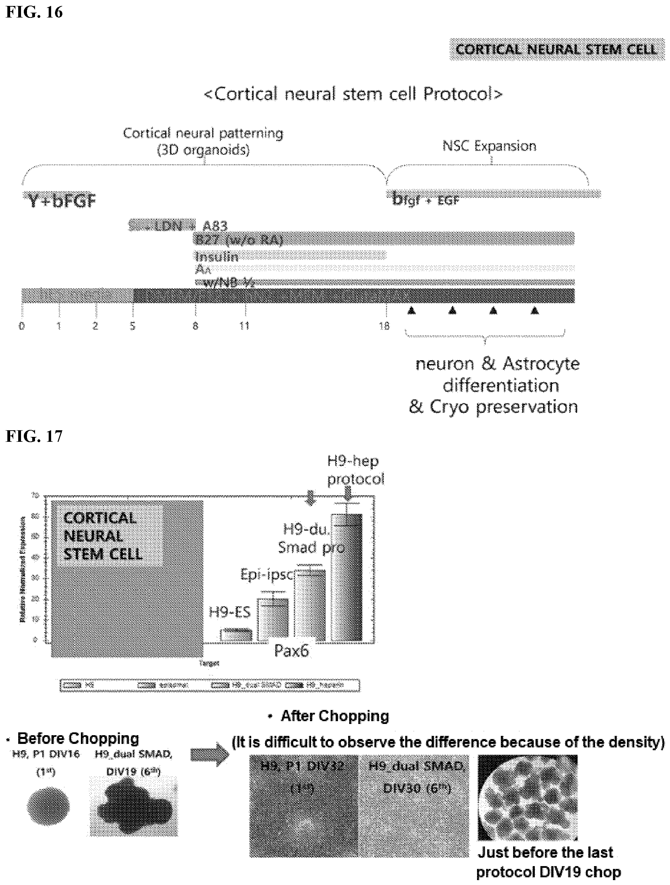

The point of the result is to confirm that the differentiation into a cerebral cortex cell population in a normal organoid form is preferentially performed, patterning is performed, and the differentiation into astrocyte progenitor cells is completed in the future. As shown in FIG. 17, it was confirmed that the formation of the organoids in a normal form was completed, and the expression of the patterning markers PAX6 and FOXG1 was maintained even after the organoids were chopped.

### Securing cerebral cortex neural stem cells and astrocyte progenitor cells from the prepared cerebral cortex 3D organoids

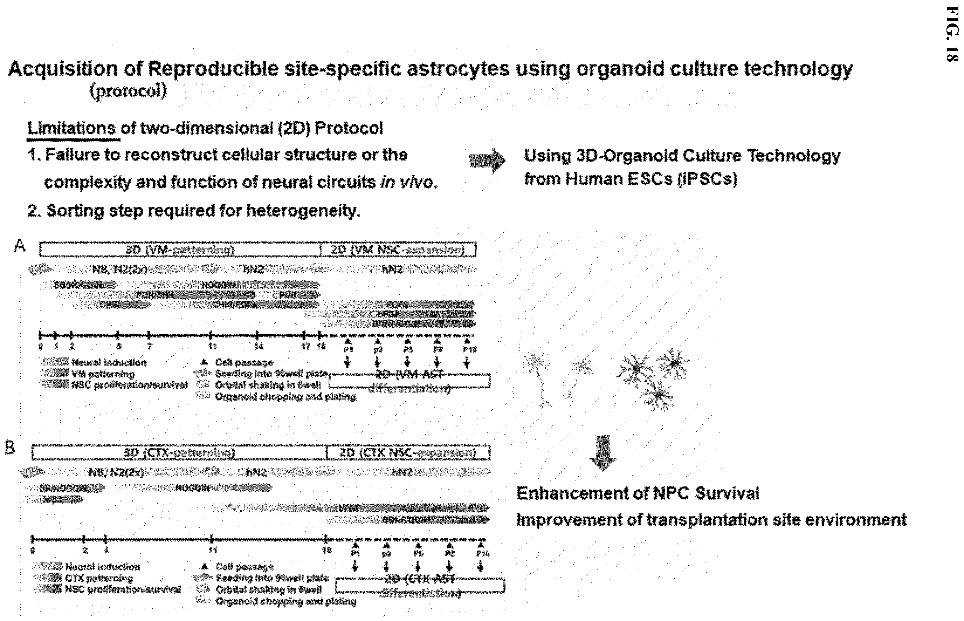

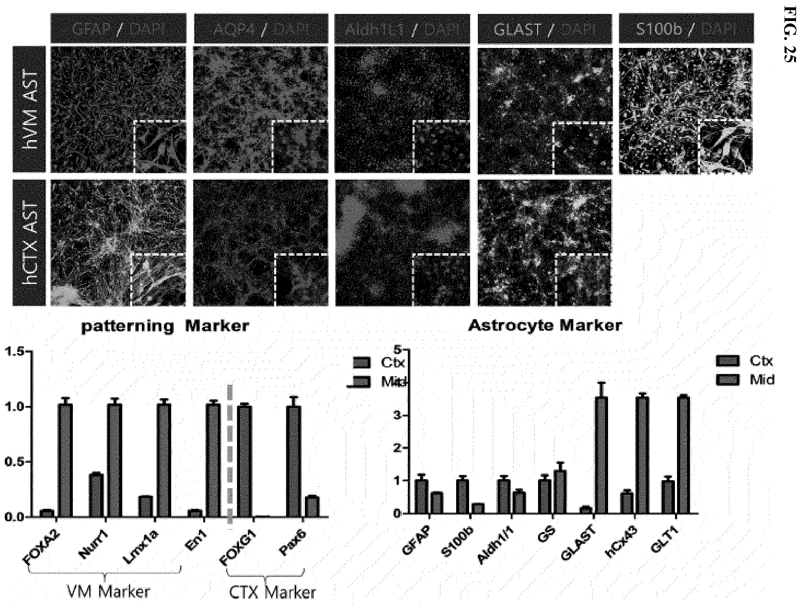

As in the mentioned protocol, SB431542 (10 µM), LDN (10 µM), and A-83 (10 µM) were added to induce a full-scale nervous system differentiation (lower drawing in FIG. 16, cerebral cortex induction scheme in FIG. 18), and it was confirmed that the organoids in a normal form were formed as shown in FIG. 17. For the confirmation, the expression of the cerebral cortex region patterning factors PAX6 and FOXG1 was confirmed by RNA-real time PCR (FIG.s 17 and 25). As in the method mentioned above, the development into astrocyte progenitor cells could be induced and secured through several subcultures. The sufficient expression of GFAP, AQP4, ALDH1L1, GLAST, and the like could be confirmed using immunostaining method, and at the same time, the expression of the patterning factors could be confirmed in this cell population (FIG. 25).

### Confirmation of differentiation of cerebral cortex neurons and astrocytes

The nervous system patterning factors SB431542 (10 µM), LDN (10 µM), and A-83 (10 µM) were added to induce a full-scale nervous system differentiation, and patterning into cerebral cortex-type brain organoids was performed. However, the process after differentiation into astrocyte progenitor cells through division of neurons and multiple passages from neural stem cells is not different from the process of differentiation through midbrain-like organoids except that it differs only in initial patterning. When compared with the differentiation of midbrain astrocyte progenitor cells, the sufficient expression of GFAP, AQP4, ALDH1L1, GLAST, and the like could be confirmed using immunostaining method, and at the same time, the expression of the patterning factors could be confirmed in each cell population (FIG. 25). It can be seen that differentiation into activated astrocytes can be completed in terms of functions and electrophysiological functions.

In this example, a method using 3D organoids capable of producing cerebral cortex specific neural stem cells was established. By utilizing 3D organoids, neural stem cells are extracted from tissues with the same structure as the actual *in vivo* cerebrum, so it can be expected that it is possible to secure healthier and actual cells.

### Example 3: Confirmation of differentiation from human induced pluripotent stem cells into midbrain astrocytes using midbrain-like organoids

### [Experimental procedure]

### Culturing of human pluripotent stem cells (hESCs/hiPSCs)

hESCs and hiPSCs were cultured based on the hESC research guidelines approved by the institutional review board (IRB) of Hanyang University (Seoul, Republic of Korea).

The hESCs and hiPSCs used in this experiment are shown in Table 8 below.

**[Table 8]**

| **hESC line** | | | | | | | | |
|---|---|---|---|---|---|---|---|---|
| **Name used in this paper** | | **Original name** | | | **Karyotype** | | **Establishing institute** | |
| HSF6 | | UC06 | | | Female (46, XX) | | UCSF | |
| H9 | | WA09 | | | Female (4b, XX) | | Wi ceil | |
| HUES6 | | HUES6 | | | Female (46, XX) | | harvard university(HSCI iPS Core) | |

| **hiPSC line** | | | | | | | | |
|---|---|---|---|---|---|---|---|---|
| **Name used in this paper** | **Original name** | | **Source (Human)** | **Reprogrammig factors** | | **Methods** | | **Establishing institute** |
| Retro-1 | Rv-hiPS 01-1 | | Newborn fibroblast | OCT4, SOX2, KLF4, MYC | | Retrovirus | | harvard |
| Epi-ips | episomal-ips1 | | Skin | OCT4, SOX2, XLF4, shp53 | | Episomal vector | | Hanyang Univ. (Our own) |
| CMC-ips#11 | CMC-hipsc-011 | | bone narrow blood | OCT4, SOX2, KLF4, MYC | | Sendai virus | | Catholic Univ. (Korea NIH SCRM) |
| PD-ips1 | PD patient derived ips-1 | | Skin | OCT4, SOX2, KLF4, shp53 | | Episomal vector | | hanyang Univ. (Our own) |
| PD-#22 | Corr. PD-ips#22 | | Skin | OCT4, SOX2, KLF4, shp53 | | Episomal vector and genome edited | | Hanyang Univ. (Our own) |

For proliferation and maintenance of undifferentiation hESC/iPSC, the cells were cultured on Matrigel^{™} or on a vitronectin (Human; Gibco Fisher Scientific, Waltham, MA) (Gibco A31804; 0.5 µg/cm²)-coated 6 cm dish (Thermo Fisher Scientific, Waltham, MA) using mTESR-1 medium (Stemcell Technologies Inc., Vancouver, BC, Canada) in a CO₂ incubator set at 37°C without a feeder layer, and medium replacement was performed daily. The undifferentiated stem cells maintained their differentiation ability by replacing the medium daily, and were subcultured using Acutase (Stemcell Technologies Inc.) every 4 to 5 days.

### Preparation of three-dimensional midbrain-like organoids

It was prepared in the same manner as in Example 1 until the midbrain-type organoids were prepared. In the early stage of development of the successfully prepared midbrain-type organoids, ZO-1 and N-cadherin were expressed along with PLZF+ and Sox2+, which are neural stem cell markers (FIGs. 7E and 7F). Anterior embryonic brain markers (OTX2) and ventral VM floor plate markers (FOXA2 and LMX1A) were expressed along with Nestin and Sox2, which can be referred to as general neural stem cell markers (FIGs. 7G to 71). When development was further induced, the neuron marker MAP2 was expressed as a layer outside the proliferation markers Ki67+ and SOX2+, and more specifically, like the brain layer of the living body, structures specifically expressed in PCNA+ (proliferative ventricular zone; VZ), MASH1+ (VZ, intermediate zone (IZ) and mantle zone (MZ)), and NURR1+ cells (IZ and MZ), respectively, were confirmed (FIGs. 7L and 7M). Characteristically, FOXA2+, LMX1A+, and NURR1+ cells, which are midbrain specific factors, were also expressed in the organoid, confirming that the organoid was directly prepared as a true midbrain-type organoid.

### Isolation of midbrain neural stem cells from midbrain-like organoids and differentiation into neurons

hESCs/hiPSCs were detachd from the dish using Acutase, placed in a neuron differentiation induction medium, and injected into a low attachment 96-well round bottom plate (Corning, Corning, NY) at 150 µL of 10,000 cells per well (day 0 of differentiation). The composition of the neuron differentiation induction medium was as follows (N2: Neurobasal (1:1, Gibco) containing B27 without vitamin A (2%, Invitrogen Fisher Scientific, Waltham, MA), GlutaMAX (1%, Invitrogen Fisher Scientific), minimum essential media-nonessential amino acid (1%, MEM-NEAA, Invitrogen Fisher Scientific), β-mercaptoethanol (0.1%, Invitrogen Fisher Scientific), SB431542 (10 µM, Tocris, Bristol, UK), Noggin (200 ng/ml, Peprotech, Rocky Hill, NJ), ascorbic acid (200 µM, Sigma-Aldrich), insulin (25 mg/L), ROCK inhibitor Y27632 (20 µM, Sigma-Aldrich, St. Louis, MO). The cells were treated with Y27632 only for 24 hours on the first day. For the expansion of ventral midbrain type neural stem cells of the organoids, sonic hedgehog (100 ng/ml, SHH, Peprotech) and purmorphamine (2 µM, Calbiochem, MilliporeSigma, Burlington, MA) were added to the neuron differentiation induction medium from day 1 of differentiation 1. On the second day, CHIR99021 (0.8 µM, Stemgent, Cambridge, MA) was added. The entire medium was replaced whenever the medium composition was changed. SB431542 was removed from day 5, and FGF8b (100 ng/ml, Peprotech) was added from day 7. On day 11, the basal medium was completely replaced with N2; and B27, GlutaMAX, MEM-NEAA, and β-mercaptoethanol were not added. The concentration of CHIR99021 added from day 11 was increased about two-fold (1.5 µM). From this point, the organoids were transferred from a 96 well plate to a 6 well plate (low attachment, Corning) and cultured on an orbital shaker at a speed of 80 rpm. Noggin and sonic hedgehog were removed from day 15, and bFGF (20 ng/ml) was added from day 17. On day 18, the organoids were digested with Acutase at 37°C for 7 minutes, and then chopped into pieces with a 30 gauge needle, and spread on vitronectin-coated 6 cm plates (Corning, cells from 24 organoids/plate). At this point, the cells were at the neural stem cell (NSC) stage, capable of being proliferated, and subcultured every 5-7 days. The medium used for NSC culture was an N2 proliferation medium, and the composition was as follows (N2 containing 10 ng/ml BDNF, 10 ng/ml GDNF, 200 µM ascorbic acid, 100 ng/ml FGF8b, 20 ng/ml bFGF, and 1 µg/ml doxycycline). The medium was replaced daily and treated with Y27632 at a concentration of 5 µM for one day at the first conversion to two-dimensional culture and at each subculture. The final differentiation into neurons was induced with an N2 differentiation medium, and the composition was as follows (10 ng/ml BDNF, 10 ng/ml GDNF, 200 µM ascorbic acid, and 500 µM db-cAMP).

### Isolation of midbrain astrocyte progenitor cells from midbrain-like organoids, and differentiation into astrocytes

If the passage of the prepared midbrain-type Og-NSCs is further increased according to the actual brain development stage, it is possible to culture Og-astrocyte progenitor cells mainly containing astrocyte progenitor cells after passage 9 without any change in medium composition, etc. Passage 9 to passage 11 can be used in a healthy state. As in Og-NSCs, the proliferation medium was an N2 proliferation medium, and the composition was N2 containing 10 ng/ml BDNF, 10 ng/ml GDNF, 200 µM ascorbic acid, 100 ng/ml FGF8b, 20 ng/ml bFGF, and 1 µg/ml doxycycline. The medium was replaced daily and treated with Y27632 at a concentration of 5 µM for one day at the first conversion to two-dimensional culture and at each subculture. Astrocyte progenitor cells in the proliferative stage was used for transplantation, and differentiation for research was induced with an N2 differentiation medium, and the composition was as follows (10 ng/ml BDNF, 10 ng/ml GDNF, 200 µM ascorbic acid, and 500 µM db-cAMP).

### [Result]

### Confirmation of preparation of 3D midbrain-like organoids

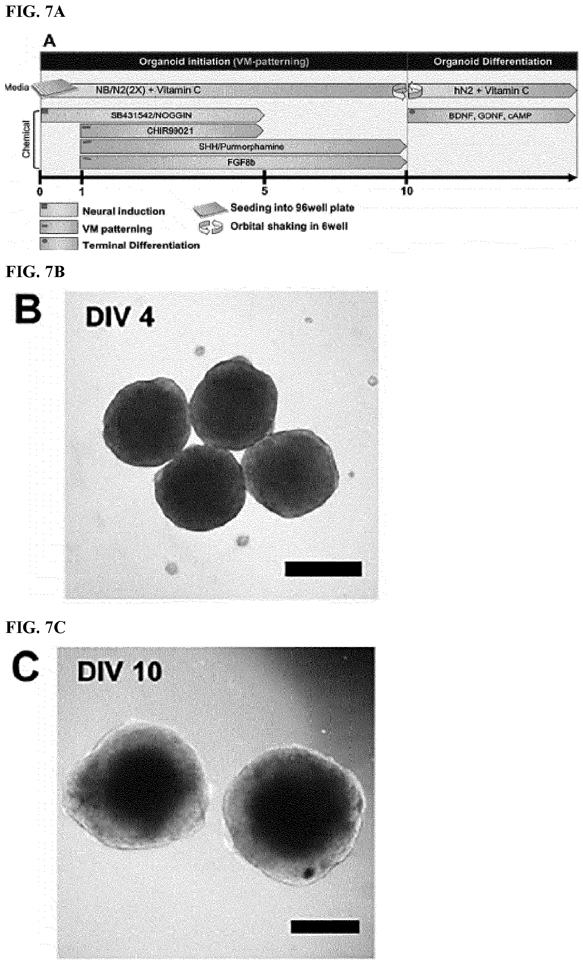

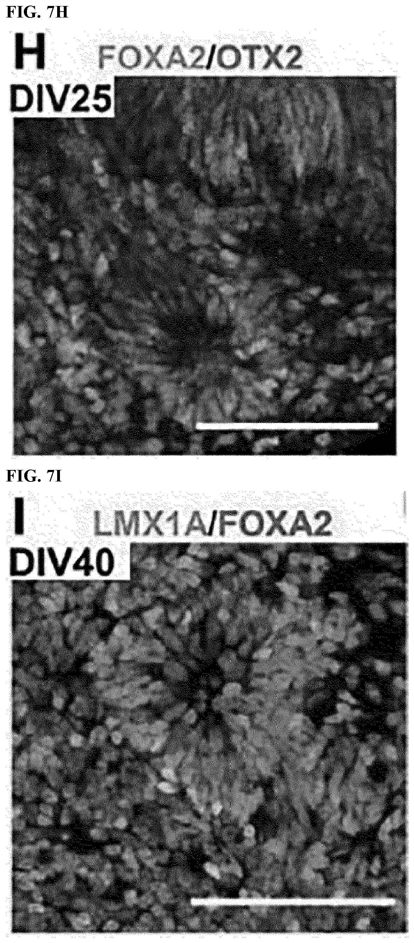

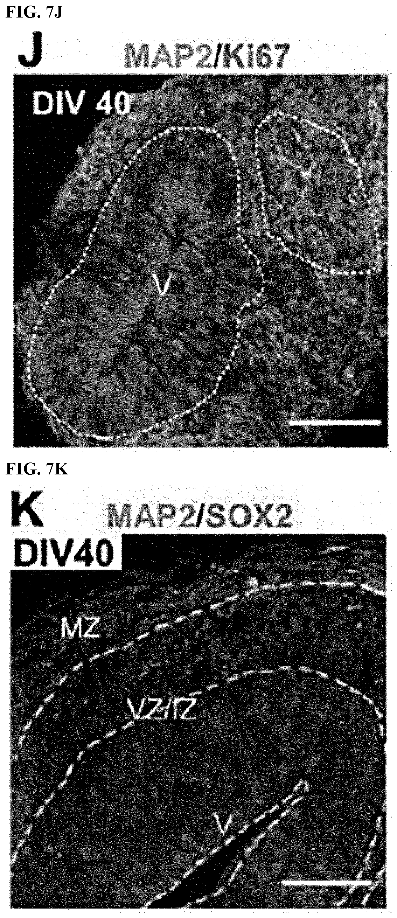

If the midbrain-type organoids shown as a result of FIG. 7 are prepared and the passage is further increased according to the actual brain development stage, it is possible to prepare midbrain-type organoids capable of being differentiated into midbrain-type neurons as well as astrocyte progenitor cells. Therefore, the results regarding the preparation and properties of midbrain-type organoids are the same as those shown during differentiation of neurons through midbrain-like organoids.

### Confirmation of securing midbrain astrocyte progenitor cells from prepared midbrain 3D organoids

### ① Direct differentiation of midbrain neural stem cells, and securing of astrocyte progenitor cells

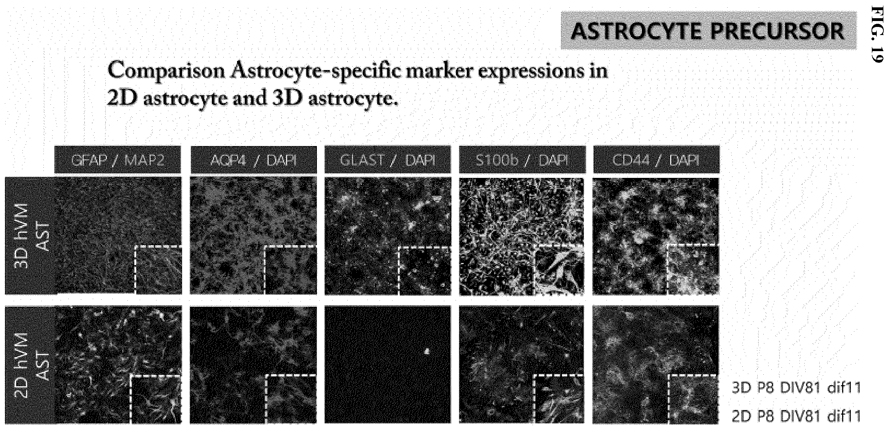

In order to induce differentiation from hESCs/iPSCs into neural stem cells and into midbrain-neural stem cells, midbrain-neural stem cells were secured using neuroectoderm inducers and midbrain-type neural stem cell inducers for about 20 days at the embryonic stem cell stage. The secured neural stem cells are easy for proliferation and stock, and a greater number of neural stem cells can be obtained through subculture. Confirmation of proper differentiation can be performed through the expression rate of neural stem cell markers such as Nestin and Sox2 and midbrain-neural stem cell markers such as Foxa2 and Lmx1a. When the present protocol is applied, differentiation into midbrain neural stem cells can be induced with a high efficiency of 95% or more as follows. In order to obtain astrocyte progenitor cells, additional subcultures are performed several times. During the initial subculture, terminally differentiated cells have a higher differentiation rate into neurons than astrocytes, but the proportion of astrocyte progenitor cells are increased as the subculture is increased. The conventional protocol was able to identify astrocytes upon differentiation after 5 subcultures, but for more efficient establishment of astrocyte progenitor cells, the neural stem cell population from passage 9 to passage 11 is used as the astrocyte progenitor cell population. At this time, the astrocyte progenitor cell marker CD44 was expressed 90% or more, and when differentiation was induced, the differentiated astrocyte marker group (GFAP, AQP4, GLAST, and S100b) was expressed 90% or more as shown in FIG. 19.

### ② Observation of higher quality effects of midbrain-type astrocyte progenitor cells

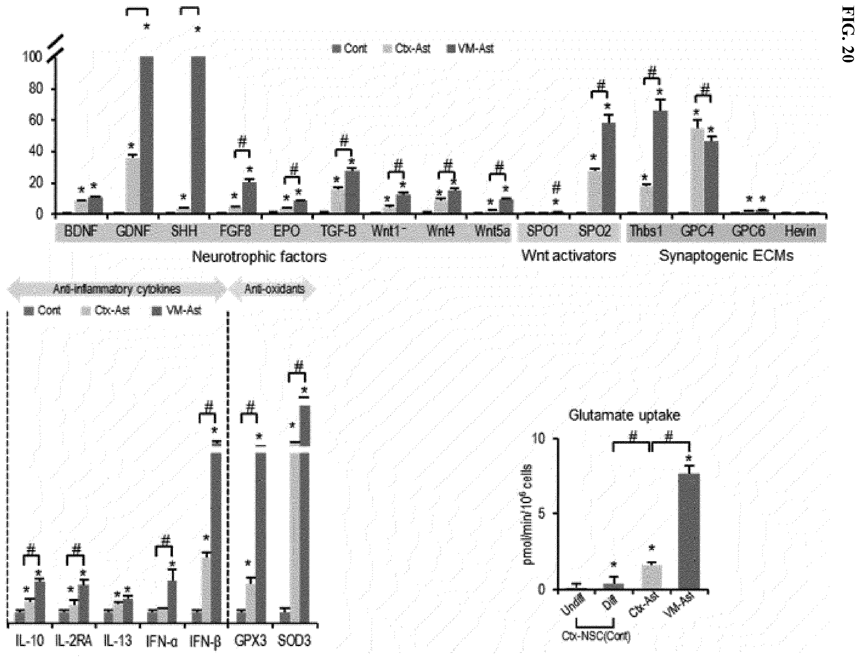

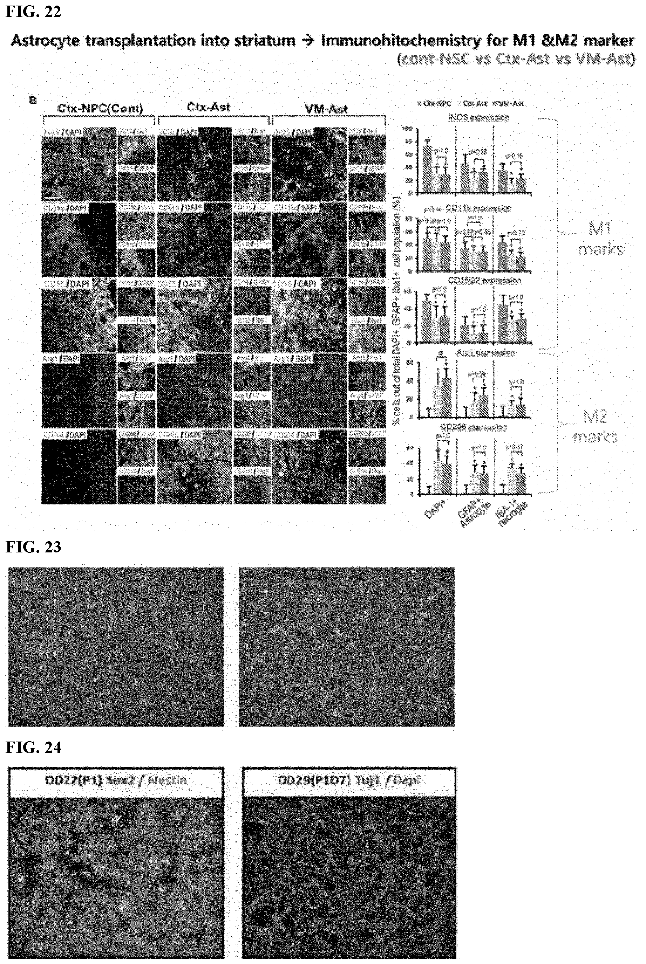

As a result of analysis of *in vitro* properties, it was observed that midbrain-type astrocytes overexpress factors related to secretion of more useful cytokines and maintenance of neuron function (FIG. 20). From *in vivo* results, it was also observed that the secretion of more useful factors promotes expression in midbrain-type astrocytes (FIG. 21). In addition, this effect of astrocytes affects microglia and is involved in the change to a better type of microglia, and a synergistic effect of anti-inflammatory effect can be expected (FIG. 22).

### Confirmation of differentiation of midbrain astrocytes

### Confirmation of protocol application using hiPSCs

An experiment was conducted to confirm whether the secured protocol was applied not only to H9 hESCs but also to other types of embryonic stem cells. To this end, iPSCs were secured from the Korea Centers for Disease Control and Prevention (CMC-hiPSC-003 and 011). In the culture stage, we succeeded in adaptive culture using a xeno-free system that does not use animal-derived components such as mouse feeder or matrigel, which were previously used for culturing human embryonic stem cells (FIG. 23), and secured a plurality of cell stocks.

As a result of applying the secured hiPSCs to the present protocol, it was confirmed that they were well differentiated into neural stem cells, and furthermore, it was confirmed that astrocyte markers such as GFAP were increased during the subculture process, as in H9 hESCs (FIG. 24).

Normal differentiation into midbrain astrocytes was confirmed (FIG. 25). That is, through this, it was confirmed that the present protocol can be applied not only to H9 hESCs but also to other types of embryonic stem cells. Among the used iPSC lines, #11 is easier for differentiation and will be a very important step in securing available induced pluripotent stem cells when practically used in the future.

### Efficient differentiation induction using TGF-β and LIF

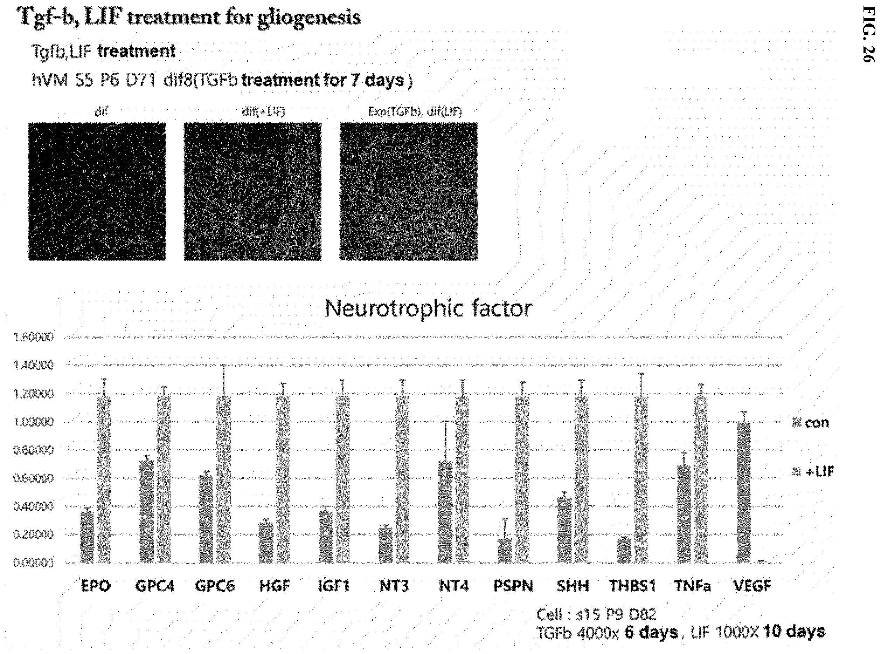

Through efficient differentiation induction using TGF-β and LIF, a technology for securing a large amount of astrocytes was secured. By treating with TGF-β in the proliferation stage and with LIF in the differentiation stage, it was observed that a greater amount of astrocytes were differentiated (by expression of the GFAP marker), and it was observed that these astrocytes became high-quality cells in which a greater amount of neuromodulatory factors were expressed (FIG. 26). In order to confirm the amount of astrocyte progenitor cells, the cells were treated with EdU, which can be marked only on dividing cells in the proliferation stage, only for 30 minutes, and fully differentiated, and then the increase or decrease of the progenitor cells of astrocytes was confirmed by confirming the amount expressed simultaneously with astrocytes. As a result, it was confirmed that the amount of astrocyte progenitor cells was increased by 1.5 times.

### In order to confirm the utility of the original material of astrocytes secured through the final protocol, the properties of the conditioned medium are preferentially analyzed

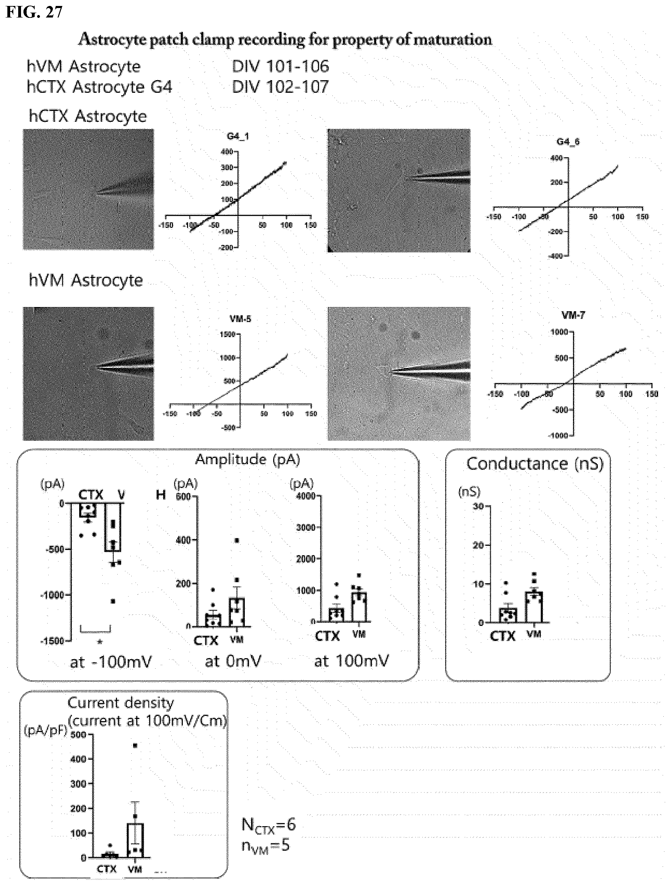

- Analysis of cell properties: In order to analyze the properties of the secured astrocytes, the physiological property was analyzed, and the physiological activity of the midbrain-type astrocytes was measured. Through this, it was confirmed that they were immature astrocytes suitable for securing differentiation derived materials and transplantation [FIG. 27]. Considering that fully mature astrocytes can be easily assimilated into a poor environment, these immature types of cells are more suitable for transplantation.
- Analysis of function: After transplantation, it was carried out to confirm the function of astrocytes, and it was observed *in vitro* and *in vivo* whether it was possible to perform functions as high-quality cells *in vivo* after transplantation. In order to find out whether the *in vitro* results are suitable for improving the surrounding environment, an experiment using a conditioned medium was conducted.

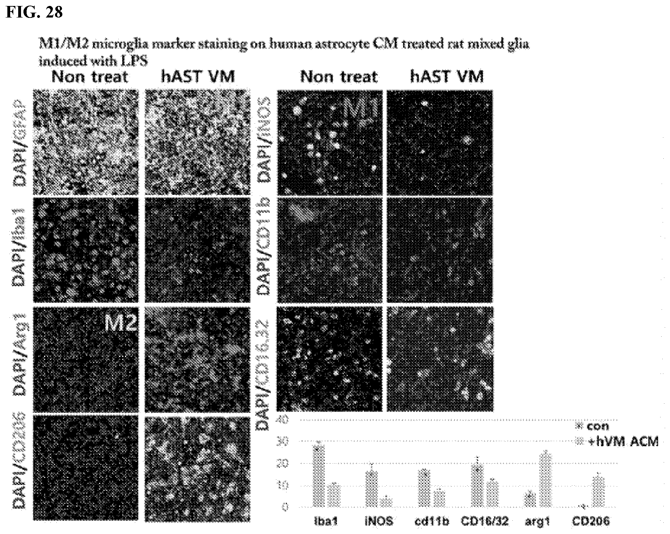

The results are shown in FIGs. 28, 29, and 30. FIG. 28 shows a result obtained by observing changes in microglia using a conditioned medium for midbrain-type astrocytes. Through this, the anti-inflammatory effect by astrocytes was verified *in vitro.* In cell culture containing microglia, when inflammation was induced by LPS treatment, treatment with conditioned medium for midbrain astrocytes reduced the expression of iNOS, Cd11b, and CD16.32, which are poor microglia markers, and increased the expression of Arg and CD206, markers showing good transformation into microglia.

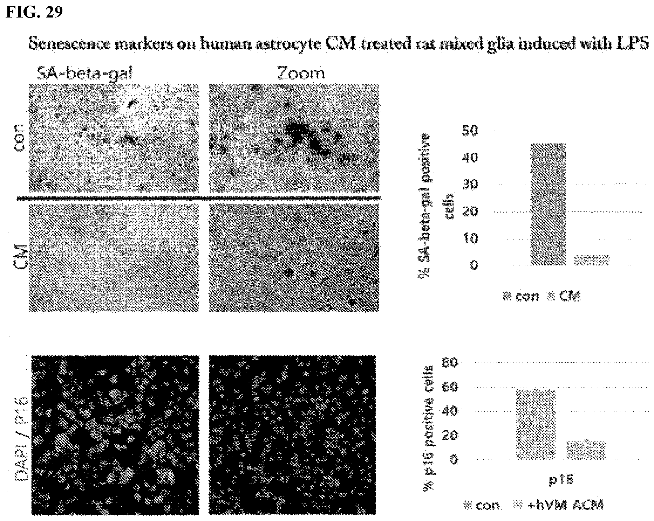

FIG. 29 shows a result obtained by confirming the possibility of inhibiting death factors with an astrocyte conditioned medium after induction of inflammation. Through this, the anti-inflammatory effect by astrocytes was verified *in vitro.* In addition, in cell culture containing microglia, when inflammation was induced by LPS treatment, treatment with conditioned medium for midbrain astrocytes reduced the expression of b-gal and p16, which are apoptosis markers.

FIG. 30 shows a result obtained by confirming the anti-inflammatory effect of an astrocyte conditioned medium after induction of inflammation using RT-PCR. Through this, the anti-inflammatory effect by astrocytes was verified *in vitro.* In addition, in cell culture containing microglia, when inflammation was induced by LPS treatment, it was confirmed that treatment with conditioned medium for midbrain astrocytes reduced the expression of inflammosome formation-related factors and increased the expression of neurogenic factors.

### Analysis of property after transplantation to confirm in vivo utility of astrocytes secured through final protocol

First, we studied the positive effect of astrocytes on the surrounding environment after transplantation into the *in vivo* brain. After transplantation of astrocytes into the striatum site, the results of anti-inflammatory factors, inflammatory factors, and cytokine secretion, and the like were confirmed. The expression of inflammatory factors such as TNF-a, IL1b, iNOS, and CD11b was reduced at the astrocyte transplantation site, and the expression of inflammatory factors such as IL1b, iNOS, and CD11b was reduced at the surrounding site, and the expression of BDNF, GDNF, and Arg1 cytokines was slightly increased at the transplantation site and the surrounding site (FIG. 31).

### The effect of improving the environment after transplantation of astrocytes in vivo was observed in vivo through changes in microglia.

The secured astrocytes were transplanted into the rat brain striatum, and then neural stem cells were transplanted into the opposite side as a control group. It was confirmed whether the microglia were converted to M1 and M2 by observing representative environmental changes.

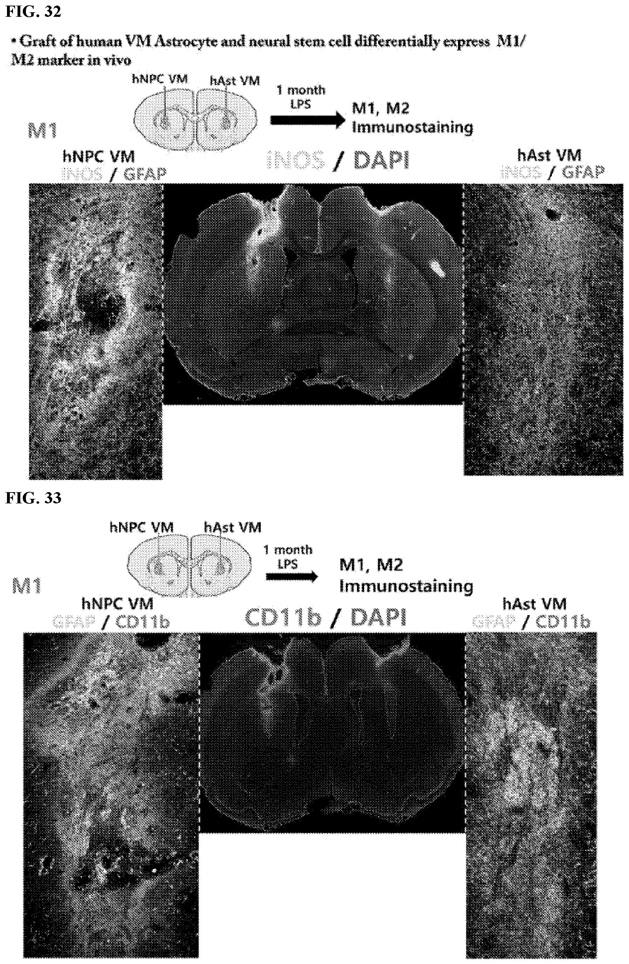

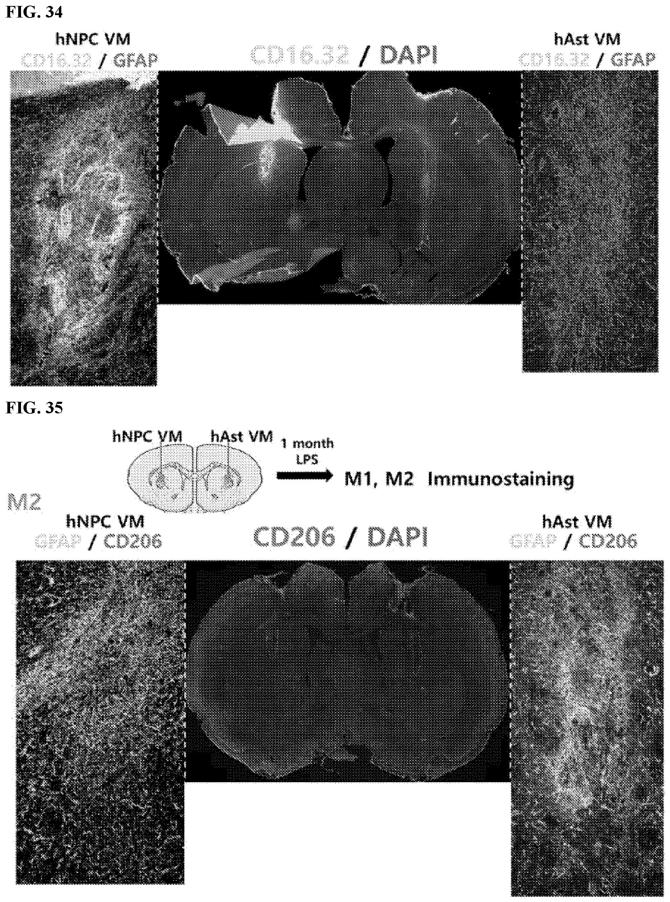

Inflammation was induced by LPS, and astrocytes were transplanted. As a result, it was observed that the expression of the poor type M1 markers iNOS, CD11b, CD16, and the like, was inhibited at the astrocyte transplantation site (FIGs. 32 to 34), and the expression of the M2 marker CD206 was increased. Therefore, a positive effect of astrocytes was confirmed (FIG. 35).

### Example 4: Confirmation of differentiation from human induced pluripotent stem cells into hypothalamus neurons and astrocytes using hypothalamus organoids

### [Experimental procedure]

### Culturing of human pluripotent stem cells (hESCs/hiPSCs)

hESCs and hiPSCs were cultured based on the hESC research guidelines approved by the institutional review board (IRB) of Hanyang University (Seoul, Republic of Korea).

The hESCs and hiPSCs used in this experiment are shown in Table 9 below.

**[Table 9]**

| **hESC line** | | | | | | | | |
|---|---|---|---|---|---|---|---|---|
| **Name used in this paper** | | **Original name** | | | **Karyotype** | | **Establishing institute** | |
| HSF6 | | UC06 | | | Female (46, XX) | | UCSF | |
| H9 | | WA09 | | | Female (46, XX) | | Wi cell | |
| HUES6 | | HUES6 | | | Female (46: XX) | | harvard university(HSCI iPS Core) | |

| **hiPSC line** | | | | | | | | |
|---|---|---|---|---|---|---|---|---|
| **Name used in this paper** | **Original name** | | **Source (Human)** | **Reprogramming factors** | | **Methods** | | **Establishing institute** |
| Retro-1 | Rv-hiPS 01-1 | | Newborn fibroblast | OCT4, SOX2, KLF4, MYC | | Retrovirus | | harvard |
| Epi-ips | episomal-ips1 | | Skin | OCT4, SOX2, KLF4, shp53 | | Episomal vector | | Hanyang Univ. (Our own) |
| CMC-ips#11 | CMC-hipsc-011 | | bone narrow blood | OCT4, SOX2, KLF4, MYC | | Sendai virus | | Catholic Univ. (Korea NIH SCRM) |
| PD-ips1 | PD patient derived ips-1 | | Skin | OCT4, SOX2, KLF4, shp53 | | Episomal vector | | hanyang Univ. (Our own) |
| PD-#22 | Corr. PD-ips#22 | | Skin | OCT4, SOX2, KLF4, shp53 | | Episomal vector and genome edited | | Hanyang Univ. (Our own) |

For proliferation and maintenance of undifferentiation hESC/iPSC, the cells were cultured on Matrigel^{™} or on a vitronectin (Human; Gibco Fisher Scientific, Waltham, MA) (Gibco A31804; 0.5 µg/cm²)-coated 6 cm dish (Thermo Fisher Scientific, Waltham, MA) using mTESR-1 medium (Stemcell Technologies Inc., Vancouver, BC, Canada) in a CO₂ incubator set at 37°C without a feeder layer, and medium replacement was performed daily. The undifferentiated stem cells maintained their differentiation ability by replacing the medium daily, and were subcultured using Acutase (Stemcell Technologies Inc.) every 4 to 5 days.

### Preparation of three-dimensional hypothalamus organoids

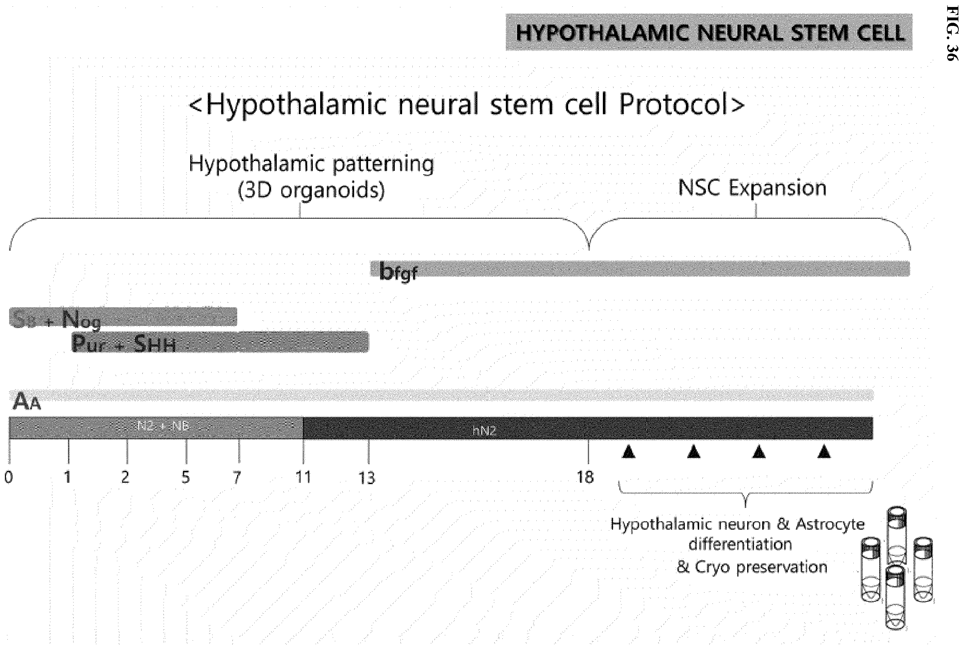

Organoids were prepared by the same protocol as in FIG. 36.

Basically, because organoids of the nervous system were prepared from embryonic/induced pluripotent stem cells, human embryonic/induced pluripotent stem cells were detachd from the dish using Acutase, placed in a neuron differentiation induction medium, and injected into a low attachment 96-well round bottom plate (Corning, Corning, NY) at 150 µL of 10,000 cells per well (day 0 of differentiation). For the preparation of organoids into the nervous system, they were treated with SB -431542, 10 µM, and Noggin, 100 ng/mL, induced into the nervous system, and patterned by treatment with the ventral inducers SHH and purmorphamine. The specific composition of the medium is shown in Tables 10 and 11 below.

### Isolation of hypothalamus neural stem cells from hypothalamus organoids, and confirmation of differentiation into neurons

The composition of the differentiation induction medium according to the date is shown in Table 10 below.

**[Table 10]**

| **Day** | **Media Composition** |
|---|---|
| **96-well (seeding: 1** × **10² cells/well)** | |
| D0 | Neurobasal : 2 × N2 (1:1) |
| | 1×B27 (- RA) |
| | 1×MEM NEAA |
| | 1×Glutamax |
| | β -mercaptoethanol, 55 uM |
| | Penicillin/Streptomycin |
| | Ascobic Acid. 200 uM |
| | Doxycycline. 1 uγ/mL |
| | Y-27632. 10 uM |
| | SB -431542. 10 uM |
| | Moxxin. 100 nγ/mL |
| D1-6 | Neurobasal : 2×N2 (1:1) |
| | 1×B27 (- RA) |
| | 1×MEM NEAA |
| | 1×Glutamax |
| | β -mercaptoethanol. 55 uM |
| | Penicillin/Streptomycin |
| | Ascorbic Acid. 200 uM |
| | SB -431542. 10 uM |
| | |
| | SHH. 100 nγ/mL. |
| | Punmorphamine. 2 uM |
| D7-12 | Neurobasal : 2×N2 (1:1) |
| | 1×B27 (- RA) |
| | 1×MEM NEAA |
| | 1×Glutamax |
| | β -mercaptoethanol. 55 uM |
| | Penicillin/Streptomycin |
| | Ascorbic Acid. 200 uM |
| | Noxxin. 100 ng/mL |
| | SHH. 100 ng/mL. |
| | Pumarphammine. 2 uM |

| Move to 6-well culture plate | |
|---|---|
| D13-20 | N2 medium |
| | Penicillin/Streptomycin |
| | Ascorbic Acid. 200 uM |
| | FGF2. 20 ng/mL. |

| **Chopping** | |
|---|---|
| M18-21 | N2 medium |
| | Penicillin/Streptomycin |
| | Ascorbic Acid. 200 uM |
| | Doxycycline. 1 ug/mL. |
| | FGF2. 20 ng/mL I |

For the first seeding, N2: Neurobasal (1:1, Gibco), B27 (without RA), 2-mercapto 55 µM, MEM-NEAA, GlutaMAX, ascorbic acid, 200 µM, SB431542 (10 µM), Noggin 100 ng/ml, ROCK inhibitor Y27632 (20 µM), doxycyclin 1 µg/ml were added. The cells were treated with Y27632 and doxycyclin only for 24 hours on the first day. Between day 1 and day 6, SHH, 100 ng/mL, and Purmorphamine, 2 µM were added while using the basal medium. Between day 7 and day 12, a medium only without SB431542 was used while using the conventional basal medium. It was transferred to a 6 well plate (low binding) on day 13 and stirred with an orbitary shaker. On days 13 to 20, only ascorbic acid, 200 µM, and FGF2, 20 ng/mL were added to the N2 medium and the cells were cultured, and the entire basal medium was used as N2 until spread on a plate after chopping in 2D state on day 21 (possible after day 18), and doxycyclin was added only on the first day of plating. The entire medium was replaced whenever the medium composition was changed, and the medium used on day 13 was used as the proliferation medium. It was treated with doxycyclin at a concentration of 1 µg/ml and Y27632 at a concentration of 5 µM for one day at each subculture. The final differentiation into neurons was induced with an N2 differentiation medium (10 ng/ml BDNF, 10 ng/ml GDNF, and 200 µM ascorbic acid). The basal medium is shown in Table 11 below.

**[Table 11]**

| **2x N2 medium** | **N2 medium** |
|---|---|
| 12 g DMEM/F12 (- HEPES buffer, - sodium bicarbonate) | 12 g DMEM/F12 (- HEPES buffer, - sodium bicarbonate) |
| 1.69 g Sodium bicarbonate | 1.69 g Sodium bicarbonate |
| 1.55 g D(+) Glucose | 1.55 g D(+) Glucose |
| 0.073 g L-Clutamine | 0.073 g L-Glutamine |
| 200 mg Apo-transferrin | 100 mg Apo-transferrin |
| "Progesterone, 40 nM | Progesterone, 20 nM |
| Putrescine, 200 µM | Putrescine, 100 µM |
| Selenite, 60 nM | Selenite, 30 nM |
| Insulin, 200 nM | Insulin, 200 nM |
| 1 L water | 1 L water |

### Isolation of hypothalamus astrocyte progenitor cells from hypothalamus organoids, and confirmation of differentiation into astrocytes

If the passage of the prepared hypothalamus-type Og-NSCs is further increased according to the actual brain development stage, it is possible to culture Og-astrocyte progenitor cells mainly containing astrocyte progenitor cells after passage 9 without any change in medium composition, etc. Passage 9 to passage 11 can be used in a healthy state. As in hypothalamus-type Og-NSCs, the proliferation medium was an N2 proliferation medium, and the composition was IX N2 supplement containing 200 µM ascorbic acid, 20 ng/ml bFGF, and 1 µg/ml doxycycline. The medium was replaced daily and treated with Y27632 at a concentration of 5 µM for one day at the first conversion to two-dimensional culture and at each subculture. Like organoid-derived astrocyte progenitor cells in the midbrain, cells in the proliferative stage with an older passage are used for transplantation studies.

### [Result]

### Confirmation of preparation of 3D hypothalamus organoids

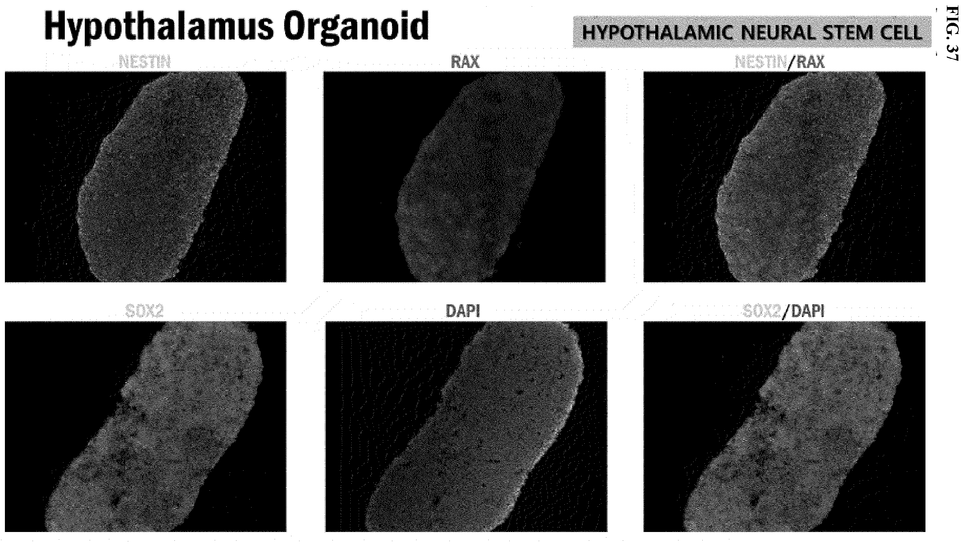

As shown in FIG. 37, the prepared hypothalamus organoids have a normal shape and have an initial morphology expressing Nestin and Sox2, which are markers of neural stem cells, together with the hypothalamus marker RAX.

### Confirmation of securing hypothalamus neural stem cells and astrocyte progenitor cells from prepared hypothalamus 3D organoids

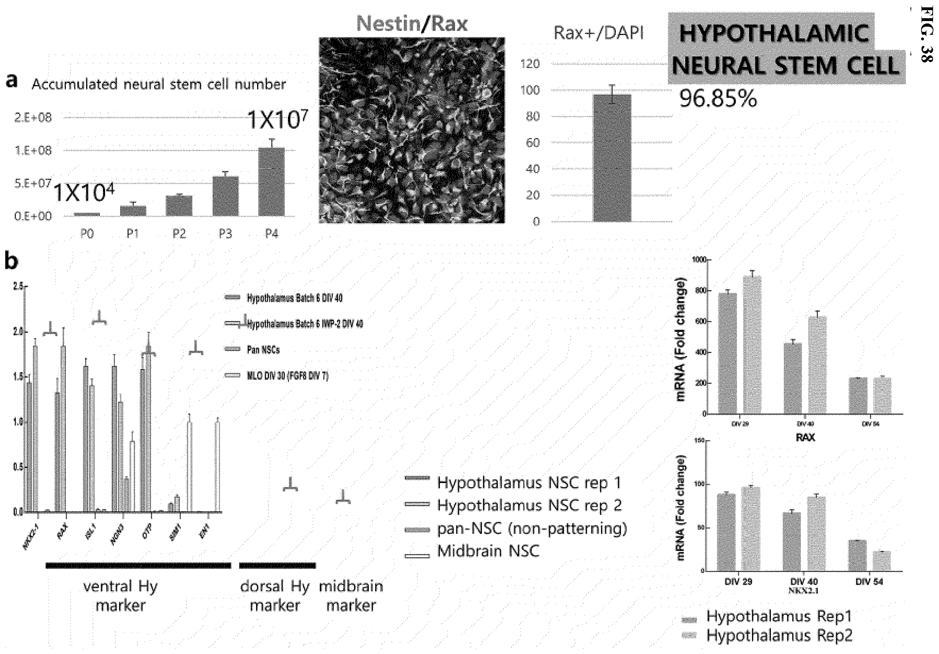

It was confirmed that the neural stem cells chopped and extracted from the hypothalamus organoids became properly patterned neural stem cells in which 96.8% of the hypothalamus markers were expressed as shown in the results of FIG. 38. Quantitatively, neural stem cells up to passage 4 can be used for neuron differentiation, and it is possible to secure about 1,000 times the number of neural stem cells from the first 10,000 cells (FIG. 38). Neural stem cells express RAX and NKX2.2 as patterning markers.

### Confirmation of differentiation of hypothalamus neurons and astrocytes

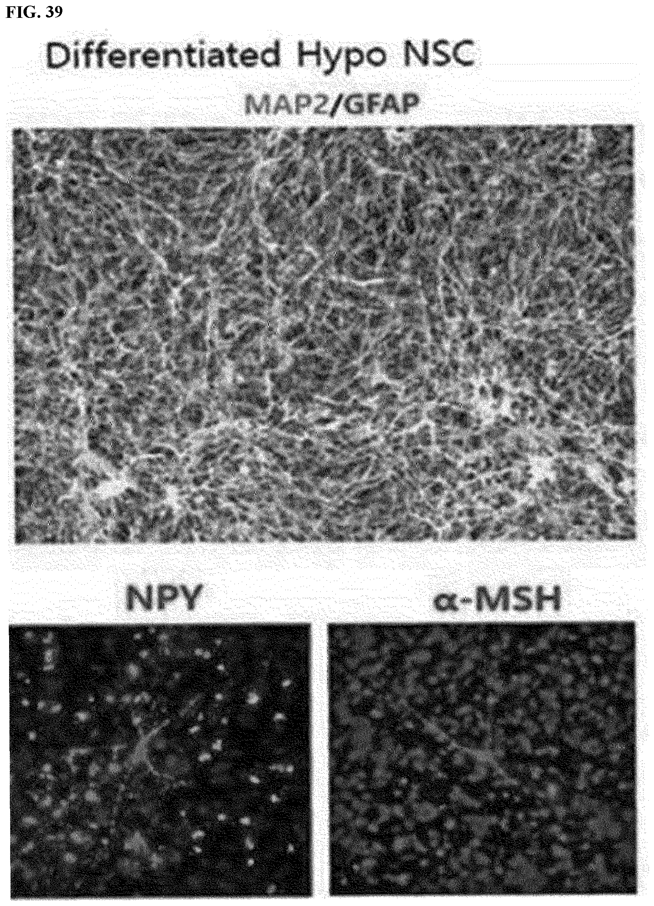

As shown in the results of FIG. 39, the prepared hypothalamus stem cells can be differentiated into MAP2 expressing neurons and GFAP expressing astrocytes during differentiation. Neurons are differentiated into cells expressing the hypothalamus specific markers NPY and a-MSH.

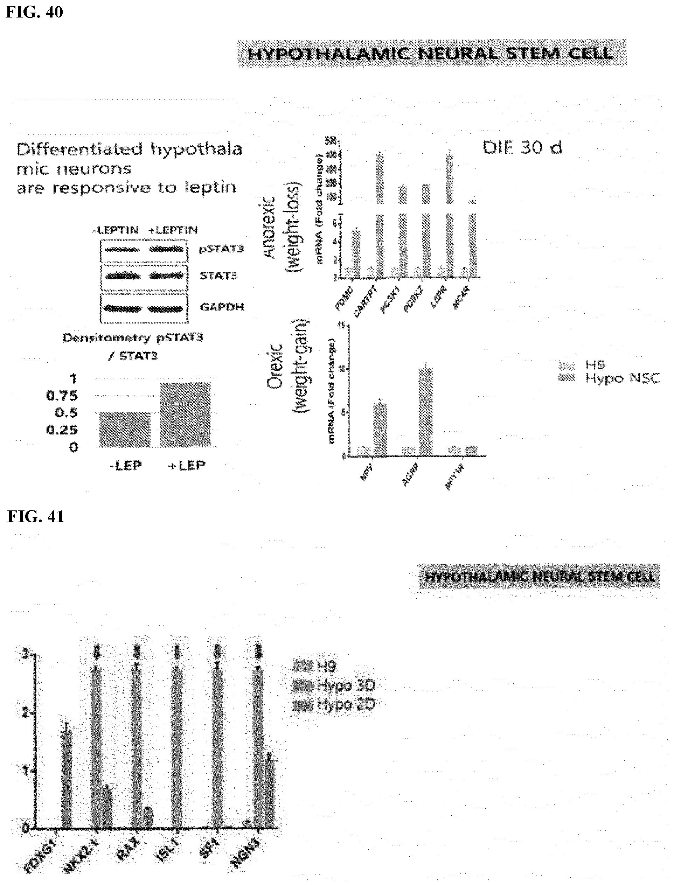

In order to confirm the formation of functionally complete cells after differentiation, when specifically observing leptin reactivity (FIG. 40), the sensitivity to the p-stat3 mechanism was measured, and when observing the gene response, the expression of the anorexic-related POMC, CARTPT, PCSK1,2, LEPR, and MC4R was increased, and the expression of orexic-related genes NPY, AGRP, and NPY1R was increased. Thus, this suggests that the cells can fully function. In particular, it can be seen, through the expression of NKX2.1, RAX, ISL, SF1, and NGN3, that the three-dimensional organoid-derived cells are more well patterned compared to the two-dimensional culture (FIG. 41).

Securing hypothalamus neural stem cells as leptin-responsive stem cells shows that they can actually perform their functions similar to those in the living body, and suggests that they have the possibility to be mainly used for research and development of therapeutic agents for metabolic diseases such as obesity and diabetes in the future (FIG. 40).

### Confirmation of in vivo transplantation possibility of hypothalamus neural stem cells developed by using 3D organoids

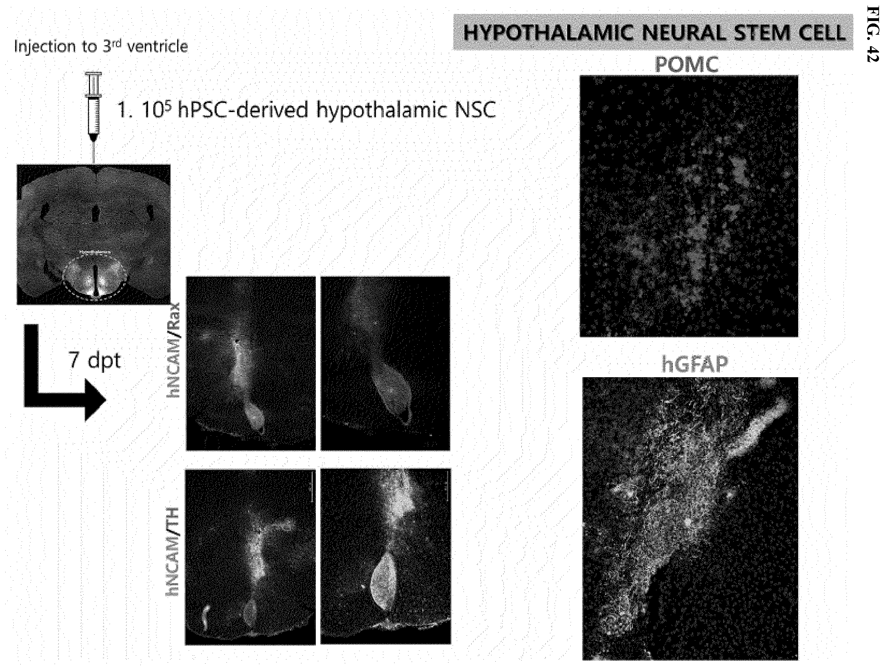

The possibility of *in vivo* transplantation of hypothalamus neural stem cells developed by using 3D organoids was confirmed. After transplanting 1 × 10⁵ hypothalamus neural stem cells into the 3^{rd} ventricle, the engraftment possibility was confirmed 7 days later. It was confirmed that the hypothalamus neural stem cells expressing hNCAM/Rax were stably engrafted, and at the same time, it was confirmed that they took the form of hypothalamus neural stem cells expressing hGFAP/POMC (FIG. 42).

## Claims

1. A method of patterning and chopping 3D organoids prepared from human pluripotent stem cells, culturing stem cells or progenitor cells, and inducing the differentiation thereof to obtain a large amount of finally differentiated cells.

2. A method to obtain a large amount of finally differentiated cells, comprising:
1) proliferating and culturing human pluripotent stem cells to prepare 3D organoids;
2) patterning and chopping the prepared 3D organoids; and
3) culturing and proliferating stem cells or progenitor cells in the chopped organoids, and inducing the differentiation thereof to obtain a large amount of finally differentiated cells.

3. The method according to claim 1 or 2, wherein the human pluripotent stem cells and stem cells are cultured using vitronectin.

4. The method according to claim 1 or 2, wherein the stem cells are ectodermal stem cells, mesodermal stem cells, or endodermal stem cells.

5. The method according to claim 1 or 2, wherein the progenitor cells are cerebral astrocyte progenitor cells or midbrain astrocyte progenitor cells.

6. The method according to claim 1 or 2, wherein the cells are neurons or astrocytes.

7. A cell therapeutic agent comprising the cells finally obtained by the method according to claim 1 or 2.

8. The cell therapeutic agent according to claim 7, wherein the cell therapeutic agent treats a disease selected from the group consisting of a neurodegenerative disease, an inflammatory degenerative disease, and a metabolic disease.

9. The cell therapeutic agent according to claim 8, wherein the neurodegenerative disease is one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease, and Pick disease.

10. The cell therapeutic agent according to claim 8, wherein the inflammatory degenerative disease is one selected from the group consisting of dementia, Lewy body dementia, frontotemporal dementia, white matter degeneration, adrenoleukodystrophy, multiple sclerosis, and Lou Gehrig's disease.

11. The cell therapeutic agent according to claim 8, wherein the metabolic disease is one selected from the group consisting of diabetes mellitus, obesity, and a metabolic disorder.

12. A drug screening method using the cells finally obtained by the method according to claim 1 or 2.

13. The drug screening method according to claim 12, wherein the drug treats a disease selected from the group consisting of a neurodegenerative disease, an inflammatory degenerative disease, and a metabolic disease.

14. The drug screening method according to claim 12, wherein the neurodegenerative disease is one selected from the group consisting of Parkinson's disease, dementia, Alzheimer's disease, Huntington's disease, amyotrophic lateral sclerosis, memory loss, myasthenia gravis, progressive supranuclear palsy, multiple system atrophy, essential tremor, corticobasal degeneration, diffuse Lewy body disease, and Pick disease.

15. The drug screening method according to claim 12, wherein the inflammatory degenerative disease is one selected from the group consisting of dementia, Lewy body dementia, frontotemporal dementia, white matter degeneration, adrenoleukodystrophy, multiple sclerosis, and Lou Gehrig's disease.

16. The drug screening method according to claim 12, wherein the metabolic disease is one selected from the group consisting of diabetes mellitus, obesity, and a metabolic disorder.
